# EUROPEAN PATENT APPLICATION

(11) **EP 4 306 540 A1**
(43) Date of publication of application: **17.01.2024**
(21) Application number: 22766178.2
(22) Date of filing: 28.02.2022
(51) Int. Cl.: C07K 16/28, C07K 16/46, C07K 19/00, C12N 15/13, C12N 15/63, A61K 39/395, A61K 39/00, A61P 35/00

(54) **ROR1 BINDING PROTEIN AND USE THEREOF**

(30) Priority: 09.03.2021 CN 202110255430
(71) Applicant: Sichuan Kelun-Biotech Biopharmaceutical Co., Ltd., Chengdu, Sichuan 611138 (CN); Klus Pharma Inc., Princeton, New Jersey 08540 (US)
(72) Inventor: TIAN, Haijun, Princeton, NJ 08540 (US); CHEN, Yangde, Princeton, NJ 08540 (US); CHANG, Ying-Hua, Princeton, NJ 08540 (US); ZHU, Zhiqiang, Princeton, NJ 08540 (US); WU, Wenhui, Princeton, NJ 08540 (US)
(74) Representative: Mewburn Ellis LLP
(86) International application number: PCT/CN2022/078327
(87) International publication number: WO 2022/188652

(57) **Abstract**

The present invention relates to the field of treatment of diseases. Specifically, the present invention relates to an anti-ROR1 antibody or an antigen-binding fragment thereof, nucleic acid molecules for encoding same, and methods for preparing same. The anti-ROR1 antibody or the antigen-binding fragment thereof of the present invention has high specificity and high affinity for ROR1, and can effectively bind ROR1 and mediate killing of ROR1 expression cells. Therefore, the present invention further relates to a pharmaceutical composition comprising the antibody or antigen-binding fragment thereof, and a use thereof in preparation of a drug which is used for prevention and/or treatment of tumors.

## Description

### FIELD OF THE INVENTION

The present invention belongs to the field of therapeutic monoclonal antibodies, specifically relates to an antibody against ROR1; and also relates to the use of the antibody in treating diseases.

### BACKGROUND OF THE INVENTION

Receptor tyrosine kinases (RTKs) are multigroup transmembrane proteins that act on receptors for cytokines, growth factors, hormones, and other signaling molecules, and play important roles in a variety of cellular processes, including growth, differentiation, angiogenesis, and progression of various cancers. Receptor tyrosine kinase-like orphan receptor 1 (ROR1) is a member of the family of receptor RTKs, which has high homology with the tyrosine kinase domain of growth factor receptors. ROR1 is low expressed in the process of embryonic development, but highly expressed in a variety of solid and hematological malignancies, including various types of leukemia, non-Hodgkin's lymphoma, breast cancer, ovarian cancer, melanoma, lung adenocarcinoma, etc. Therefore, ROR1 is a novel drug target with broad-spectrum anticancer potential.

In recent years, genetically engineered antibodies targeting ROR1 have been successively developed for the research of tumor immunotherapy, including single-chain fragment variables (scFvs), chimeric antibodies, Fab antibodies, antibody-drug conjugates (ADCs), chimeric antigen receptors (CARs) and bispecific antibodies (BiAbs), etc. At present, the anti-ROR1 monoclonal antibody with rapid research progress is cirmtuzumab from Oncternal Therapeutics, and its clinical data show good anticancer effect, and it can better inhibit the proliferation, migration and survival of chronic leukemia cells.

There is currently no ROR1-targeting antibody drug on the market. Therefore, it is urgent and necessary to develop an anti-ROR1 antibody with higher specificity, lower toxicity, better clinical efficacy, and more convenient administration, which will provide patients with more medication options.

### CONTENT OF THE INVENTION

In the present application, the inventors first developed a murine antibody with excellent properties that can specifically recognize/bind to ROR1 but not to ROR2. On this basis, the inventors put in a lot of creative work and carried out in-depth research and modification on the murine antibody, thus developing the chimeric antibody and humanized antibody of the murine antibody. The humanized antibody of the present invention not only has a very high degree of humanization, but also has biological functions essentially comparable to (or even better than) those of the murine antibody and the human-mouse chimeric antibody (the chimeric antibody has the same heavy chain variable region and light chain variable region as the murine antibody).

Therefore, the antibody (especially humanized antibody) of the present invention is extremely advantageous, which retains the functions and properties of the parental murine antibody, such as binding to ROR1 with high affinity and specificity, and thus has the potential to prevent and treat tumors; in addition, the antibody of the present invention can more effectively induce ROR1-mediated internalization when bind to ROR1 on the cell surface, so it can be used as a carrier for targeted therapy. The humanized antibody of the present invention has a very high degree of humanization, so that it can be safely administered to a human subject without causing immunogenic reaction. Therefore, the antibody of the present invention has great clinical value.

### Antibody of the present invention

In one aspect, the present invention provides an antibody or antigen-binding fragment thereof capable of specifically binding to ROR1.

In some embodiments, the present invention provides an antibody or antigen-binding fragment thereof capable of specifically binding to an extracellular domain (ECD) of ROR1.

In some embodiments, the antibody or antigen-binding fragment thereof comprises the following complementarity determining regions (CDRs):
(a) CDR-H1, CDR-H2 and CDR-H3 contained in the heavy chain variable region (VH) as set forth in any one of SEQ ID NOs: 1, and 102-106; and/or
   CDR-L1, CDR-L2 and CDR-L3 contained in the light chain variable region (VL) as set forth in any one of SEQ ID NOs: 2, and 107-110;
   or
(b) CDR-H1, CDR-H2 and CDR-H3 contained in the heavy chain variable region (VH) as set forth in any one of SEQ ID NOs: 3, and 84-86; and/or
   CDR-L1, CDR-L2 and CDR-L3 contained in the light chain variable region (VL) as set forth in any one of SEQ ID NOs: 4, and 87-101;
   or
(c) CDR-H1, CDR-H2 and CDR-H3 contained in the heavy chain variable region (VH) as set forth in any one of SEQ ID NOs: 5, and 111-115; and/or
   CDR-L1, CDR-L2 and CDR-L3 contained in the light chain variable region (VL) as set forth in any one of SEQ ID NOs: 6, 107, and 116-118;
   or
(d) CDR-H1, CDR-H2, and CDR-H3 contained in the following heavy chain variable region (VH), and/or CDR-L1, CDR-L2 and CDR-L3 contained in the following light chain variable region (VL); wherein, the heavy chain variable region (VH) and/or the light chain variable region (VL) has at least one CDR that contains a mutation as compared with the heavy chain variable region and/or light chain variable region as described in any one of (a) to (c), and the mutation is a substitution, deletion or addition of one or several amino acids (e.g., a substitution, deletion or addition of 1, 2 or 3 amino acids); preferably, the substitution is a conservative substitution.

In certain preferred embodiments, the CDRs are defined according to the Chothia, AbM, Kabat or IMGT numbering system; preferably, the VH and/or VL of the antibody or antigen-binding fragment thereof comprise framework regions (FRs) derived from a human or murine immunoglobulin, preferably, the antibody or antigen-binding fragment thereof binds to human ROR1.

In some embodiments, there is provided an antibody or antigen-binding fragment thereof capable of specifically binding to ROR1, and the antibody or antigen-binding fragment thereof comprises: a heavy chain variable region (VH) and/or a light chain variable region (VL).

In certain embodiments, the antibody or antigen-binding fragment thereof of the present invention comprises a heavy chain variable region (VH) and/or a light chain variable region (VL) as follows, wherein CDRs are defined by the Chothia numbering system:
(a) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth SEQ ID NO: 47; and/or,
   a light chain variable region (VL) comprising the following 3 CDRs: CDR-L1 having a sequence as set forth in any one of SEQ ID NOs: 48-55, CDR-L2 having a sequence as set forth in any one of SEQ ID NOs: 56-58, and CDR-L3 having a sequence as set forth in SEQ ID NO: 44;
   or
(b) a heavy chain variable region (VH) comprising the following 3 CDRs: CDR-H1 having a sequence as set forth in SEQ ID NO: 31, CDR-H2 having a sequence as set forth in any one of SEQ ID NOs: 32-34, and CDR-H3 having a sequence as set forth in SEQ ID NO: 20; and/or,
   a light chain variable region (VL) comprising the following 3 CDRs: CDR-L1 having a sequence as set forth in any one of SEQ ID NOs: 21-23 and 77, CDR-L2 having a sequence as set forth in SEQ ID NO: 24 or 25, and CDR-L3 having a sequence as set forth in SEQ ID NO: 15;
   or
(c) a heavy chain variable region (VH) comprising the following 3 CDRs: CDR-H1 having a sequence as set forth in SEQ ID NO: 82, CDR-H2 having a sequence as set forth in SEQ ID NO: 83, and CDR-H3 having a sequence as set forth in SEQ ID NO: 74; and/or,
   a light chain variable region (VL) comprising the following 3 CDRs: CDR-L1 having a sequence as set forth in any one of SEQ ID NOs: 75-77, CDR-L2 having a sequence as set forth in SEQ ID NO: 24 or 78, and CDR-L3 having a sequence as set forth in SEQ ID NO: 15.

In certain embodiments, the antibody or antigen-binding fragment thereof comprises the following CDRs as defined by the Chothia numbering system:
(1) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or, a light chain variable region (VL) comprising the following 3 CDRs: CDR-L1 having a sequence as set forth in SEQ ID NO: 48, CDR-L2 having a sequence as set forth in SEQ ID NO: 56, and CDR-L3 having a sequence as set forth in SEQ ID NO: 44;
   or
(2) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
   a light chain variable region (VL) comprising the following 3 CDRs: CDR-L1 having a sequence as set forth in SEQ ID NO: 48, CDR-L2 having a sequence as set forth in SEQ ID NO: 57, and CDR-L3 having a sequence as set forth in SEQ ID NO: 44;
   or
(3) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
   a light chain variable region (VL) comprising the following 3 CDRs: CDR-L1 having a sequence as set forth in SEQ ID NO: 49, CDR-L2 having a sequence as set forth in SEQ ID NO: 57, and CDR-L3 having a sequence as set forth in SEQ ID NO: 44;
   or
(4) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
   a light chain variable region (VL) comprising the following 3 CDRs: CDR-L1 having a sequence as set forth in SEQ ID NO: 50, CDR-L2 having a sequence as set forth in SEQ ID NO: 58, and CDR-L3 having a sequence as set forth in SEQ ID NO: 44;
   or
(5) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
   a light chain variable region (VL) comprising the following 3 CDRs: CDR-L1 having a sequence as set forth in SEQ ID NO: 51, CDR-L2 having a sequence as set forth in SEQ ID NO: 56, and CDR-L3 having a sequence as set forth in SEQ ID NO: 44;
   or
(6) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
   a light chain variable region (VL) comprising the following 3 CDRs: CDR-L1 having a sequence as set forth in SEQ ID NO: 52, CDR-L2 having a sequence as set forth in SEQ ID NO: 56, and CDR-L3 having a sequence as set forth in SEQ ID NO: 44;
   or
(7) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
   a light chain variable region (VL) comprising the following 3 CDRs: CDR-L1 having a sequence as set forth in SEQ ID NO: 53, CDR-L2 having a sequence as set forth in SEQ ID NO: 56, and CDR-L3 having a sequence as set forth in SEQ ID NO: 44;
   or
(8) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
   a light chain variable region (VL) comprising the following 3 CDRs: CDR-L1 having a sequence as set forth in SEQ ID NO: 54, CDR-L2 having a sequence as set forth in SEQ ID NO: 56, and CDR-L3 having a sequence as set forth in SEQ ID NO: 44;
   or
(9) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
   a light chain variable region (VL) comprising the following 3 CDRs: CDR-L1 having a sequence as set forth in SEQ ID NO: 55, CDR-L2 having a sequence as set forth in SEQ ID NO: 56, and CDR-L3 having a sequence as set forth in SEQ ID NO: 44;
   or
(10) a heavy chain variable region (VH) comprising the following 3 CDRs: CDR-H1 having a sequence as set forth in SEQ ID NO: 31, CDR-H2 having a sequence as set forth in SEQ ID NO: 32, and CDR-H3 having a sequence as set forth in SEQ ID NO: 20; and/or,
   a light chain variable region (VL) comprising the following 3 CDRs: CDR-L1 having a sequence as set forth in SEQ ID NO: 21, CDR-L2 having a sequence as set forth in SEQ ID NO: 24, and CDR-L3 having a sequence as set forth in SEQ ID NO: 15;
   or
(11) a heavy chain variable region (VH) comprising the following 3 CDRs: CDR-H1 having a sequence as set forth in SEQ ID NO: 31, CDR-H2 having a sequence as set forth in SEQ ID NO: 32, and CDR-H3 having a sequence as set forth in SEQ ID NO: 20; and/or,
   a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
   or
(12) a heavy chain variable region (VH) comprising the following 3 CDRs: CDR-H1 having a sequence as set forth in SEQ ID NO: 31, CDR-H2 having a sequence as set forth in SEQ ID NO: 32, and CDR-H3 having a sequence as set forth in SEQ ID NO: 20; and/or,
   a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
   or
(13) a heavy chain variable region (VH) comprising the following 3 CDRs: CDR-H1 having a sequence as set forth in SEQ ID NO: 31, CDR-H2 having a sequence as set forth in SEQ ID NO: 32, and CDR-H3 having a sequence as set forth in SEQ ID NO: 20; and/or,
   a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
   or
(14) a heavy chain variable region (VH) comprising the following 3 CDRs: CDR-H1 having a sequence as set forth in SEQ ID NO: 31, CDR-H2 having a sequence as set forth in SEQ ID NO: 33, and CDR-H3 having a sequence as set forth in SEQ ID NO: 20; and/or,
   a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
   or
(15) a heavy chain variable region (VH) comprising the following 3 CDRs: CDR-H1 having a sequence as set forth in SEQ ID NO: 31, CDR-H2 having a sequence as set forth in SEQ ID NO: 33, and CDR-H3 having a sequence as set forth in SEQ ID NO: 20; and/or,
   a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
   or
(16) a heavy chain variable region (VH) comprising the following 3 CDRs: CDR-H1 having a sequence as set forth in SEQ ID NO: 31, CDR-H2 having a sequence as set forth in SEQ ID NO: 33, and CDR-H3 having a sequence as set forth in SEQ ID NO: 20; and/or,
   a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
   or
(17) a heavy chain variable region (VH) comprising the following 3 CDRs: CDR-H1 having a sequence as set forth in SEQ ID NO: 31, CDR-H2 having a sequence as set forth in SEQ ID NO: 34, and CDR-H3 having a sequence as set forth in SEQ ID NO: 20; and/or,
   a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
   or
(18) a heavy chain variable region (VH) comprising the following 3 CDRs: CDR-H1 having a sequence as set forth in SEQ ID NO: 31, CDR-H2 having a sequence as set forth in SEQ ID NO: 34, and CDR-H3 having a sequence as set forth in SEQ ID NO: 20; and/or,
   a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
   or
(19) a heavy chain variable region (VH) comprising the following 3 CDRs: CDR-H1 having a sequence as set forth in SEQ ID NO: 31, CDR-H2 having a sequence as set forth in SEQ ID NO: 34, and CDR-H3 having a sequence as set forth in SEQ ID NO: 20; and/or,
   a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
   or
(20) a heavy chain variable region (VH) comprising the following 3 CDRs: CDR-H1 having a sequence as set forth in SEQ ID NO: 82, CDR-H2 having a sequence as set forth in SEQ ID NO: 83, and CDR-H3 having a sequence as set forth in SEQ ID NO: 74; and/or,
   a light chain variable region (VL) comprising the following 3 CDRs: CDR-L1 having a sequence as set forth in SEQ ID NO: 75, CDR-L2 having a sequence as set forth in SEQ ID NO: 24, and CDR-L3 having a sequence as set forth in SEQ ID NO: 15;
   or
(21) a heavy chain variable region (VH) comprising the following 3 CDRs: CDR-H1 having a sequence as set forth in SEQ ID NO: 82, CDR-H2 having a sequence as set forth in SEQ ID NO: 83, and CDR-H3 having a sequence as set forth in SEQ ID NO: 74; and/or,
   a light chain variable region (VL) comprising the following 3 CDRs: CDR-L1 having a sequence as set forth in SEQ ID NO: 77, CDR-L2 having a sequence as set forth in SEQ ID NO: 78, and CDR-L3 having a sequence as set forth in SEQ ID NO: 15;
   or
(22) a heavy chain variable region (VH) comprising the following 3 CDRs: CDR-H1 having a sequence as set forth in SEQ ID NO: 82, CDR-H2 having a sequence as set forth in SEQ ID NO: 83, and CDR-H3 having a sequence as set forth in SEQ ID NO: 74; and/or,
   a light chain variable region (VL) comprising the following 3 CDRs: CDR-L1 having a sequence as set forth in SEQ ID NO: 76, CDR-L2 having a sequence as set forth in SEQ ID NO: 78, and CDR-L3 having a sequence as set forth in SEQ ID NO: 15.

In certain embodiments, the antibody or antigen-binding fragment thereof of the present invention comprises a heavy chain variable region (VH) and/or a light chain variable region (VL) as follows, wherein CDRs are defined by the AbM numbering system:
(a) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
   a light chain variable region (VL) comprising the following 3 CDRs: CDR-L1 having a sequence as set forth in any one of SEQ ID NOs: 48-55, CDR-L2 having a sequence as set forth in any one of SEQ ID NOs: 56-58, and CDR-L3 having a sequence as set forth in SEQ ID NO: 44;
   or
(b) a heavy chain variable region (VH) comprising the following 3 CDRs: CDR-H1 having a sequence as set forth in SEQ ID NO: 16, CDR-H2 having a sequence as set forth in any one of SEQ ID NOs: 17-19, and CDR-H3 having a sequence as set forth in SEQ ID NO: 20; and/or,
   a light chain variable region (VL) comprising the following 3 CDRs: CDR-L1 having a sequence as set forth in any one of SEQ ID NOs: 21-23, and 77, CDR-L2 having a sequence as set forth in SEQ ID NO: 24 or 25, and CDR-L3 having a sequence as set forth in SEQ ID NO: 15;
   or
(c) a heavy chain variable region (VH) comprising the following 3 CDRs: CDR-H1 having a sequence as set forth in SEQ ID NO: 72, CDR-H2 having a sequence as set forth in SEQ ID NO: 73, and CDR-H3 having a sequence as set forth in SEQ ID NO: 74; and/or,
   a light chain variable region (VL) comprising the following 3 CDRs: CDR-L1 having a sequence as set forth in any one of SEQ ID NOs: 75-77, CDR-L2 having a sequence as set forth in SEQ ID NO: 24 or 78, and CDR-L3 having a sequence as set forth in SEQ ID NO: 15.

In certain embodiments, the antibody or antigen-binding fragment thereof comprises the following CDRs as defined by the AbM numbering system:
(1) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
   a light chain variable region (VL) comprising the following 3 CDRs: CDR-L1 having a sequence as set forth in SEQ ID NO: 48, CDR-L2 having a sequence as set forth in SEQ ID NO: 56, and CDR-L3 having a sequence as set forth in SEQ ID NO: 44;
   or
(2) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
   a light chain variable region (VL) comprising the following 3 CDRs: CDR-L1 having a sequence as set forth in SEQ ID NO: 48, CDR-L2 having a sequence as set forth in SEQ ID NO: 57, and CDR-L3 having a sequence as set forth in SEQ ID NO: 44;
   or
(3) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
   a light chain variable region (VL) comprising the following 3 CDRs: CDR-L1 having a sequence as set forth in SEQ ID NO: 49, CDR-L2 having a sequence as set forth in SEQ ID NO: 57, and CDR-L3 having a sequence as set forth in SEQ ID NO: 44;
   or
(4) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
   a light chain variable region (VL) comprising the following 3 CDRs: CDR-L1 having a sequence as set forth in SEQ ID NO: 50, CDR-L2 having a sequence as set forth in SEQ ID NO: 58, and CDR-L3 having a sequence as set forth in SEQ ID NO: 44;
   or
(5) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
   a light chain variable region (VL) comprising the following 3 CDRs: CDR-L1 having a sequence as set forth in SEQ ID NO: 51, CDR-L2 having a sequence as set forth in SEQ ID NO: 56, and CDR-L3 having a sequence as set forth in SEQ ID NO: 44;
   or
(6) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
   a light chain variable region (VL) comprising the following 3 CDRs: CDR-L1 having a sequence as set forth in SEQ ID NO: 52, CDR-L2 having a sequence as set forth in SEQ ID NO: 56, and CDR-L3 having a sequence as set forth in SEQ ID NO: 44;
   or
(7) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
   a light chain variable region (VL) comprising the following 3 CDRs: CDR-L1 having a sequence as set forth in SEQ ID NO: 53, CDR-L2 having a sequence as set forth in SEQ ID NO: 56, and CDR-L3 having a sequence as set forth in SEQ ID NO: 44;
   or
(8) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
   a light chain variable region (VL) comprising the following 3 CDRs: CDR-L1 having a sequence as set forth in SEQ ID NO: 54, CDR-L2 having a sequence as set forth in SEQ ID NO: 56, and CDR-L3 having a sequence as set forth in SEQ ID NO: 44;
   or
(9) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
   a light chain variable region (VL) comprising the following 3 CDRs: CDR-L1 having a sequence as set forth in SEQ ID NO: 55, CDR-L2 having a sequence as set forth in SEQ ID NO: 56, and CDR-L3 having a sequence as set forth in SEQ ID NO: 44;
   or
(10) a heavy chain variable region (VH) comprising the following 3 CDRs: CDR-H1 having a sequence as set forth in SEQ ID NO: 16, CDR-H2 having a sequence as set forth in SEQ ID NO: 17, and CDR-H3 having a sequence as set forth in SEQ ID NO: 20; and/or,
   a light chain variable region (VL) comprising the following 3 CDRs: CDR-L1 having a sequence as set forth in SEQ ID NO: 21, CDR-L2 having a sequence as set forth in SEQ ID NO: 24, and CDR-L3 having a sequence as set forth in SEQ ID NO: 15;
   or
(11) a heavy chain variable region (VH) comprising the following 3 CDRs: CDR-H1 having a sequence as set forth in SEQ ID NO: 16, CDR-H2 having a sequence as set forth in SEQ ID NO: 17, and CDR-H3 having a sequence as set forth in SEQ ID NO: 20; and/or,
   the light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
   or
(12) a heavy chain variable region (VH) comprising the following 3 CDRs: CDR-H1 having a sequence as set forth in SEQ ID NO: 16, CDR-H2 having a sequence as set forth in SEQ ID NO: 17, and CDR-H3 having a sequence as set forth in SEQ ID NO: 20; and/or,
   a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
   or
(13) a heavy chain variable region (VH) comprising the following 3 CDRs: CDR-H1 having a sequence as set forth in SEQ ID NO: 16, CDR-H2 having a sequence as set forth in SEQ ID NO: 17, and CDR-H3 having a sequence as set forth in SEQ ID NO: 20; and/or,
   a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
   or
(14) a heavy chain variable region (VH) comprising the following 3 CDRs: CDR-H1 having a sequence as set forth in SEQ ID NO: 16, CDR-H2 having a sequence as set forth in SEQ ID NO: 18, and CDR-H3 having a sequence as set forth in SEQ ID NO: 20; and/or,
   a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
   or
(15) a heavy chain variable region (VH) comprising the following 3 CDRs: CDR-H1 having a sequence as set forth in SEQ ID NO: 16, CDR-H2 having a sequence as set forth in SEQ ID NO: 18, and CDR-H3 having a sequence as set forth in SEQ ID NO: 20; and/or,
   a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
   or
(16) a heavy chain variable region (VH) comprising the following 3 CDRs: CDR-H1 having a sequence as set forth in SEQ ID NO: 16, CDR-H2 having a sequence as set forth in SEQ ID NO: 18, and CDR-H3 having a sequence as set forth in SEQ ID NO: 20; and/or,
   a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
   or
(17) a heavy chain variable region (VH) comprising the following 3 CDRs: CDR-H1 having a sequence as set forth in SEQ ID NO: 16, CDR-H2 having a sequence as set forth in SEQ ID NO: 19, and CDR-H3 having a sequence as set forth in SEQ ID NO: 20; and/or,
   a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
   or
(18) a heavy chain variable region (VH) comprising the following 3 CDRs: CDR-H1 having a sequence as set forth in SEQ ID NO: 16, CDR-H2 having a sequence as set forth in SEQ ID NO: 19, and CDR-H3 having a sequence as set forth in SEQ ID NO: 20; and/or,
   a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
   or
(19) a heavy chain variable region (VH) comprising the following 3 CDRs: CDR-H1 having a sequence as set forth in SEQ ID NO: 16, CDR-H2 having a sequence as set forth in SEQ ID NO: 19, and CDR-H3 having a sequence as set forth in SEQ ID NO: 20; and/or,
   a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
   or
(20) a heavy chain variable region (VH) comprising the following 3 CDRs: CDR-H1 having a sequence as set forth in SEQ ID NO: 72, CDR-H2 having a sequence as set forth in SEQ ID NO: 73, and CDR-H3 having a sequence as set forth in SEQ ID NO: 74; and/or,
   a light chain variable region (VL) comprising the following 3 CDRs: CDR-L1 having a sequence as set forth in SEQ ID NO: 75, CDR-L2 having a sequence as set forth in SEQ ID NO: 24, and CDR-L3 having a sequence as set forth in SEQ ID NO: 15;
   or
(21) a heavy chain variable region (VH) comprising the following 3 CDRs: CDR-H1 having a sequence as set forth in SEQ ID NO: 72, CDR-H2 having a sequence as set forth in SEQ ID NO: 73, and CDR-H3 having a sequence as set forth in SEQ ID NO: 74; and/or,
   a light chain variable region (VL) comprising the following 3 CDRs: CDR-L1 having a sequence as set forth in SEQ ID NO: 77, CDR-L2 having a sequence as set forth in SEQ ID NO: 78, and CDR-L3 having a sequence as set forth in SEQ ID NO: 15;
   or
(22) a heavy chain variable region (VH) comprising the following 3 CDRs: CDR-H1 having a sequence as set forth in SEQ ID NO: 72, CDR-H2 having a sequence as set forth in SEQ ID NO: 73, and CDR-H3 having a sequence as set forth in SEQ ID NO: 74; and/or,
   a light chain variable region (VL) comprising the following 3 CDRs: CDR-L1 having a sequence as set forth in SEQ ID NO: 76, CDR-L2 having a sequence as set forth in SEQ ID NO: 78, and CDR-L3 having a sequence as set forth in SEQ ID NO: 15.

In certain embodiments, the antibody or antigen-binding fragment thereof of the present invention comprises a heavy chain variable region (VH) and/or a light chain variable region (VL) as follows, wherein CDRs are defined by the Kabat numbering system:
(a) a heavy chain variable region (VH) comprising the following 3 CDRs: CDR-H1 having a sequence as set forth in SEQ ID NO: 60, CDR-H2 having a sequence as set forth in any one of SEQ ID NOs: 61-63, CDR-H3 having a sequence as set forth in SEQ ID NO: 47; and/or,
   a light chain variable region (VL) comprising the following 3 CDRs: CDR-L1 having a sequence as set forth in any one of SEQ ID NOs: 48-55, CDR-L2 having a sequence as set forth in any one of SEQ ID NOs: 56-58, CDR-L3 having a sequence as set forth in SEQ ID NO: 44;
   or
(b) a heavy chain variable region (VH) comprising the following 3 CDRs: CDR-H1 having a sequence as set forth in SEQ ID NO: 27, CDR-H2 having a sequence as set forth in any one of SEQ ID NOs: 28-30, CDR-H3 having a sequence as set forth in SEQ ID NO: 20; and/or,
   a light chain variable region (VL) comprising the following 3 CDRs: CDR-L1 having a sequence as set forth in any one of SEQ ID NOs: 21-23, and 77, CDR-L2 having a sequence as set forth in any one of SEQ ID NOs: 24-25, CDR-L3 having a sequence as set forth in SEQ ID NO: 15;
   or
(c) a heavy chain variable region (VH) comprising the following 3 CDRs: CDR-H1 having a sequence as set forth in SEQ ID NO: 79, CDR-H2 having a sequence as set forth in SEQ ID NO: 80 or 81, CDR-H3 having a sequence as set forth in SEQ ID NO: 74; and/or,
   a light chain variable region (VL) comprising the following 3 CDRs: CDR-L1 having a sequence as set forth in any one of SEQ ID NOs: 75-77, CDR-L2 having a sequence as set forth in SEQ ID NO: 24 or 78, and CDR-L3 having a sequence as set forth in SEQ ID NO: 15.

In certain embodiments, the antibody or antigen-binding fragment thereof comprises the following CDRs as defined by the Kabat numbering system:
(1) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
   a light chain variable region (VL) comprising the following 3 CDRs: CDR-L1 having a sequence as set forth in SEQ ID NO: 48, CDR-L2 having a sequence as set forth in SEQ ID NO: 56, and CDR-L3 having a sequence as set forth in SEQ ID NO: 44;
   or
(2) a heavy chain variable region (VH) comprising the following 3 CDRs: CDR-H1 having a sequence as set forth in SEQ ID NO: 60, CDR-H2 having a sequence as set forth in SEQ ID NO: 62, and CDR-H3 having a sequence as set forth in SEQ ID NO: 47; and/or,
   a light chain variable region (VL) comprising the following 3 CDRs: CDR-L1 having a sequence as set forth in SEQ ID NO: 48, CDR-L2 having a sequence as set forth in SEQ ID NO: 56, and CDR-L3 having a sequence as set forth in SEQ ID NO: 44;
   or
(3) a heavy chain variable region (VH) comprising the following 3 CDRs: CDR-H1 having a sequence as set forth in SEQ ID NO: 60, CDR-H2 having a sequence as set forth in SEQ ID NO: 62, and CDR-H3 having a sequence as set forth in SEQ ID NO: 47; and/or,
   a light chain variable region (VL) comprising the following 3 CDRs: CDR-L1 having a sequence as set forth in SEQ ID NO: 48, CDR-L2 having a sequence as set forth in SEQ ID NO: 57, and CDR-L3 having a sequence as set forth in SEQ ID NO: 44;
   or
(4) a heavy chain variable region (VH) comprising the following 3 CDRs: CDR-H1 having a sequence as set forth in SEQ ID NO: 60, CDR-H2 having a sequence as set forth in SEQ ID NO: 62, and CDR-H3 having a sequence as set forth in SEQ ID NO: 47; and/or,
   a light chain variable region (VL) comprising the following 3 CDRs: CDR-L1 having a sequence as set forth in SEQ ID NO: 49, CDR-L2 having a sequence as set forth in SEQ ID NO: 57, and CDR-L3 having a sequence as set forth in SEQ ID NO: 44;
   or
(5) a heavy chain variable region (VH) comprising the following 3 CDRs: CDR-H1 having a sequence as set forth in SEQ ID NO: 60, CDR-H2 having a sequence as set forth in SEQ ID NO: 62, and CDR-H3 having a sequence as set forth in SEQ ID NO: 47; and/or,
   a light chain variable region (VL) comprising the following 3 CDRs: CDR-L1 having a sequence as set forth in SEQ ID NO: 50, CDR-L2 having a sequence as set forth in SEQ ID NO: 58, and CDR-L3 having a sequence as set forth in SEQ ID NO: 44;
   or
(6) a heavy chain variable region (VH) comprising the following 3 CDRs: CDR-H1 having a sequence as set forth in SEQ ID NO: 60, CDR-H2 having a sequence as set forth in SEQ ID NO: 62, and CDR-H3 having a sequence as set forth in SEQ ID NO: 47; and/or,
   a light chain variable region (VL) comprising the following 3 CDRs: CDR-L1 having a sequence as set forth in SEQ ID NO: 51, CDR-L2 having a sequence as set forth in SEQ ID NO: 56, and CDR-L3 having a sequence as set forth in SEQ ID NO: 44;
   or
(7) a heavy chain variable region (VH) comprising the following 3 CDRs: CDR-H1 having a sequence as set forth in SEQ ID NO: 60, CDR-H2 having a sequence as set forth in SEQ ID NO: 63, and CDR-H3 having a sequence as set forth in SEQ ID NO: 47; and/or,
   a light chain variable region (VL) comprising the following 3 CDRs: CDR-L1 having a sequence as set forth in SEQ ID NO: 48, CDR-L2 having a sequence as set forth in SEQ ID NO: 56, and CDR-L3 having a sequence as set forth in SEQ ID NO: 44;
   or
(8) a heavy chain variable region (VH) comprising the following 3 CDRs: CDR-H1 having a sequence as set forth in SEQ ID NO: 60, CDR-H2 having a sequence as set forth in SEQ ID NO: 63, and CDR-H3 having a sequence as set forth in SEQ ID NO: 47; and/or,
   a light chain variable region (VL) comprising the following 3 CDRs: CDR-L1 having a sequence as set forth in SEQ ID NO: 48, CDR-L2 having a sequence as set forth in SEQ ID NO: 57, and CDR-L3 having a sequence as set forth in SEQ ID NO: 44;
   or
(9) a heavy chain variable region (VH) comprising the following 3 CDRs: CDR-H1 having a sequence as set forth in SEQ ID NO: 60, CDR-H2 having a sequence as set forth in SEQ ID NO: 63, and CDR-H3 having a sequence as set forth in SEQ ID NO: 47; and/or,
   a light chain variable region (VL) comprising the following 3 CDRs: CDR-L1 having a sequence as set forth in SEQ ID NO: 49, CDR-L2 having a sequence as set forth in SEQ ID NO: 57, and CDR-L3 having a sequence as set forth in SEQ ID NO: 44;
   or
(10) a heavy chain variable region (VH) comprising the following 3 CDRs: CDR-H1 having a sequence as set forth in SEQ ID NO: 60, CDR-H2 having a sequence as set forth in SEQ ID NO: 63, and CDR-H3 having a sequence as set forth in SEQ ID NO: 47; and/or,
   a light chain variable region (VL) comprising the following 3 CDRs: CDR-L1 having a sequence as set forth in SEQ ID NO: 50, CDR-L2 having a sequence as set forth in SEQ ID NO: 58, and CDR-L3 having a sequence as set forth in SEQ ID NO: 44;
   or
(11) a heavy chain variable region (VH) comprising the following 3 CDRs: CDR-H1 having a sequence as set forth in SEQ ID NO: 60, CDR-H2 having a sequence as set forth in SEQ ID NO: 63, and CDR-H3 having a sequence as set forth in SEQ ID NO: 47; and/or,
   a light chain variable region (VL) comprising the following 3 CDRs: CDR-L1 having a sequence as set forth in SEQ ID NO: 51, CDR-L2 having a sequence as set forth in SEQ ID NO: 56, and CDR-L3 having a sequence as set forth in SEQ ID NO: 44;
   or
(12) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
   a light chain variable region (VL) comprising the following 3 CDRs: CDR-L1 having a sequence as set forth in SEQ ID NO: 48, CDR-L2 having a sequence as set forth in SEQ ID NO: 57, and CDR-L3 having a sequence as set forth in SEQ ID NO: 44;
   or
(13) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
   a light chain variable region (VL) comprising the following 3 CDRs: CDR-L1 having a sequence as set forth in SEQ ID NO: 49, CDR-L2 having a sequence as set forth in SEQ ID NO: 57, and CDR-L3 having a sequence as set forth in SEQ ID NO: 44;
   or
(14) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
   a light chain variable region (VL) comprising the following 3 CDRs: CDR-L1 having a sequence as set forth in SEQ ID NO: 50, CDR-L2 having a sequence as set forth in SEQ ID NO: 58, and CDR-L3 having a sequence as set forth in SEQ ID NO: 44;
   or
(15) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
   a light chain variable region (VL) comprising the following 3 CDRs: CDR-L1 having a sequence as set forth in SEQ ID NO: 51, CDR-L2 having a sequence as set forth in SEQ ID NO: 56, and CDR-L3 having a sequence as set forth in SEQ ID NO: 44;
   or
(16) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
   a light chain variable region (VL) comprising the following 3 CDRs: CDR-L1 having a sequence as set forth in SEQ ID NO: 52, CDR-L2 having a sequence as set forth in SEQ ID NO: 56, and CDR-L3 having a sequence as set forth in SEQ ID NO: 44;
   or
(17) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
   a light chain variable region (VL) comprising the following 3 CDRs: CDR-L1 having a sequence as set forth in SEQ ID NO: 53, CDR-L2 having a sequence as set forth in SEQ ID NO: 56, and CDR-L3 having a sequence as set forth in SEQ ID NO: 44;
   or
(18) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
   a light chain variable region (VL) comprising the following 3 CDRs: CDR-L1 having a sequence as set forth in SEQ ID NO: 54, CDR-L2 having a sequence as set forth in SEQ ID NO: 56, and CDR-L3 having a sequence as set forth in SEQ ID NO: 44;
   or
(19) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
   a light chain variable region (VL) comprising the following 3 CDRs: CDR-L1 having a sequence as set forth in SEQ ID NO: 55, CDR-L2 having a sequence as set forth in SEQ ID NO: 56, and CDR-L3 having a sequence as set forth in SEQ ID NO: 44;
   or
(20) a heavy chain variable region (VH) comprising the following 3 CDRs: CDR-H1 having a sequence as set forth in SEQ ID NO: 27, CDR-H2 having a sequence as set forth in SEQ ID NO: 28, and CDR-H3 having a sequence as set forth in SEQ ID NO: 20; and/or,
   a light chain variable region (VL) comprising the following 3 CDRs: CDR-L1 having a sequence as set forth in SEQ ID NO: 21, CDR-L2 having a sequence as set forth in SEQ ID NO: 24, and CDR-L3 having a sequence as set forth in SEQ ID NO: 15;
   or
(21) a heavy chain variable region (VH) comprising the following 3 CDRs: CDR-H1 having a sequence as set forth in SEQ ID NO: 27, CDR-H2 having a sequence as set forth in SEQ ID NO: 28, and CDR-H3 having a sequence as set forth in SEQ ID NO: 20; and/or,
   a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
   or
(22) a heavy chain variable region (VH) comprising the following 3 CDRs: CDR-H1 having a sequence as set forth in SEQ ID NO: 27, CDR-H2 having a sequence as set forth in SEQ ID NO: 28, and CDR-H3 having a sequence as set forth in SEQ ID NO: 20; and/or,
   a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
   or
(23) a heavy chain variable region (VH) comprising the following 3 CDRs: CDR-H1 having a sequence as set forth in SEQ ID NO: 27, CDR-H2 having a sequence as set forth in SEQ ID NO: 28, and CDR-H3 having a sequence as set forth in SEQ ID NO: 20; and/or,
   a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
   or
(24) a heavy chain variable region (VH) comprising the following 3 CDRs: CDR-H1 having a sequence as set forth in SEQ ID NO: 27, CDR-H2 having a sequence as set forth in SEQ ID NO: 29, and CDR-H3 having a sequence as set forth in SEQ ID NO: 20; and/or,
   a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
   or
(25) a heavy chain variable region (VH) comprising the following 3 CDRs: CDR-H1 having a sequence as set forth in SEQ ID NO: 27, CDR-H2 having a sequence as set forth in SEQ ID NO: 29, and CDR-H3 having a sequence as set forth in SEQ ID NO: 20; and/or,
   a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
   or
(26) a heavy chain variable region (VH) comprising the following 3 CDRs: CDR-H1 having a sequence as set forth in SEQ ID NO: 27, CDR-H2 having a sequence as set forth in SEQ ID NO: 29, and CDR-H3 having a sequence as set forth in SEQ ID NO: 20; and/or,
   a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
   or
(27) a heavy chain variable region (VH) comprising the following 3 CDRs: CDR-H1 having a sequence as set forth in SEQ ID NO: 27, CDR-H2 having a sequence as set forth in SEQ ID NO: 30, and CDR-H3 having a sequence as set forth in SEQ ID NO: 20; and/or,
   a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
   or
(28) a heavy chain variable region (VH) comprising the following 3 CDRs: CDR-H1 having a sequence as set forth in SEQ ID NO: 27, CDR-H2 having a sequence as set forth in SEQ ID NO: 30, and CDR-H3 having a sequence as set forth in SEQ ID NO: 20; and/or,
   a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
   or
(29) a heavy chain variable region (VH) comprising the following 3 CDRs: CDR-H1 having a sequence as set forth in SEQ ID NO: 27, CDR-H2 having a sequence as set forth in SEQ ID NO: 30, and CDR-H3 having a sequence as set forth in SEQ ID NO: 20; and/or,
   a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
   or
(30) a heavy chain variable region (VH) comprising the following 3 CDRs: CDR-H1 having a sequence as set forth in SEQ ID NO: 79, CDR-H2 having a sequence as set forth in SEQ ID NO: 80, and CDR-H3 having a sequence as set forth in SEQ ID NO: 74; and/or,
   a light chain variable region (VL) comprising the following 3 CDRs: CDR-L1 having a sequence as set forth in SEQ ID NO: 75, CDR-L2 having a sequence as set forth in SEQ ID NO: 24, and CDR-L3 having a sequence as set forth in SEQ ID NO: 15;
   or
(31) a heavy chain variable region (VH) comprising the following 3 CDRs: CDR-H1 having a sequence as set forth in SEQ ID NO: 79, CDR-H2 having a sequence as set forth in SEQ ID NO: 81, and CDR-H3 having a sequence as set forth in SEQ ID NO: 74; and/or,
   a light chain variable region (VL) comprising the following 3 CDRs: CDR-L1 having a sequence as set forth in SEQ ID NO: 77, CDR-L2 having a sequence as set forth in SEQ ID NO: 78, and CDR-L3 having a sequence as set forth in SEQ ID NO: 15;
   or
(32) a heavy chain variable region (VH) comprising the following 3 CDRs: CDR-H1 having a sequence as set forth in SEQ ID NO: 79, CDR-H2 having a sequence as set forth in SEQ ID NO: 81, and CDR-H3 having a sequence as set forth in SEQ ID NO: 74; and/or,
   a light chain variable region (VL) comprising the following 3 CDRs: CDR-L1 having a sequence as set forth in SEQ ID NO: 76, CDR-L2 having a sequence as set forth in SEQ ID NO: 78, and CDR-L3 having a sequence as set forth in SEQ ID NO: 15;
   or
(33) a heavy chain variable region (VH) comprising the following 3 CDRs: CDR-H1 having a sequence as set forth in SEQ ID NO: 79, CDR-H2 having a sequence as set forth in SEQ ID NO: 80, and CDR-H3 having a sequence as set forth in SEQ ID NO: 74; and/or,
   a light chain variable region (VL) comprising the following 3 CDRs: CDR-L1 having a sequence as set forth in SEQ ID NO: 77, CDR-L2 having a sequence as set forth in SEQ ID NO: 78, and CDR-L3 having a sequence as set forth in SEQ ID NO: 15;
   or
(34) a heavy chain variable region (VH) comprising the following 3 CDRs: CDR-H1 having a sequence as set forth in SEQ ID NO: 79, CDR-H2 having a sequence as set forth in SEQ ID NO: 80, and CDR-H3 having a sequence as set forth in SEQ ID NO: 74; and/or,
   a light chain variable region (VL) comprising the following 3 CDRs: CDR-L1 having a sequence as set forth in SEQ ID NO: 76, CDR-L2 having a sequence as set forth in SEQ ID NO: 78, and CDR-L3 having a sequence as set forth in SEQ ID NO: 15.

In certain embodiments, the antibody or antigen-binding fragment thereof of the present invention comprises a heavy chain variable region (VH) and/or a light chain variable region (VL) as follows, wherein CDRs are defined by the IMGT numbering system:
(a) a heavy chain variable region (VH) comprising the following 3 CDRs: CDR-H1 having a sequence as set forth in SEQ ID NO: 35, CDR-H2 having a sequence as set forth in SEQ ID NO: 36, and CDR-H3 having a sequence as set forth in SEQ ID NO: 37; and/or,
   a light chain variable region (VL) comprising the following 3 CDRs: CDR-L1 having a sequence as set forth in any one of SEQ ID NOs: 38-42, CDR-L2 having a sequence as set forth in SEQ ID NO: 43, and CDR-L3 having a sequence as set forth in SEQ ID NO: 44;
   or
(b) a heavy chain variable region (VH) comprising the following 3 CDRs: CDR-H1 having a sequence as set forth in SEQ ID NO: 7, CDR-H2 having a sequence as set forth in any one of SEQ ID NOs: 8-10, and CDR-H3 having a sequence as set forth in SEQ ID NO: 11; and/or,
   a light chain variable region (VL) comprising the following 3 CDRs: CDR-L1 having a sequence as set forth in SEQ ID NO: 12 or 13, CDR-L2 having a sequence as set forth in SEQ ID NO: 14, and CDR-L3 having a sequence as set forth in SEQ ID NO: 15;
   or
(c) a heavy chain variable region (VH) comprising the following 3 CDRs: CDR-H1 having a sequence as set forth in SEQ ID NO: 66, CDR-H2 having a sequence as set forth in SEQ ID NO: 67, and CDR-H3 having a sequence as set forth in SEQ ID NO: 68; and/or,
   a light chain variable region (VL) comprising the following 3 CDRs: CDR-L1 having a sequence as set forth in SEQ ID NO: 69 or 70, CDR-L2 having a sequence as set forth in SEQ ID NO: 71, and CDR-L3 having a sequence as set forth in SEQ ID NO: 15.

In certain embodiments, the antibody or antigen-binding fragment thereof comprises the following CDRs as defined by the IMGT numbering system:
(1) a heavy chain variable region (VH) comprising the following 3 CDRs: CDR-H1 having a sequence as set forth in SEQ ID NO: 35, CDR-H2 having a sequence as set forth in SEQ ID NO: 36, and CDR-H3 having a sequence as set forth in SEQ ID NO: 37; and/or,
   a light chain variable region (VL) comprising the following 3 CDRs: CDR-L1 having a sequence as set forth in SEQ ID NO: 38, CDR-L2 having a sequence as set forth in SEQ ID NO: 43, and CDR-L3 having a sequence as set forth in SEQ ID NO: 44;
   or
(2) a heavy chain variable region (VH) comprising the following 3 CDRs: CDR-H1 having a sequence as set forth in SEQ ID NO: 35, CDR-H2 having a sequence as set forth in SEQ ID NO: 36, and CDR-H3 having a sequence as set forth in SEQ ID NO: 37; and/or,
   a light chain variable region (VL) comprising the following 3 CDRs: CDR-L1 having a sequence as set forth in SEQ ID NO: 39, CDR-L2 having a sequence as set forth in SEQ ID NO: 43, and CDR-L3 having a sequence as set forth in SEQ ID NO: 44;
   or
(3) a heavy chain variable region (VH) comprising the following 3 CDRs: CDR-H1 having a sequence as set forth in SEQ ID NO: 35, CDR-H2 having a sequence as set forth in SEQ ID NO: 36, and CDR-H3 having a sequence as set forth in SEQ ID NO: 37; and/or,
   a light chain variable region (VL) comprising the following 3 CDRs: CDR-L1 having a sequence as set forth in SEQ ID NO: 40, CDR-L2 having a sequence as set forth in SEQ ID NO: 43, and CDR-L3 having a sequence as set forth in SEQ ID NO: 44;
   or
(4) a heavy chain variable region (VH) comprising the following 3 CDRs: CDR-H1 having a sequence as set forth in SEQ ID NO: 35, CDR-H2 having a sequence as set forth in SEQ ID NO: 36, and CDR-H3 having a sequence as set forth in SEQ ID NO: 37; and/or,
   a light chain variable region (VL) comprising the following 3 CDRs: CDR-L1 having a sequence as set forth in SEQ ID NO: 41, CDR-L2 having a sequence as set forth in SEQ ID NO: 43, and CDR-L3 having a sequence as set forth in SEQ ID NO: 44;
   or
(5) a heavy chain variable region (VH) comprising the following 3 CDRs: CDR-H1 having a sequence as set forth in SEQ ID NO: 35, CDR-H2 having a sequence as set forth in SEQ ID NO: 36, and CDR-H3 having a sequence as set forth in SEQ ID NO: 37; and/or,
   a light chain variable region (VL) comprising the following 3 CDRs: CDR-L1 having a sequence as set forth in SEQ ID NO: 42, CDR-L2 having a sequence as set forth in SEQ ID NO: 43, and CDR-L3 having a sequence as set forth in SEQ ID NO: 44;
   or
(6) a heavy chain variable region (VH) comprising the following 3 CDRs: CDR-H1 having a sequence as set forth in SEQ ID NO: 7, CDR-H2 having a sequence as set forth in SEQ ID NO: 8, and CDR-H3 having a sequence as set forth in SEQ ID NO: 11; and/or,
   a light chain variable region (VL) comprising the following 3 CDRs: CDR-L1 having a sequence as set forth in SEQ ID NO: 12, CDR-L2 having a sequence as set forth in SEQ ID NO: 14, and CDR-L3 having a sequence as set forth in SEQ ID NO: 15;
   or
(7) a heavy chain variable region (VH) comprising the following 3 CDRs: CDR-H1 having a sequence as set forth in SEQ ID NO: 7, CDR-H2 having a sequence as set forth in SEQ ID NO: 8, and CDR-H3 having a sequence as set forth in SEQ ID NO: 11; and/or,
   a light chain variable region (VL) comprising the following 3 CDRs: CDR-L1 having a sequence as set forth in SEQ ID NO: 13, CDR-L2 having a sequence as set forth in SEQ ID NO: 14, and CDR-L3 having a sequence as set forth in SEQ ID NO: 15;
   or
(8) a heavy chain variable region (VH) comprising the following 3 CDRs: CDR-H1 having a sequence as set forth in SEQ ID NO: 7, CDR-H2 having a sequence as set forth in SEQ ID NO: 9, and CDR-H3 having a sequence as set forth in SEQ ID NO: 11; and/or,
   a light chain variable region (VL) comprising the following 3 CDRs: CDR-L1 having a sequence as set forth in SEQ ID NO: 12, CDR-L2 having a sequence as set forth in SEQ ID NO: 14, and CDR-L3 having a sequence as set forth in SEQ ID NO: 15;
   or
(9) a heavy chain variable region (VH) comprising the following 3 CDRs: CDR-H1 having a sequence as set forth in SEQ ID NO: 7, CDR-H2 having a sequence as set forth in SEQ ID NO: 9, and CDR-H3 having a sequence as set forth in SEQ ID NO: 11; and/or,
   a light chain variable region (VL) comprising the following 3 CDRs: CDR-L1 having a sequence as set forth in SEQ ID NO: 13, CDR-L2 having a sequence as set forth in SEQ ID NO: 14, and CDR-L3 having a sequence as set forth in SEQ ID NO: 15;
   or
(10) a heavy chain variable region (VH) comprising the following 3 CDRs: CDR-H1 having a sequence as set forth in SEQ ID NO: 7, CDR-H2 having a sequence as set forth in SEQ ID NO: 10, and CDR-H3 having a sequence as set forth in SEQ ID NO: 11; and/or,
   a light chain variable region (VL) comprising the following 3 CDRs: CDR-L1 having a sequence as set forth in SEQ ID NO: 12, CDR-L2 having a sequence as set forth in SEQ ID NO: 14, and CDR-L3 having a sequence as set forth in SEQ ID NO: 15;
   or
(11) a heavy chain variable region (VH) comprising the following 3 CDRs: CDR-H1 having a sequence as set forth in SEQ ID NO: 7, CDR-H2 having a sequence as set forth in SEQ ID NO: 10, and CDR-H3 having a sequence as set forth in SEQ ID NO: 11; and/or,
   a light chain variable region (VL) comprising the following 3 CDRs: CDR-L1 having a sequence as set forth in SEQ ID NO: 13, CDR-L2 having a sequence as set forth in SEQ ID NO: 14, and CDR-L3 having a sequence as set forth in SEQ ID NO: 15;
   or
(12) a heavy chain variable region (VH) comprising the following 3 CDRs: CDR-H1 having a sequence as set forth in SEQ ID NO: 66, CDR-H2 having a sequence as set forth in SEQ ID NO: 67, and CDR-H3 having a sequence as set forth in SEQ ID NO: 68; and/or,
   a light chain variable region (VL) comprising the following 3 CDRs: CDR-L1 having a sequence as set forth in SEQ ID NO: 69, CDR-L2 having a sequence as set forth in SEQ ID NO: 71, and CDR-L3 having a sequence as set forth in SEQ ID NO: 15;
   or
(13) a heavy chain variable region (VH) comprising the following 3 CDRs: CDR-H1 having a sequence as set forth in SEQ ID NO: 66, CDR-H2 having a sequence as set forth in SEQ ID NO: 67, and CDR-H3 having a sequence as set forth in SEQ ID NO: 68; and/or,
   a light chain variable region (VL) comprising the following 3 CDRs: CDR-L1 having a sequence as set forth in SEQ ID NO: 70, CDR-L2 having a sequence as set forth in SEQ ID NO: 71, and CDR-L3 having a sequence as set forth in SEQ ID NO: 15.

In certain embodiments, the antibody or antigen-binding fragment thereof of the present invention comprises a heavy chain variable region (VH) and/or a light chain variable region (VL) as follows, wherein the heavy chain variable region (VH) and/or light chain variable region (VL) has at least one CDR with a mutation as compared with the heavy chain variable region and/or light chain variable region as described in any one of the aforementioned items under definitions of Chothia, AbM, Kabat or IMGT, and the mutation is a substitution, deletion or addition of one or several amino acids (e.g., a substitution, deletion or addition of 1, 2 or 3 amino acids); preferably, the substitution is a conservative substitution.

In certain preferred embodiments, the VH of the antibody or antigen-binding fragment thereof of the present invention comprises a heavy chain variable region (VH) framework region (FR) derived from a murine immunoglobulin, and/or the VL of the antibody or antigen-binding fragment thereof comprises a light chain variable region (VL) framework region (FR) derived from a murine immunoglobulin. Accordingly, in certain preferred embodiments, the antibody or antigen-binding fragment thereof of the present invention is of murine origin.

In certain preferred embodiments, the VH of the antibody or antigen-binding fragment thereof of the present invention comprises a heavy chain variable region (VH) framework region (FR) derived from a human immunoglobulin, and/or the VL of the antibody or antigen-binding fragment thereof comprises a light chain variable region (VL) framework region (FR) derived from a human immunoglobulin. Accordingly, in certain preferred embodiments, the antibody or antigen-binding fragment thereof of the present invention is humanized. In such embodiments, the heavy chain variable region FR and/or the light chain variable region FR of the antibody or antigen-binding fragment thereof of the present invention may comprise one or more non-human (e.g., murine) amino acid residues, for example, the heavy chain framework region FR and/or the light chain framework region FR may comprise one or more amino acid backmutations with corresponding murine amino acid residues in these backmutations.

In certain preferred embodiments, the antibody or antigen-binding fragment thereof of the present invention comprises:
(a) a heavy chain framework region of a human immunoglobulin, or a variant thereof, in which the variant comprises a conservative substitution of up to 20 amino acids (e.g., a conservative substitution of up to 15, up to 10, or up to 5 amino acids; for example, a conservative substitution of 1, 2, 3, 4 or 5 amino acids) as compared to the germline antibody gene sequence from which it is derived; and/or
(b) a light chain framework region of a human immunoglobulin, or a variant thereof, in which the variant comprises a conservative substitution of up to 20 amino acids (e.g., a conservative substitution of up to 15, up to 10, or up to 5 amino acids; for example, a conservative substitution of 1, 2, 3, 4 or 5 amino acids) as compared to the germline antibody gene sequence from which it is derived.

In certain preferred embodiments, the antibody or antigen-binding fragment thereof of the present invention has a humanization degree of at least 75%, at least 80%, at least 85%, at least 90%, at least 91%, at least 92%, at least 93% %, at least 94%, at least 95%, at least 96%, at least 97%, at least 98%, at least 99%, or 100%.

In certain embodiments, the antibody or antigen-binding fragment thereof of the present invention comprises:
(a) a heavy chain variable region (VH) comprising an amino acid sequence selected from the group consisting of:
   (i) a sequence as set forth in any one of SEQ ID NOs: 1, 3, 5, 84-86, 102-106, and 111-115;
   (ii) a sequence comprising a substitution, deletion or addition of one or several amino acids (e.g., a substitution, deletion or addition of 1, 2, 3, 4 or 5 amino acids) as compared to a sequence as set forth in any one of SEQ ID NOs: 1, 3, 5, 84-86, 102-106, and 111-115; or
   (iii) a sequence having a sequence identity of at least 75%, at least 80%, at least 85%, at least 90%, at least 91%, at least 92%, at least 93%, at least 94%, at least 95%, at least 96%, at least 97%, at least 98%, at least 99%, or 100% as compared to a sequence as set forth in any one of SEQ ID NOs: 1, 3, 5, 84-86, 102-106, and 111-115;
   and/or
(b) a light chain variable region (VL) comprising an amino acid sequence selected from the group consisting of:
   (iv) a sequence as set forth in any one of SEQ ID NOs: 2, 4, 6, 87-101, 107-110, and 116-118;
   (v) a sequence comprising a substitution, deletion or addition of one or several amino acids (e.g., a substitution, deletion or addition of 1, 2, 3, 4 or 5 amino acids) as compared to a sequence as set forth in any one of SEQ ID NOs: 2, 4, 6, 87-101, 107-110, and 116-118; or
   (vi) a sequence having a sequence identity of at least 75%, at least 80%, at least 85%, at least 90%, at least 91%, at least 92%, at least 93%, at least 94%, at least 95%, at least 96%, at least 97%, at least 98%, at least 99%, or 100% as compared to a sequence as set forth in any one of SEQ ID NOs: 2, 4, 6, 87-101, 107-110, and 116-118.

In certain embodiments, the antibody or antigen-binding fragment thereof of the present invention comprises a heavy chain variable region (VH) and/or a light chain variable region (VL) as follows: a VH as set forth in any one of SEQ ID NOs: 1, 3, 5, 84-86, 102-106, and 111-115, and/or, a VL as set forth in any one of SEQ ID NOs: 2, 4, 6, 87-101, 107-110, and 116-118.

In certain embodiments, the antibody or antigen-binding fragment thereof of the present invention comprises a heavy chain variable region (VH) and/or a light chain variable region (VL) as follows: a VH as set forth in any one of SEQ ID NOs: 86, 102, and 114, and/or, a VL as set forth in any one of SEQ ID NOs: 92, 107, and 117.

In certain embodiments, the VH and/or VL of the antibody or antigen-binding fragment thereof has a sequence identity of at least 70%, at least 80%, at least 85%, at least 90%, at least 91%, at least 92%, at least 93%, at least 94%, at least 95%, at least 96 %, at least 97%, at least 98%, at least 99%, or 100% as compared to the VH sequence as set forth in any one of SEQ ID NOs: 86, 102, and 114 and/or the VL sequence as set forth in any one of SEQ ID NOs: 92, 107, and 117.

In certain embodiments, the VH and/or VL of the antibody or antigen-binding fragment thereof comprises a substitution, deletion or addition of one or several amino acids (e.g., a substitution, deletion or addition of 1, 2, 3, 4 or 5 amino acids) as compared to the VH sequence as set forth in any one of SEQ ID NOs: 86, 102, and 114 and/or the VL sequence as set forth in any one of SEQ ID NOs: 92, 107, and 117. In preferred embodiments, the substitution is a conservative substitution.

In certain preferred embodiments, the antibody or antigen-binding fragment thereof of the present invention comprises:
(1) a VH as set forth in SEQ ID NO: 1 and a VL as set forth in SEQ ID NO: 2;
(2) a VH as set forth in SEQ ID NO: 3 and a VL as set forth in SEQ ID NO: 4;
(3) a VH as set forth in SEQ ID NO: 5 and a VL as set forth in SEQ ID NO: 6;
(4) a VH as set forth in SEQ ID NO: 84 and a VL as set forth in SEQ ID NO: 87;
(5) a VH as set forth in SEQ ID NO: 84 and a VL as set forth in SEQ ID NO: 88;
(6) a VH as set forth in SEQ ID NO: 84 and a VL as set forth in SEQ ID NO: 89;
(7) a VH as set forth in SEQ ID NO: 84 and a VL as set forth in SEQ ID NO: 90;
(8) a VH as set forth in SEQ ID NO: 84 and a VL as set forth in SEQ ID NO: 91;
(9) a VH as set forth in SEQ ID NO: 85 and a VL as set forth in SEQ ID NO: 87;
(10) a VH as set forth in SEQ ID NO: 85 and a VL as set forth in SEQ ID NO: 88;
(11) a VH as set forth in SEQ ID NO: 85 and a VL as set forth in SEQ ID NO: 92;
(12) a VH as set forth in SEQ ID NO: 85 and a VL as set forth in SEQ ID NO: 90;
(13) a VH as set forth in SEQ ID NO: 85 and a VL as set forth in SEQ ID NO: 91;
(14) a VH as set forth in SEQ ID NO: 86 and a VL as set forth in SEQ ID NO: 87;
(15) a VH as set forth in SEQ ID NO: 86 and a VL as set forth in SEQ ID NO: 88;
(16) a VH as set forth in SEQ ID NO: 86 and a VL as set forth in SEQ ID NO: 89;
(17) a VH as set forth in SEQ ID NO: 86 and a VL as set forth in SEQ ID NO: 90;
(18) a VH as set forth in SEQ ID NO: 86 and a VL as set forth in SEQ ID NO: 91;
(19) a VH as set forth in SEQ ID NO: 86 and a VL as set forth in SEQ ID NO: 92;
(20) a VH as set forth in SEQ ID NO: 86 and a VL as set forth in SEQ ID NO: 93;
(21) a VH as set forth in SEQ ID NO: 86 and a VL as set forth in SEQ ID NO: 94;
(22) a VH as set forth in SEQ ID NO: 86 and a VL as set forth in SEQ ID NO: 95;
(23) a VH as set forth in SEQ ID NO: 86 and a VL as set forth in SEQ ID NO: 96;
(24) a VH as set forth in SEQ ID NO: 86 and a VL as set forth in SEQ ID NO: 97;
(25) a VH as set forth in SEQ ID NO: 86 and a VL as set forth in SEQ ID NO: 98;
(26) a VH as set forth in SEQ ID NO: 86 and a VL as set forth in SEQ ID NO: 99;
(27) a VH as set forth in SEQ ID NO: 86 and a VL as set forth in SEQ ID NO: 100;
(28) a VH as set forth in SEQ ID NO: 86 and a VL as set forth in SEQ ID NO: 101;
(29) a VH as set forth in SEQ ID NO: 102 and a VL as set forth in SEQ ID NO: 107;
(30) a VH as set forth in SEQ ID NO: 102 and a VL as set forth in SEQ ID NO: 108;
(31) a VH as set forth in SEQ ID NO: 102 and a VL as set forth in SEQ ID NO: 109;
(32) a VH as set forth in SEQ ID NO: 102 and a VL as set forth in SEQ ID NO: 110;
(33) a VH as set forth in SEQ ID NO: 103 and a VL as set forth in SEQ ID NO: 107;
(34) a VH as set forth in SEQ ID NO: 103 and a VL as set forth in SEQ ID NO: 108;
(35) a VH as set forth in SEQ ID NO: 103 and a VL as set forth in SEQ ID NO: 109;
(36) a VH as set forth in SEQ ID NO: 103 and a VL as set forth in SEQ ID NO: 110;
(37) a VH as set forth in SEQ ID NO: 104 and a VL as set forth in SEQ ID NO: 107;
(38) a VH as set forth in SEQ ID NO: 104 and a VL as set forth in SEQ ID NO: 108;
(39) a VH as set forth in SEQ ID NO: 104 and a VL as set forth in SEQ ID NO: 109;
(40) a VH as set forth in SEQ ID NO: 104 and a VL as set forth in SEQ ID NO: 110;
(41) a VH as set forth in SEQ ID NO: 105 and a VL as set forth in SEQ ID NO: 107;
(42) a VH as set forth in SEQ ID NO: 105 and a VL as set forth in SEQ ID NO: 108;
(43) a VH as set forth in SEQ ID NO: 105 and a VL as set forth in SEQ ID NO: 109;
(44) a VH as set forth in SEQ ID NO: 105 and a VL as set forth in SEQ ID NO: 110;
(45) a VH as set forth in SEQ ID NO: 106 and a VL as set forth in SEQ ID NO: 107;
(46) a VH as set forth in SEQ ID NO: 106 and a VL as set forth in SEQ ID NO: 108;
(47) a VH as set forth in SEQ ID NO: 106 and a VL as set forth in SEQ ID NO: 109;
(48) a VH as set forth in SEQ ID NO: 106 and a VL as set forth in SEQ ID NO: 110;
(49) a VH as set forth in SEQ ID NO: 111 and a VL as set forth in SEQ ID NO: 107;
(50) a VH as set forth in SEQ ID NO: 111 and a VL as set forth in SEQ ID NO: 116;
(51) a VH as set forth in SEQ ID NO: 111 and a VL as set forth in SEQ ID NO: 117;
(52) a VH as set forth in SEQ ID NO: 111 and a VL as set forth in SEQ ID NO: 118;
(53) a VH as set forth in SEQ ID NO: 112 and a VL as set forth in SEQ ID NO: 107;
(54) a VH as set forth in SEQ ID NO: 112 and a VL as set forth in SEQ ID NO: 116;
(55) a VH as set forth in SEQ ID NO: 112 and a VL as set forth in SEQ ID NO: 117;
(56) a VH as set forth in SEQ ID NO: 112 and a VL as set forth in SEQ ID NO: 118;
(57) a VH as set forth in SEQ ID NO: 113 and a VL as set forth in SEQ ID NO: 107;
(58) a VH as set forth in SEQ ID NO: 113 and a VL as set forth in SEQ ID NO: 116;
(59) a VH as set forth in SEQ ID NO: 113 and a VL as set forth in SEQ ID NO: 117;
(60) a VH as set forth in SEQ ID NO: 113 and a VL as set forth in SEQ ID NO: 118;
(61) a VH as set forth in SEQ ID NO: 114 and a VL as set forth in SEQ ID NO: 107;
(62) a VH as set forth in SEQ ID NO: 114 and a VL as set forth in SEQ ID NO: 116;
(63) a VH as set forth in SEQ ID NO: 114 and a VL as set forth in SEQ ID NO: 117;
(64) a VH as set forth in SEQ ID NO: 114 and a VL as set forth in SEQ ID NO: 118;
(65) a VH as set forth in SEQ ID NO: 115 and a VL as set forth in SEQ ID NO: 107;
(66) a VH as set forth in SEQ ID NO: 115 and a VL as set forth in SEQ ID NO: 116;
(67) a VH as set forth in SEQ ID NO: 115 and a VL as set forth in SEQ ID NO: 117;
(68) a VH as set forth in SEQ ID NO: 115 and a VL as set forth in SEQ ID NO: 118.

In some preferred embodiments, as compared with any one of (1) to (68), the heavy chain variable region (VH) has a sequence identity of at least 70%, at least 80%, at least 85%, at least 90%, at least 91%, at least 92%, at least 93%, at least 94%, at least 95%, at least 96%, at least 97%, at least 98%, at least 99%, or 100%; and/or, the light chain variable region (VL) has a sequence identity of at least 70%, at least 80%, at least 85%, at least 90%, at least 91%, at least 92%, at least 93%, at least 94%, at least 95%, at least 96%, at least 97%, at least 98%, at least 99%, or 100%.

In some preferred embodiments, as compared with any one of (1) to (68), the heavy chain variable region (VH) has a substitution, deletion or addition of one or several amino acids (e.g., a substitution, deletion or addition of 1, 2, 3, 4 or 5 amino acids); and/or, the light chain variable region (VL) has a substitution, deletion or addition of one or several amino acids (e.g., a substitution, deletion or addition of 1, 2, 3, 4 or 5 amino acids); preferably, the substitution is a conservative substitution.

In any one of the above aspects, the antibody or antigen-binding fragment thereof of the present invention may further comprise a constant region sequence derived from a mammalian (e.g., murine or human) immunoglobulin or a variant thereof. In certain embodiments, the heavy chain of the antibody or antigen-binding fragment thereof of the present invention comprises a human or murine immunoglobulin heavy chain constant region (CH) or a variant thereof, the variant differs from the wild-type sequence from which it is derived in a substitution, deletion or addition of one or more amino acids (e.g., a substitution, deletion or addition of at most 20, at most 15, at most 10, or at most 5 amino acids; for example, a substitution, deletion or addition of 1, 2, 3, 4 or 5 amino acids); and/or, the light chain of the antibody or antigen-binding fragment thereof of the present invention comprises a human or murine immunoglobulin light chain constant region (CL) or a variant thereof, the variant differs from the wild-type sequence from which it is derived in a substitution, deletion or addition of one or more amino acids (e.g., a substitution, deletion or addition of at most 20, at most 15, at most 10, or at most 5 amino acids; for example, a substitution, deletion or addition of 1, 2, 3, 4 or 5 amino acids).

In certain preferred embodiments, the heavy chain of the antibody or antigen-binding fragment thereof of the present invention comprises a human immunoglobulin heavy chain constant region (CH) or a variant thereof, the variant differs from the wild-type sequence from which it is derived in a conservative substitution of at most 20 amino acids (e.g., a conservative substitution of at most 15, at most 10, or at most 5 amino acids; for example, a conservative substitution of 1, 2, 3, 4 or 5 amino acids); and/or, the light chain of the antibody or antigen-binding fragment thereof of the present invention comprises a human immunoglobulin light chain constant region (CL) or a variant thereof, the variant differs from the wild-type sequence from which it is derived in a conservative substitution of at most 20 amino acids (e.g., a conservative substitution of at most 15, at most 10, or at most 5 amino acids; for example, a conservative substitution of 1, 2, 3, 4 or 5 amino acids).

In certain embodiments, the heavy chain of the antibody or antigen-binding fragment thereof of the present invention comprises a murine immunoglobulin heavy chain constant region (CH) or a variant thereof, the variant differs from the wild-type sequence from which it is derived in a conservative substitution of at most 20 amino acids (e.g., a conservative substitution of at most 15, at most 10, or at most 5 amino acids; for example, a conservative substitution of 1, 2, 3, 4 or 5 amino acids); and/or, the light chain of the antibody or antigen-binding fragment thereof of the present invention comprises a murine immunoglobulin light chain constant region (CL) or a variant thereof, the variant differs from the wild-type sequence from which it is derived in a conservative substitution of at most 20 amino acids (e.g., a conservative substitution of at most 15, at most 10, or at most 5 amino acids; for example, a conservative substitution of 1, 2, 3, 4 or 5 amino acids).

In some embodiments, the constant region is altered, for example, mutated, to modify a property of the anti-ROR1 antibody molecule (e.g., to alter one or more of the following properties: Fc receptor binding, antibody glycosylation, number of cysteine residues, effector cell function, or complement function). Substitution of at least one amino acid residue in the antibody constant region with a different residue may result in a change of function, for example, change in affinity of antibody to effector ligand (e.g., FcR or complement C1q), thereby altering (e.g., enhancing) effector function. The Fc region of antibody mediates several important effector functions, such as ADCC, phagocytosis, CDC, etc. In certain instances, these effector functions are required for therapeutic antibodies.

In certain embodiments, the anti-ROR1 antibody molecule comprises a heavy chain constant region (Fc), which is selected from, for example, heavy chain constant regions of IgG1, IgG2, IgG3, IgG4, IgM, IgA1, IgA2, IgD and IgE; in particular selected from, for example, heavy chain constant regions of IgG1, IgG2, IgG3 and IgG4, more particularly selected from heavy chain constant regions of IgG1 or IgG4 (e.g., human IgG1 or IgG4). In some embodiments, the anti-ROR1 antibody molecule has a light chain constant region, which is selected from, for example, a κ or λ light chain constant region, preferably a κ light chain constant region (e.g., a human κ light chain).

In some embodiments, the antibody or antigen-binding fragment thereof comprises a heavy chain constant region selected from the group consisting of:
(1) a human IgG1 heavy chain constant region; or
(2) a human IgG4 heavy chain constant region.

In certain preferred embodiments, the antibody or antigen-binding fragment thereof of the present invention comprises:
(a) a heavy chain constant region (CH) comprising an amino acid sequence selected from the group consisting of:
   (i) a sequence as set forth in SEQ ID NO: 119;
   (ii) a sequence having a substitution, deletion or addition of one or several amino acids (e.g., a substitution, deletion or addition of 1, 2, 3, 4 or 5 amino acids) as compared to a sequence as set forth in SEQ ID NO: 119; or
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to a sequence as set forth in SEQ ID NO: 119;
   and/or
(b) a light chain constant region (CL) comprising an amino acid sequence selected from the group consisting of:
   (iv) a sequence as set forth in SEQ ID NO: 120;
   (v) a sequence having a substitution, deletion or addition of one or several amino acids (e.g., a substitution, deletion or addition of 1, 2, 3, 4 or 5 amino acids) as compared to a sequence as set forth in SEQ ID NO: 120; or
   (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to a sequence as set forth in SEQ ID NO: 120.

In certain preferred embodiments, the substitution described in (ii) or (v) is a conservative substitution.

In certain preferred embodiments, the antibody or antigen-binding fragment thereof of the present invention comprises a heavy chain constant region (CH) as set forth in SEQ ID NO: 119 and a light chain constant region (CL) as set forth in SEQ ID NO: 120.

In some preferred embodiments, the antibody of the present invention comprises a heavy chain having a VH as set forth in SEQ ID NO: 1 and a heavy chain constant region (CH) as set forth in SEQ ID NO: 119, and, comprises a light chain having a VL as set forth in SEQ ID NO: 2 and a light chain constant region (CL) as set forth in SEQ ID NO: 120.

In some preferred embodiments, the antibody of the present invention comprises a heavy chain having a VH as set forth in SEQ ID NO: 3 and a heavy chain constant region (CH) as set forth in SEQ ID NO: 119, and, comprises a light chain having a VL as set forth in SEQ ID NO: 4 and a light chain constant region (CL) as set forth in SEQ ID NO: 120.

In some preferred embodiments, the antibody of the present invention comprises a heavy chain having a VH as set forth in SEQ ID NO: 5 and a heavy chain constant region (CH) as set forth in SEQ ID NO: 119, and, comprises a light chain having a VL as set forth in SEQ ID NO: 6 and a light chain constant region (CL) as set forth in SEQ ID NO: 120.

In some preferred embodiments, the antibody of the present invention comprises a heavy chain having a VH as set forth in SEQ ID NO: 84 and a heavy chain constant region (CH) as set forth in SEQ ID NO: 119, and, comprises a light chain having a VL as set forth in SEQ ID NO: 87 and a light chain constant region (CL) as set forth in SEQ ID NO: 120.

In some preferred embodiments, the antibody of the present invention comprises a heavy chain having a VH as set forth in SEQ ID NO: 84 and a heavy chain constant region (CH) as set forth in SEQ ID NO: 119, and, comprises a light chain having a VL as set forth in SEQ ID NO: 88 and a light chain constant region (CL) as set forth in SEQ ID NO: 120.

In some preferred embodiments, the antibody of the present invention comprises a heavy chain having a VH as set forth in SEQ ID NO: 84 and a heavy chain constant region (CH) as set forth in SEQ ID NO: 119, and, comprises a light chain having a VL as set forth in SEQ ID NO: 89 and a light chain constant region (CL) as set forth in SEQ ID NO: 120.

In some preferred embodiments, the antibody of the present invention comprises a heavy chain having a VH as set forth in SEQ ID NO: 84 and a heavy chain constant region (CH) as set forth in SEQ ID NO: 119, and, comprises a light chain having a VL as set forth in SEQ ID NO: 90 and a light chain constant region (CL) as set forth in SEQ ID NO: 120.

In some preferred embodiments, the antibody of the present invention comprises a heavy chain having a VH as set forth in SEQ ID NO: 84 and a heavy chain constant region (CH) as set forth in SEQ ID NO: 119, and, comprises a light chain having a VL as set forth in SEQ ID NO: 91 and a light chain constant region (CL) as set forth in SEQ ID NO: 120.

In some preferred embodiments, the antibody of the present invention comprises a heavy chain having a VH as set forth in SEQ ID NO: 85 and a heavy chain constant region (CH) as set forth in SEQ ID NO: 119, and, comprises a light chain having a VL as set forth in SEQ ID NO: 87 and a light chain constant region (CL) as set forth in SEQ ID NO: 120.

In some preferred embodiments, the antibody of the present invention comprises a heavy chain having a VH as set forth in SEQ ID NO: 85 and a heavy chain constant region (CH) as set forth in SEQ ID NO: 119, and, comprises a light chain having a VL as set forth in SEQ ID NO: 88 and a light chain constant region (CL) as set forth in SEQ ID NO: 120.

In some preferred embodiments, the antibody of the present invention comprises a heavy chain having a VH as set forth in SEQ ID NO: 85 and a heavy chain constant region (CH) as set forth in SEQ ID NO: 119, and, comprises a light chain having a VL as set forth in SEQ ID NO: 92 and a light chain constant region (CL) as set forth in SEQ ID NO: 120.

In some preferred embodiments, the antibody of the present invention comprises a heavy chain having a VH as set forth in SEQ ID NO: 85 and a heavy chain constant region (CH) as set forth in SEQ ID NO: 119, and, comprises a light chain having a VL as set forth in SEQ ID NO: 90 and a light chain constant region (CL) as set forth in SEQ ID NO: 120.

In some preferred embodiments, the antibody of the present invention comprises a heavy chain having a VH as set forth in SEQ ID NO: 85 and a heavy chain constant region (CH) as set forth in SEQ ID NO: 119, and, comprises a light chain having a VL as set forth in SEQ ID NO: 91 and a light chain constant region (CL) as set forth in SEQ ID NO: 120.

In some preferred embodiments, the antibody of the present invention comprises a heavy chain having a VH as set forth in SEQ ID NO: 86 and a heavy chain constant region (CH) as set forth in SEQ ID NO: 119, and, comprises a light chain having a VL as set forth in SEQ ID NO: 87 and a light chain constant region (CL) as set forth in SEQ ID NO: 120.

In some preferred embodiments, the antibody of the present invention comprises a heavy chain having a VH as set forth in SEQ ID NO: 86 and a heavy chain constant region (CH) as set forth in SEQ ID NO: 119, and, comprises a light chain having a VL as set forth in SEQ ID NO: 88 and a light chain constant region (CL) as set forth in SEQ ID NO: 120.

In some preferred embodiments, the antibody of the present invention comprises a heavy chain having a VH as set forth in SEQ ID NO: 86 and a heavy chain constant region (CH) as set forth in SEQ ID NO: 119, and, comprises a light chain having a VL as set forth in SEQ ID NO: 89 and a light chain constant region (CL) as set forth in SEQ ID NO: 120.

In some preferred embodiments, the antibody of the present invention comprises a heavy chain having a VH as set forth in SEQ ID NO: 86 and a heavy chain constant region (CH) as set forth in SEQ ID NO: 119, and, comprises a light chain having a VL as set forth in SEQ ID NO: 90 and a light chain constant region (CL) as set forth in SEQ ID NO: 120.

In some preferred embodiments, the antibody of the present invention comprises a heavy chain having a VH as set forth in SEQ ID NO: 86 and a heavy chain constant region (CH) as set forth in SEQ ID NO: 119, and, comprises a light chain having a VL as set forth in SEQ ID NO: 91 and a light chain constant region (CL) as set forth in SEQ ID NO: 120.

In some preferred embodiments, the antibody of the present invention comprises a heavy chain having a VH as set forth in SEQ ID NO: 86 and a heavy chain constant region (CH) as set forth in SEQ ID NO: 119, and, comprises a light chain having a VL as set forth in SEQ ID NO: 92 and a light chain constant region (CL) as set forth in SEQ ID NO: 120.

In some preferred embodiments, the antibody of the present invention comprises a heavy chain having a VH as set forth in SEQ ID NO: 86 and a heavy chain constant region (CH) as set forth in SEQ ID NO: 119, and, comprises a light chain having a VL as set forth in SEQ ID NO: 93 and a light chain constant region (CL) as set forth in SEQ ID NO: 120.

In some preferred embodiments, the antibody of the present invention comprises a heavy chain having a VH as set forth in SEQ ID NO: 86 and a heavy chain constant region (CH) as set forth in SEQ ID NO: 119, and, comprises a light chain having a VL as set forth in SEQ ID NO: 94 and a light chain constant region (CL) as set forth in SEQ ID NO: 120.

In some preferred embodiments, the antibody of the present invention comprises a heavy chain having a VH as set forth in SEQ ID NO: 86 and a heavy chain constant region (CH) as set forth in SEQ ID NO: 119, and, comprises a light chain having a VL as set forth in SEQ ID NO: 95 and a light chain constant region (CL) as set forth in SEQ ID NO: 120.

In some preferred embodiments, the antibody of the present invention comprises a heavy chain having a VH as set forth in SEQ ID NO: 86 and a heavy chain constant region (CH) as set forth in SEQ ID NO: 119, and, comprises a light chain having a VL as set forth in SEQ ID NO: 96 and a light chain constant region (CL) as set forth in SEQ ID NO: 120.

In some preferred embodiments, the antibody of the present invention comprises a heavy chain having a VH as set forth in SEQ ID NO: 86 and a heavy chain constant region (CH) as set forth in SEQ ID NO: 119, and, comprises a light chain having a VL as set forth in SEQ ID NO: 97 and a light chain constant region (CL) as set forth in SEQ ID NO: 120.

In some preferred embodiments, the antibody of the present invention comprises a heavy chain having a VH as set forth in SEQ ID NO: 86 and a heavy chain constant region (CH) as set forth in SEQ ID NO: 119, and, comprises a light chain having a VL as set forth in SEQ ID NO: 98 and a light chain constant region (CL) as set forth in SEQ ID NO: 120.

In some preferred embodiments, the antibody of the present invention comprises a heavy chain having a VH as set forth in SEQ ID NO: 86 and a heavy chain constant region (CH) as set forth in SEQ ID NO: 119, and, comprises a light chain having a VL as set forth in SEQ ID NO: 99 and a light chain constant region (CL) as set forth in SEQ ID NO: 120.

In some preferred embodiments, the antibody of the present invention comprises a heavy chain having a VH as set forth in SEQ ID NO: 86 and a heavy chain constant region (CH) as set forth in SEQ ID NO: 119, and, comprises a light chain having a VL as set forth in SEQ ID NO: 100 and a light chain constant region (CL) as set forth in SEQ ID NO: 120.

In some preferred embodiments, the antibody of the present invention comprises a heavy chain having a VH as set forth in SEQ ID NO: 86 and a heavy chain constant region (CH) as set forth in SEQ ID NO: 119, and, comprises a light chain having a VL as set forth in SEQ ID NO: 101 and a light chain constant region (CL) as set forth in SEQ ID NO: 120.

In some preferred embodiments, the antibody of the present invention comprises a heavy chain having a VH as set forth in SEQ ID NO: 102 and a heavy chain constant region (CH) as set forth in SEQ ID NO: 119, and, comprises a light chain having a VL as set forth in SEQ ID NO: 107 and a light chain constant region (CL) as set forth in SEQ ID NO: 120.

In some preferred embodiments, the antibody of the present invention comprises a heavy chain having a VH as set forth in SEQ ID NO: 102 and a heavy chain constant region (CH) as set forth in SEQ ID NO: 119, and, comprises a light chain having a VL as set forth in SEQ ID NO: 108 and a light chain constant region (CL) as set forth in SEQ ID NO: 120.

In some preferred embodiments, the antibody of the present invention comprises a heavy chain having a VH as set forth in SEQ ID NO: 102 and a heavy chain constant region (CH) as set forth in SEQ ID NO: 119, and, comprises a light chain having a VL as set forth in SEQ ID NO: 109 and a light chain constant region (CL) as set forth in SEQ ID NO: 120.

In some preferred embodiments, the antibody of the present invention comprises a heavy chain having a VH as set forth in SEQ ID NO: 102 and a heavy chain constant region (CH) as set forth in SEQ ID NO: 119, and, comprises a light chain having a VL as set forth in SEQ ID NO: 110 and a light chain constant region (CL) as set forth in SEQ ID NO: 120.

In some preferred embodiments, the antibody of the present invention comprises a heavy chain having a VH as set forth in SEQ ID NO: 103 and a heavy chain constant region (CH) as set forth in SEQ ID NO: 119, and, comprises a light chain having a VL as set forth in SEQ ID NO: 107 and a light chain constant region (CL) as set forth in SEQ ID NO: 120.

In some preferred embodiments, the antibody of the present invention comprises a heavy chain having a VH as set forth in SEQ ID NO: 103 and a heavy chain constant region (CH) as set forth in SEQ ID NO: 119, and, comprises a light chain having a VL as set forth in SEQ ID NO: 108 and a light chain constant region (CL) as set forth in SEQ ID NO: 120.

In some preferred embodiments, the antibody of the present invention comprises a heavy chain having a VH as set forth in SEQ ID NO: 103 and a heavy chain constant region (CH) as set forth in SEQ ID NO: 119, and, comprises a light chain having a VL as set forth in SEQ ID NO: 109 and a light chain constant region (CL) as set forth in SEQ ID NO: 120.

In some preferred embodiments, the antibody of the present invention comprises a heavy chain having a VH as set forth in SEQ ID NO: 103 and a heavy chain constant region (CH) as set forth in SEQ ID NO: 119, and, comprises a light chain having a VL as set forth in SEQ ID NO: 110 and a light chain constant region (CL) as set forth in SEQ ID NO: 120.

In some preferred embodiments, the antibody of the present invention comprises a heavy chain having a VH as set forth in SEQ ID NO: 104 and a heavy chain constant region (CH) as set forth in SEQ ID NO: 119, and, comprises a light chain having a VL as set forth in SEQ ID NO: 107 and a light chain constant region (CL) as set forth in SEQ ID NO: 120.

In some preferred embodiments, the antibody of the present invention comprises a heavy chain having a VH as set forth in SEQ ID NO: 104 and a heavy chain constant region (CH) as set forth in SEQ ID NO: 119, and, comprises a light chain having a VL as set forth in SEQ ID NO: 108 and a light chain constant region (CL) as set forth in SEQ ID NO: 120.

In some preferred embodiments, the antibody of the present invention comprises a heavy chain having a VH as set forth in SEQ ID NO: 104 and a heavy chain constant region (CH) as set forth in SEQ ID NO: 119, and, comprises a light chain having a VL as set forth in SEQ ID NO: 109 and a light chain constant region (CL) as set forth in SEQ ID NO: 120.

In some preferred embodiments, the antibody of the present invention comprises a heavy chain having a VH as set forth in SEQ ID NO: 104 and a heavy chain constant region (CH) as set forth in SEQ ID NO: 119, and, comprises a light chain having a VL as set forth in SEQ ID NO: 110 and a light chain constant region (CL) as set forth in SEQ ID NO: 120.

In some preferred embodiments, the antibody of the present invention comprises a heavy chain having a VH as set forth in SEQ ID NO: 105 and a heavy chain constant region (CH) as set forth in SEQ ID NO: 119, and, comprises a light chain having a VL as set forth in SEQ ID NO: 107 and a light chain constant region (CL) as set forth in SEQ ID NO: 120.

In some preferred embodiments, the antibody of the present invention comprises a heavy chain having a VH as set forth in SEQ ID NO: 105 and a heavy chain constant region (CH) as set forth in SEQ ID NO: 119, and, comprises a light chain having a VL as set forth in SEQ ID NO: 108 and a light chain constant region (CL) as set forth in SEQ ID NO: 120.

In some preferred embodiments, the antibody of the present invention comprises a heavy chain having a VH as set forth in SEQ ID NO: 105 and a heavy chain constant region (CH) as set forth in SEQ ID NO: 119, and, comprises a light chain having a VL as set forth in SEQ ID NO: 109 and a light chain constant region (CL) as set forth in SEQ ID NO: 120.

In some preferred embodiments, the antibody of the present invention comprises a heavy chain having a VH as set forth in SEQ ID NO: 105 and a heavy chain constant region (CH) as set forth in SEQ ID NO: 119, and, comprises a light chain having a VL as set forth in SEQ ID NO: 110 and a light chain constant region (CL) as set forth in SEQ ID NO: 120.

In some preferred embodiments, the antibody of the present invention comprises a heavy chain having a VH as set forth in SEQ ID NO: 106 and a heavy chain constant region (CH) as set forth in SEQ ID NO: 119, and, comprises a light chain having a VL as set forth in SEQ ID NO: 107 and a light chain constant region (CL) as set forth in SEQ ID NO: 120.

In some preferred embodiments, the antibody of the present invention comprises a heavy chain having a VH as set forth in SEQ ID NO: 106 and a heavy chain constant region (CH) as set forth in SEQ ID NO: 119, and, comprises a light chain having a VL as set forth in SEQ ID NO: 108 and a light chain constant region (CL) as set forth in SEQ ID NO: 120.

In some preferred embodiments, the antibody of the present invention comprises a heavy chain having a VH as set forth in SEQ ID NO: 106 and a heavy chain constant region (CH) as set forth in SEQ ID NO: 119, and, comprises a light chain having a VL as set forth in SEQ ID NO: 109 and a light chain constant region (CL) as set forth in SEQ ID NO: 120.

In some preferred embodiments, the antibody of the present invention comprises a heavy chain having a VH as set forth in SEQ ID NO: 106 and a heavy chain constant region (CH) as set forth in SEQ ID NO: 119, and, comprises a light chain having a VL as set forth in SEQ ID NO: 110 and a light chain constant region (CL) as set forth in SEQ ID NO: 120.

In some preferred embodiments, the antibody of the present invention comprises a heavy chain having a VH as set forth in SEQ ID NO: 111 and a heavy chain constant region (CH) as set forth in SEQ ID NO: 119, and, comprises a light chain having a VL as set forth in SEQ ID NO: 107 and a light chain constant region (CL) as set forth in SEQ ID NO: 120.

In some preferred embodiments, the antibody of the present invention comprises a heavy chain having a VH as set forth in SEQ ID NO: 111 and a heavy chain constant region (CH) as set forth in SEQ ID NO: 119, and, comprises a light chain having a VL as set forth in SEQ ID NO: 116 and a light chain constant region (CL) as set forth in SEQ ID NO: 120.

In some preferred embodiments, the antibody of the present invention comprises a heavy chain having a VH as set forth in SEQ ID NO: 111 and a heavy chain constant region (CH) as set forth in SEQ ID NO: 119, and, comprises a light chain having a VL as set forth in SEQ ID NO: 117 and a light chain constant region (CL) as set forth in SEQ ID NO: 120.

In some preferred embodiments, the antibody of the present invention comprises a heavy chain having a VH as set forth in SEQ ID NO: 111 and a heavy chain constant region (CH) as set forth in SEQ ID NO: 119, and, comprises a light chain having a VL as set forth in SEQ ID NO: 118 and a light chain constant region (CL) as set forth in SEQ ID NO: 120.

In some preferred embodiments, the antibody of the present invention comprises a heavy chain having a VH as set forth in SEQ ID NO: 112 and a heavy chain constant region (CH) as set forth in SEQ ID NO: 119, and, comprises a light chain having a VL as set forth in SEQ ID NO: 107 and a light chain constant region (CL) as set forth in SEQ ID NO: 120.

In some preferred embodiments, the antibody of the present invention comprises a heavy chain having a VH as set forth in SEQ ID NO: 112 and a heavy chain constant region (CH) as set forth in SEQ ID NO: 119, and, comprises a light chain having a VL as set forth in SEQ ID NO: 116 and a light chain constant region (CL) as set forth in SEQ ID NO: 120.

In some preferred embodiments, the antibody of the present invention comprises a heavy chain having a VH as set forth in SEQ ID NO: 112 and a heavy chain constant region (CH) as set forth in SEQ ID NO: 119, and, comprises a light chain having a VL as set forth in SEQ ID NO: 117 and a light chain constant region (CL) as set forth in SEQ ID NO: 120.

In some preferred embodiments, the antibody of the present invention comprises a heavy chain having a VH as set forth in SEQ ID NO: 112 and a heavy chain constant region (CH) as set forth in SEQ ID NO: 119, and, comprises a light chain having a VL as set forth in SEQ ID NO: 118 and a light chain constant region (CL) as set forth in SEQ ID NO: 120.

In some preferred embodiments, the antibody of the present invention comprises a heavy chain having a VH as set forth in SEQ ID NO: 113 and a heavy chain constant region (CH) as set forth in SEQ ID NO: 119, and, comprises a light chain having a VL as set forth in SEQ ID NO: 107 and a light chain constant region (CL) as set forth in SEQ ID NO: 120.

In some preferred embodiments, the antibody of the present invention comprises a heavy chain having a VH as set forth in SEQ ID NO: 113 and a heavy chain constant region (CH) as set forth in SEQ ID NO: 119, and, comprises a light chain having a VL as set forth in SEQ ID NO: 116 and a light chain constant region (CL) as set forth in SEQ ID NO: 120.

In some preferred embodiments, the antibody of the present invention comprises a heavy chain having a VH as set forth in SEQ ID NO: 113 and a heavy chain constant region (CH) as set forth in SEQ ID NO: 119, and, comprises a light chain having a VL as set forth in SEQ ID NO: 117 and a light chain constant region (CL) as set forth in SEQ ID NO: 120.

In some preferred embodiments, the antibody of the present invention comprises a heavy chain having a VH as set forth in SEQ ID NO: 113 and a heavy chain constant region (CH) as set forth in SEQ ID NO: 119, and, comprises a light chain having a VL as set forth in SEQ ID NO: 118 and a light chain constant region (CL) as set forth in SEQ ID NO: 120.

In some preferred embodiments, the antibody of the present invention comprises a heavy chain having a VH as set forth in SEQ ID NO: 114 and a heavy chain constant region (CH) as set forth in SEQ ID NO: 119, and, comprises a light chain having a VL as set forth in SEQ ID NO: 107 and a light chain constant region (CL) as set forth in SEQ ID NO: 120.

In some preferred embodiments, the antibody of the present invention comprises a heavy chain having a VH as set forth in SEQ ID NO: 114 and a heavy chain constant region (CH) as set forth in SEQ ID NO: 119, and, comprises a light chain having a VL as set forth in SEQ ID NO: 116 and a light chain constant region (CL) as set forth in SEQ ID NO: 120.

In some preferred embodiments, the antibody of the present invention comprises a heavy chain having a VH as set forth in SEQ ID NO: 114 and a heavy chain constant region (CH) as set forth in SEQ ID NO: 119, and, comprises a light chain having a VL as set forth in SEQ ID NO: 117 and a light chain constant region (CL) as set forth in SEQ ID NO: 120.

In some preferred embodiments, the antibody of the present invention comprises a heavy chain having a VH as set forth in SEQ ID NO: 114 and a heavy chain constant region (CH) as set forth in SEQ ID NO: 119, and, comprises a light chain having a VL as set forth in SEQ ID NO: 118 and a light chain constant region (CL) as set forth in SEQ ID NO: 120.

In some preferred embodiments, the antibody of the present invention comprises a heavy chain having a VH as set forth in SEQ ID NO: 115 and a heavy chain constant region (CH) as set forth in SEQ ID NO: 119, and, comprises a light chain having a VL as set forth in SEQ ID NO: 107 and a light chain constant region (CL) as set forth in SEQ ID NO: 120.

In some preferred embodiments, the antibody of the present invention comprises a heavy chain having a VH as set forth in SEQ ID NO: 115 and a heavy chain constant region (CH) as set forth in SEQ ID NO: 119, and, comprises a light chain having a VL as set forth in SEQ ID NO: 116 and a light chain constant region (CL) as set forth in SEQ ID NO: 120.

In some preferred embodiments, the antibody of the present invention comprises a heavy chain having a VH as set forth in SEQ ID NO: 115 and a heavy chain constant region (CH) as set forth in SEQ ID NO: 119, and, comprises a light chain having a VL as set forth in SEQ ID NO: 117 and a light chain constant region (CL) as set forth in SEQ ID NO: 120.

In some preferred embodiments, the antibody of the present invention comprises a heavy chain having a VH as set forth in SEQ ID NO: 115 and a heavy chain constant region (CH) as set forth in SEQ ID NO: 119, and, comprises a light chain having a VL as set forth in SEQ ID NO: 118 and a light chain constant region (CL) as set forth in SEQ ID NO: 120.

In certain preferred embodiments, the antibody of the present invention is a chimeric or humanized antibody. In certain preferred embodiments, the antibody or antigen-binding fragment thereof of the present invention is selected from the group consisting of: ScFv, Fab, Fab', (Fab')₂, Fv fragments, disulfide-bonded Fv(dsFv), diabody, bispecific antibody, and multispecific antibody.

In certain embodiments, the antibody molecule or antigen-binding fragment thereof of the present invention may exhibit at least one of the following properties:
(a) binding to ROR1 (e.g., human ROR1) with a K_{D} of less than about 100 nM, for example, less than about 50 nM, 40 nM, 30 nM, 20 nM, 10 nM, 1 nM, 0.1 nM or lower; for example, the K_{D} is determined by biolayer interferometry (BLI) (e.g., ForteBio Octet^{®});
(b) binding to ROR1 (e.g., human ROR1) with an EC50 of less than about 500 nM, for example, less than about 100 nM, 10 nM, 1 nM, 0.9 nM, 0.8 nM, 0.7 nM, 0.6 nM, 0.5 nM, 0.4 nM, 0.3 nM, 0.2 nM, 0.1 nM or lower; for example, the EC50 is determined by flow cytometry or competitive ELISA;
(c) the antibody or antigen-binding fragment thereof not binding to ROR2 (e.g., human ROR2).

In certain embodiments, the antibody or antigen-binding fragment thereof has ADCC activity.

In certain embodiments, the antibody or antigen-binding fragment thereof has CDC activity.

In certain preferred embodiments, the antibody or antigen-binding fragment thereof has ADCC and CDC activity.

In certain preferred embodiments, the antibody or antigen-binding fragment thereof has enhanced ADCC and/or CDC activity.

In some preferred embodiments, the antibody or antigen-binding fragment thereof of the present invention has at least one of the following biological functions:
(a) inducing apoptosis of tumor cells;
(b) inhibiting growth, proliferation, differentiation, and/or angiogenesis of tumor cells;
(c) inducing and/or increasing complement-dependent cytotoxic activity;
(d) inducing and/or increasing antibody-dependent cytotoxic activity;
(e) inhibiting expression and activation of ROR1;
(f) inhibiting ROR1-mediated cell signaling;
(g) preventing and/or treating ROR1-mediated disease/disorder;
(h) promoting internalization of ROR1 in cells (e.g., tumor cells); or
(i) any combination of (a) to (h).

In certain preferred embodiments, the antibody or antigen-binding fragment thereof of the present invention possesses any combination of the above-mentioned biological functions.

### Derivatized antibody

The antibody or antigen-binding fragment thereof of the present invention can be derivatized, for example, linked to another molecule (e.g., another polypeptide or protein). Typically, derivatization (e.g., labeling) of an antibody or antigen-binding fragment thereof will not adversely affect its binding to ROR1 (particularly human ROR1). Accordingly, it is also intended that the antibody or antigen-binding fragment thereof of the present invention comprises such derivatized forms. For example, the antibody or antigen-binding fragment thereof of the present invention can be functionally linked (by chemical coupling, genetic fusion, non-covalent linkage, or other manners) to one or more other molecular moieties, such as another antibody (e.g., to form a bispecific antibody), detection reagent, pharmaceutical reagent, and/or protein or polypeptide (e.g., avidin or polyhistidine tag) capable of mediating the binding of the antibody or antigen-binding fragment to another molecule.

One type of derivatized antibody (e.g., bispecific antibody) is produced by cross-linking 2 or more antibodies (of the same type or of different types). Methods for obtaining bispecific antibodies are well known in the art, examples of which include, but are not limited to, chemical cross-linking methods, cell engineering methods (hybridoma methods) or genetic engineering methods.

Another type of derivatized antibody is a labeled antibody. For example, the antibody or antigen-binding fragment thereof of the present invention can be linked to a detectable label. The detectable label of the present invention can be any substance detectable by fluorescent, spectroscopic, photochemical, biochemical, immunological, electrical, optical or chemical means. Such labels are well known in the art, examples of which include, but are not limited to, enzymes (e.g., horseradish peroxidase, alkaline phosphatase, β-galactosidase, urease, glucose oxidase, etc.), radionuclides (e.g., 3H, 125I, 35S, 14C, or 32P), fluorescent dyes (e.g., fluorescein isothiocyanate (FITC), fluorescein, tetramethylrhodamine isothiocyanate (TRITC), phycoerythrin (PE), Texas Red, rhodamine, quantum dots or cyanine dye derivatives (e.g., Cy7, Alexa 750)), acridinium esters, magnetic beads (e.g., Dynabeads^{®}), calorimetric markers such as colloidal gold or colored glass or plastic (e.g., polystyrene, polypropylene, latex, etc.) beads, and biotin for binding to the above-mentioned label-modified avidin (e.g., streptavidin). Patents that teach the use of such labels include, but are not limited to, US Patents 3,817,837; 3,850,752; 3,939,350; 3,996,345; 4,277,437; 4,275,149; and 4,366,241 (all of which are incorporated herein by reference in their entirety). The detectable labels as described above can be detected by methods known in the art. For example, radioactive labels can be detected using photographic film or scintillation counter, and fluorescent labels can be detected using photodetector to detect emitted light. Enzyme labels are generally detected by providing a substrate and detecting a product produced by the action of the enzyme on the substrate, and thermometric labels are detected by simple visualization of a colored label. In certain embodiments, such labels can be adapted for use in immunological detection (e.g., enzyme-linked immunoassay, radioimmunoassay, fluorescent immunoassay, chemiluminescence immunoassay, etc.). In certain embodiments, a detectable label as described above can be attached to the antibody or antigen-binding fragment thereof of the present invention via linkers of various lengths to reduce potential steric hindrance.

In addition, the antibody or antigen-binding fragment thereof of the present invention may also be derivatized with chemical groups, such as polyethylene glycol (PEG), methyl or ethyl, or glycosyl groups. These groups can be used to improve the biological properties of the antibody, such as increasing serum half-life.

As one of the antibody derivatives, the present invention provides a conjugate, which comprises the monoclonal antibody or antigen-binding fragment thereof of the present invention and a coupling moiety, the coupling moiety is selected from the group consisting of: detectable label, radioactive isotope, fluorescent substance, luminescent substance, colored substance, enzyme, polyethylene glycol (PEG), nuclide, nucleic acid, small molecule toxin, and polypeptide, protein, receptor, or ligand with binding activity, as well as other active substance capable of inhibiting tumor cell growth, promoting tumor cell apoptosis or necrosis.

As one of the antibody derivatives, the present invention provides a chimeric antigen receptor, which comprises the monoclonal antibody or antigen-binding fragment thereof. In certain preferred embodiments, the chimeric antigen receptor comprises a monoclonal antibody or antigen-binding fragment thereof (e.g., ScFv) of the present invention, a transmembrane domain, and one or more intracellular T cell signaling domains. The present invention also provides a host cell comprising or expressing the chimeric antigen receptor, such as immune cell (e.g., T lymphocyte, NK cell).

As one of the antibody derivatives, the present invention provides a multispecific antibody, which is formed by conjugating a first antibody or fragment thereof with other antibodies or fragments thereof or antibody mimetics, wherein each antibody or fragment thereof or antibody mimetic retains original binding specificity, and the first antibody or fragment thereof is the antibody or antigen-binding fragment thereof of the present invention; preferably, the multispecific antibody is a bispecific antibody or a trispecific antibody or a tetraspecific antibody.

### Preparation of antibody

The antibody of the present invention can be prepared by various methods known in the art, such as by genetic engineering recombinant techniques. For example, a DNA molecule encoding the heavy and light chain genes of the antibody of the present invention can be obtained by chemical synthesis or PCR amplification; the resulting DNA molecule is inserted into an expression vector, and then transfected into a host cell. Then, the transfected host cell is cultured under a specific condition to express the antibody of the present invention.

The antigen-binding fragment of the present invention can be obtained by hydrolysis of an intact antibody molecule (see: Morimoto et al., J. Biochem. Biophys. Methods 24:107-117 (1992) and Brennan et al., Science 229:81 (1985)). Alternatively, the antigen-binding fragment can also be produced directly from a recombinant host cell (reviewed in Hudson, Curr. Opin. Immunol. 11:548-557 (1999); Little et al., Immunol. Today, 21:364-370 (2000)). For example, a Fab' fragment can be obtained directly from a host cell; a F(ab')₂ fragment can be formed by chemically coupling Fab' fragments (Carter et al., Bio/Technology, 10: 163-167 (1992)). In addition, a Fv, Fab or F(ab')₂ fragment can also be directly isolated from a culture medium of recombinant host cells. Other techniques for preparing these antigen-binding fragments are well known to those of ordinary skill in the art.

Accordingly, in another aspect, the present invention provides an isolated nucleic acid molecule, which comprises a nucleotide sequence encoding the antibody or antigen-binding fragment thereof of the present invention, or a heavy chain variable region and/or light chain variable region thereof. According to the codon degeneracy in the field, in some embodiments, the nucleotide sequence can be replaced according to the codon degeneracy. In certain embodiments, the nucleotide sequence is codon optimized.

In certain preferred embodiments, the present invention provides an isolated nucleic acid molecule, which comprises a nucleic acid molecule encoding an antibody heavy chain variable region, and/or a nucleic acid molecule encoding an antibody light chain variable region, wherein, the nucleic acid molecule encoding the antibody heavy chain variable region has a sequence selected from the following: (a) a nucleotide sequence as set forth in any one of SEQ ID NOs: 121, 123, 125, 127-129, 145-149, and 154-158, or (b) a sequence substantially identical to the nucleotide sequence described in (a) (e.g., as compared to the nucleotide sequence described in (a), a sequence having a sequence identity of at least about 85%, 90%, 95%, 99% or higher, or a sequence with a substitution of one or more nucleotides), or (c) a sequence differing from the nucleotide sequence described in (a) by no more than 3, 6, 15, 30 or 45 nucleotides; the nucleic acid molecule encoding the antibody light chain variable region has a sequence selected from the following: (d) a nucleotide sequence as set forth in any one of SEQ ID NOs: 122, 124, 126, 130-144, 150-153, and 159-161, or (e) a sequence substantially identical to the nucleotide sequence described in (d) (e.g., as compared to the nucleotide sequence described in (d), a sequence having a sequence identity of at least about 85%, 90%, 95%, 99% or more, or a sequence having a substitution of one or more nucleotides), or (1) a sequence differing from the nucleotide sequence described in (d) by no more than 3, 6, 15, 30 or 45 nucleotides.

In some preferred embodiments, the nucleic acid molecule encoding the antibody heavy chain variable region has a nucleotide sequence as set forth in any one of SEQ ID NOs: 121, 123, 125, 127-129, 145-149, and 154-158, and the nucleic acid molecule encoding the antibody light chain variable region has a nucleotide sequence as set forth in any one of SEQ ID NOs: 122, 124, 126, 130-144, 150-153, and 159-161. In certain preferred embodiments, the isolated nucleic acid molecule of the present invention comprises a nucleic acid molecule as set forth in any one of SEQ ID NOs: 121, 123, 125, 127-129, 145-149, and 154-158 for encoding the antibody heavy chain variable region, and/or a nucleic acid molecule as set forth in any one of SEQ ID NOs: 122, 124, 126, 130-144, 150-153, and 159-161 for encoding the antibody light chain variable region.

In certain preferred embodiments, the present invention provides an isolated nucleic acid molecule, which comprises a nucleic acid molecule encoding an antibody heavy chain constant region, and/or a nucleic acid molecule encoding an antibody light chain constant region, wherein the nucleic acid molecule encoding the antibody heavy chain constant region has a sequence selected from the following: (a) a nucleotide sequence as set forth in SEQ ID NO: 59, or (b) a sequence substantially identical to the nucleotide sequence described in (a) (e.g., as compared with the nucleotide sequence described in (a), a sequence having a sequence identity of at least about 85%, 90%, 95%, 99% or greater, or a sequence having a substitution of one or more nucleotides), or (c) a nucleotide sequence differing from the nucleotide sequence described in (a) by no more than 3, 6, 15, 30 or 45 nucleotides, and/or, the nucleic acid molecule encoding the antibody light chain constant region has a sequence selected from the following: (d) a nucleotide sequence as set forth in SEQ ID NO: 26, or (e) a sequence substantially identical to the nucleotide sequence described in (d) (e.g., as compared to the nucleotide sequence described in (d), a sequence having a sequence identity of at least about 85%, 90%, 95%, 99% or greater, or a sequence having a substitution of one or more nucleotides), or (1) a sequence differing from the nucleotide sequence described in (d) by no more than 3, 6, 15, 30 or 45 nucleotides.

In some preferred embodiments, the nucleic acid molecule encoding the antibody heavy chain constant region has a nucleotide sequence as set forth in SEQ ID NO: 59, and/or the nucleic acid molecule encoding the antibody light chain constant region has a nucleotide sequence as set forth in SEQ ID NO: 26. In some preferred embodiments, the isolated nucleic acid molecule of the present invention comprises a nucleotide sequence as set forth in SEQ ID NO: 59, which encodes the antibody heavy chain constant region, and/or comprises a nucleotide sequence as set forth in SEQ ID NO: 26, which encodes the antibody light chain constant region.

In another aspect of the present invention, the present invention provides a vector (e.g., a cloning vector or an expression vector), which comprises the isolated nucleic acid molecule of the present invention. In certain preferred embodiments, the vector of the present invention is, for example, plasmid, cosmid, phage, lentivirus, and the like. In certain preferred embodiments, the vector is capable of expressing the antibody or antigen-binding fragment thereof of the present invention in a subject (e.g., a mammal, for example, a human).

In certain embodiments, the antibody or antigen-binding fragment thereof of the present invention can be used to construct a chimeric antigen receptor (CAR), and the chimeric antigen receptor comprises an extracellular antigen-binding domain (e.g., ScFv) that specifically binds to ROR1, a transmembrane domain, and one or more intracellular T cell signaling domains. In such embodiments, the isolated nucleic acid molecule of the present invention may comprise a nucleotide sequence encoding a chimeric antigen receptor, and the nucleotide sequence encoding the chimeric antigen receptor further comprises a nucleotide sequence encoding the antibody or antigen-binding fragment thereof of the present invention (e.g., ScFv). In certain embodiments, the isolated nucleic acid molecule of the present invention encodes a chimeric antigen receptor comprising an antigen-binding fragment (e.g., ScFv) of the antibody of the present invention.

In certain embodiments, the antibody or antigen-binding fragment thereof of the present invention can be used to construct a chimeric antigen receptor-modified immune cell, and the chimeric antigen receptor-modified immune cell comprises a chimeric antigen receptor (CAR) and an immune cell (e.g., T lymphocyte, NK cell).

In another aspect of the present invention, the present invention provides a host cell, which comprises the isolated nucleic acid molecule of the present invention or the vector of the present invention. The host cell can be eukaryotic cell (e.g., mammalian cell, insect cell, yeast cell) or prokaryotic cell (e.g., *E. coli*)*.* Suitable eukaryotic cell includes, but is not limited to, NS0 cell, Vero cell, Hela cell, COS cell, CHO cell, ExpiCHO cell, HEK293 cell, Expi293 cell, BHK cell, and MDCKII cell. Suitable insect cell includes, but is not limited to, Sf9 cell. In certain preferred embodiments, the host cell of the present invention is a mammalian cell, such as CHO (e.g., CHO-K1, CHO-S, CHO DXB11, ExpiCHO, CHO DG44).

In certain embodiments, the host cell of the present invention may be a chimeric antigen receptor T cell (CAR-T). In such embodiments, the isolated nucleic acid molecule contained in the host cell may comprise a nucleotide sequence encoding a chimeric antigen receptor, the nucleotide sequence encoding the chimeric antigen receptor further comprises a nucleotide sequence encoding the antibody or antigen-binding fragment thereof of the present invention (e.g., ScFv). In certain embodiments, the isolated nucleic acid molecule contained in the host cell encodes a chimeric antigen receptor comprising an antigen-binding fragment (e.g., ScFv) of the antibody of the present invention.

In another aspect of the present invention, there is provided a method for preparing the antibody or antigen-binding fragment thereof of the present invention, which comprises culturing the host cell of the present invention under conditions that allow the expression of the antibody or antigen-binding fragment thereof, and recovering the antibody or antigen-binding fragment thereof from a culture for culturing the host cell.

### Therapeutic method and pharmaceutical composition

In another aspect of the present invention, a pharmaceutical composition is disclosed, which comprises the antibody or antigen-binding fragment thereof, carrier, host cell, conjugate, chimeric antigen receptor, or multispecific antibody of the present invention, and a pharmaceutically acceptable carrier and/or excipient.

In certain preferred embodiments, the pharmaceutical composition of the present invention comprises the antibody or antigen-binding fragment thereof of the present invention, and a pharmaceutically acceptable carrier and/or excipient.

In certain preferred embodiments, the pharmaceutical composition of the present invention comprises the vector or host cell of the present invention, and a pharmaceutically acceptable carrier and/or excipient. In such embodiments, the isolated nucleic acid molecule comprised by the vector comprises a nucleotide sequence encoding a chimeric antigen receptor, and the nucleotide sequence encoding the chimeric antigen receptor further comprises a nucleotide sequence encoding the antibody or antigen-binding fragment thereof of the present invention (e.g., ScFv); the host cell comprises the isolated nucleic acid molecule or vector as described above. In certain preferred embodiments, the isolated nucleic acid molecule encodes a chimeric antigen receptor comprising the antigen-binding fragment (e.g., ScFv) of the antibody of the present invention. In certain preferred embodiments, the host cell is T cell. In certain preferred embodiments, the host cell is a chimeric antigen receptor T cell (CAR-T).

In certain preferred embodiments, the pharmaceutical composition may further comprise an additional pharmaceutically active agent. In certain preferred embodiments, the additional pharmaceutically active agent is a drug for the treatment of lymphoma, breast cancer, colorectal cancer, ovarian cancer, lung cancer and/or pancreatic cancer. In certain preferred embodiments, the additional pharmaceutically active agent is a drug having antitumor activity. In certain preferred embodiments, the additional pharmaceutically active agent is one or more selected from the group consisting of epidermal growth factor receptor (EGFR) inhibitor, immune checkpoint inhibitor, B cell antigen inhibitor, BTK inhibitor, chemotherapy drug.

In certain preferred embodiments, in the pharmaceutical composition, the antibody or antigen-binding fragment thereof of the present invention and the additional pharmaceutically active agent are provided as separate components or as components of a single composition. Accordingly, the antibody or antigen-binding fragment thereof of the present invention and the additional pharmaceutically active agent may be administered simultaneously, separately or sequentially.

In certain preferred embodiments, the pharmaceutical composition may further comprise an additional pharmaceutically active agent. The additional pharmaceutically active agent is one or more selected from the group consisting of epidermal growth factor receptor (EGFR) inhibitor, immune checkpoint inhibitor, B cell antigen inhibitor, BTK inhibitor, and chemotherapy drug.

In certain preferred embodiments, the additional pharmaceutically active agent may be one or more selected from the group consisting of anti-PD1/PD-L1 antibody, anti-CD20 antibody, and anti-EGFR antibody.

In another aspect, the antibody or antigen-binding fragment thereof, vector, host cell, conjugate, chimeric antigen receptor, or multispecific antibody in the pharmaceutical composition of the present invention is sufficient in subject to:
(a) induce tumor cell apoptosis;
(b) inhibit tumor cell growth, proliferation, differentiation, and/or angiogenesis;
(c) induce and/or increase complement-dependent cytotoxic activity;
(d) induce and/or increase antibody-dependent cytotoxic activity;
(e) inhibit the expression and activation of ROR1;
(f) inhibit ROR1-mediated cell signaling;
(g) prevent and/or treat a ROR1-mediated disease/disorder; or
(h) any combination of (a) to (g).

Another aspect of the present invention provides a use of the antibody or antigen-binding fragment thereof, vector, host cell, conjugate, chimeric antigen receptor, or multispecific antibody of the present invention in the manufacture of a medicament, and the medicament is used for:
(a) inducing tumor cell apoptosis;
(b) inhibiting tumor cell growth, proliferation, differentiation, and/or angiogenesis;
(c) inducing and/or increasing complement-dependent cytotoxic activity;
(d) inducing and/or increasing antibody-dependent cytotoxic activity;
(e) inhibiting the expression and activation of ROR1;
(f) inhibiting ROR1-mediated cell signaling;
(g) preventing and/or treating a ROR1-mediated disease/disorder; or
(h) any combination of (a) to (g).

In certain preferred embodiments, when the vector or host cell of the present invention is used in the manufacture of a medicament, the isolated nucleic acid molecule contained in the vector comprises a nucleotide sequence encoding a chimeric antigen receptor, the nucleotide sequence encoding the chimeric antigen receptor further comprises a nucleotide sequence encoding the antibody or antigen-binding fragment thereof of the present invention (e.g., scFv); the host cell comprises the isolated nucleic acid molecule or vector as described above. In certain preferred embodiments, the isolated nucleic acid molecule encodes a chimeric antigen receptor comprising an antigen-binding fragment (e.g., scFv) of the antibody of the present invention. In certain preferred embodiments, the host cell is a T cell. In certain preferred embodiments, the host cell is a chimeric antigen receptor T cell (CAR-T).

In certain preferred embodiments, when the vector or host cell of the present invention is used in the manufacture of a medicament, the medicament is used for the treatment of a tumor in a subject (e.g., a human).

In some preferred embodiments, the tumor associated with the antibody or antigen-binding fragment thereof, vector, host cell, conjugate, chimeric antigen receptor, or multispecific antibody of the present invention is selected from the group consisting of lymphoma, breast cancer, colorectal cancer, ovarian cancer, lung cancer and/or pancreatic cancer.

In certain preferred embodiments, the tumor associated with the antibody or antigen-binding fragment thereof of the present invention is selected from the group consisting of lymphoma, breast cancer, colorectal cancer, ovarian cancer, lung cancer and/or pancreatic cancer.

In certain preferred embodiments, the tumor associated with the antibody or antigen-binding fragment thereof of the present invention is RORl-positive.

In another aspect, the present invention provides a method for preventing and/or treating a tumor in a subject. In another aspect, the present invention provides a method for delaying a tumor progression in a subject. In another aspect, the present invention provides a method for reducing or inhibiting a tumor recurrence in a subject. The methods described above comprise administering to a subject in need thereof an effective amount of the antibody or antigen-binding fragment thereof, vector, host cell, conjugate, chimeric antigen receptor, multispecific antibody, or pharmaceutical composition of the present invention.

In certain embodiments, the present invention provides a method for preventing and/or treating a tumor in a subject, comprising administering to a subject in need thereof an effective amount of the antibody or antigen-binding fragment thereof, vector, host cell, conjugate, chimeric antigen receptor, or multispecific antibody, and additional pharmaceutically active agent of the present invention; the additional pharmaceutically active agent may be administered separately, in combination, simultaneously, or sequentially.

In some preferred embodiments, the additional pharmaceutically active agent is one or more selected from the group consisting of: epidermal growth factor receptor (EGFR) inhibitor, immune checkpoint inhibitor, B cell antigen inhibitor, BTK inhibitor, chemotherapy drug.

In certain preferred embodiments, the additional pharmaceutically active agent may be one or more selected from the group consisting of anti-PDl/PD-Ll antibody, anti-CD20 antibody, and anti-EGFR antibody.

When the host cell of the present invention is used in the methods described above, the host cell expresses a chimeric antigen receptor comprising an antigen-binding fragment (e.g., ScFv) of the antibody of the present invention. Accordingly, in certain preferred embodiments, the isolated nucleic acid molecule contained in the host cell comprises a nucleotide sequence encoding a chimeric antigen receptor, the nucleotide sequence encoding the chimeric antigen receptor further comprising a nucleotide sequence encoding the antibody or antigen-binding fragment thereof of the present invention (e.g., ScFv). In certain preferred embodiments, the isolated nucleic acid molecule encodes a chimeric antigen receptor comprising an antigen-binding fragment (e.g., ScFv) of the antibody of the present invention. In certain preferred embodiments, the host cell is a T cell. In certain preferred embodiments, the host cell is a chimeric antigen receptor T cell (CAR-T).

In another aspect, the methods described above further comprise administering to the subject a second therapy, and the second therapy is selected from the group consisting of surgery, chemotherapy, radiotherapy, immunotherapy, gene therapy, DNA therapy, RNA therapy, nanotherapy, viral therapy, adjuvant therapy and any combination thereof.

In certain embodiments, the second therapy can be applied separately or in combination with the methods described above; alternatively, the second therapy can be applied separately or in combination, simultaneously or sequentially with the methods described above.

In some preferred embodiments, the tumor associated with the antibody or antigen-binding fragment thereof, vector, host cell, conjugate, chimeric antigen receptor or immune cell modified thereby, or multispecific antibody of the present invention is selected from the group consisting of lymphoma, breast cancer, colorectal cancer, ovarian cancer, lung cancer and/or pancreatic cancer.

In certain preferred embodiments, the tumor associated with the antibody or antigen-binding fragment thereof of the present invention is selected from the group consisting of lymphoma, breast cancer, colorectal cancer, ovarian cancer, lung cancer and/or pancreatic cancer.

In certain preferred embodiments, the tumor associated with the antibody or antigen-binding fragment thereof of the present invention is ROR1-positive.

The antibody or antigen-binding fragment thereof of the present invention and the pharmaceutical composition of the present invention can be formulated into any dosage form known in the medical field, for example, tablet, pill, suspension, emulsion, solution, gel, capsule, powder, granule, elixir, lozenge, suppository, injection (including injection solution, sterile powder for injection and concentrated solution for injection), inhalant, spray, etc. The preferred dosage form depends on the intended mode of administration and therapeutic use. The pharmaceutical composition of the present invention should be sterile and stable under the conditions of manufacture and storage. A preferred dosage form is injection. Such injection can be a sterile injectable solution. For example, the sterile injectable solution can be prepared by the following method: incorporating in an appropriate solvent a necessary dose of the recombinant protein of the present invention, optionally with other desired ingredients (including, but not limited to, pH adjusting agent, surfactant, adjuvant, ionic strength enhancer, isotonic agent, preservative, diluent, or any combination thereof), and then filtering for sterilization. In addition, the sterile injectable solution can be processed as a sterile lyophilized powder (e.g., by vacuum drying or freeze-drying) for ease of storage and use. Such sterile lyophilized powder can be dispersed in a suitable carrier, such as sterile pyrogen-free water, before use.

Furthermore, the antibody or antigen-binding fragment thereof of the present invention may be presented in a pharmaceutical composition in unit dosage form for ease of administration.

The antibody or antigen-binding fragment thereof, and pharmaceutical composition of the present invention may be administered by any suitable method known in the art, including but not limited to, oral, buccal, sublingual, ocular, topical, parenteral, rectal, intrathecal, intra-cisterna, inguinal, intravesical, local (e.g., powder, ointment, or drop), or nasal route. However, for many therapeutic uses, the preferred administration route/mode is parenteral administration (e.g., intravenous injection, subcutaneous injection, intraperitoneal injection, intramuscular injection). The skilled in the art will appreciate that the route and/or mode of administration will vary depending on the intended purpose. In a certain embodiment, the antibody or antigen-binding fragment thereof and pharmaceutical composition of the present invention is administered by intravenous injection or bolus injection.

The pharmaceutical composition of the present invention may comprise a "therapeutically effective amount" or "prophylactically effective amount" of the antibody or antigen-binding fragment thereof of the present invention. "Prophylactically effective amount" refers to an amount sufficient to prevent, arrest, or delay the onset of a disease. "Therapeutically effective amount" refers to an amount sufficient to cure or at least partially prevent the disease and its complications in a patient already suffering from the disease. The therapeutically effective amount of the antibody or antigen-binding fragment thereof of the present invention may vary depending on the severity of the disease to be treated, the general state of the patient's own immune system, the patient's general conditions such as age, weight and sex, the administration mode of drug, and other therapies administered at the same time, etc.

In the present invention, dosing regimens can be adjusted to obtain the optimum desired response (e.g., a therapeutic or prophylactic response). For example, a single dose can be administered; multiple doses can also be administered over a period of time; or the dose can be proportionally reduced or increased according to the level of urgency of the therapeutic situation.

In the present invention, the subject may be a mammal, such as a human.

### Detection method and kit

The antibody or antigen-binding fragment thereof of the present invention can specifically bind to ROR1, and thus can be used to detect the presence or level of ROR1 in a sample.

Accordingly, in another aspect, the present invention provides a kit, which comprises the antibody or antigen-binding fragment thereof of the present invention. In certain preferred embodiments, the antibody or antigen-binding fragment thereof of the present invention bears a detectable label. In a preferred embodiment, the kit further comprises a second antibody that specifically recognizes the antibody or antigen-binding fragment thereof of the present invention. Preferably, the second antibody further comprises a detectable label.

In the present invention, the detectable label can be any substance detectable by fluorescent, spectroscopic, photochemical, biochemical, immunological, electrical, optical or chemical means. It is particularly preferred that such labels are suitable for immunological detection (e.g., enzyme-linked immunoassay, radioimmunoassay, fluorescent immunoassay, chemiluminescent immunoassay, etc.). Such labels are well known in the art and include, but are not limited to, enzyme (e.g., horseradish peroxidase, alkaline phosphatase, β-galactosidase, urease, glucose oxidase, etc.), radionuclide (e.g., 3H, 125I, 35S, 14C, or 32P), fluorescent dye (e.g., fluorescein isothiocyanate (FITC), fluorescein, tetramethylrhodamine isothiocyanate (TRITC), phycoerythrin (PE), Texas Red, rhodamine, quantum dots or cyanine dye derivatives (e.g., Cy7, Alexa 750)), acridinium esters, magnetic beads (e.g., Dynabeads^{®}), calorimetric markers such as colloidal gold or colored glass or plastic (e.g., polystyrene, polypropylene, latex, etc.) beads, and biotin for binding to the above-mentioned label-modified avidin (e.g., streptavidin). Patents that teach the use of such labels include, but are not limited to, US Patents 3,817,837; 3,850,752; 3,939,350; 3,996,345; 4,277,437; 4,275,149; and 4,366,241 (all of which are incorporated herein by reference in their entirety). The detectable labels as described above can be detected by methods known in the art. For example, radioactive labels can be detected using photographic film or scintillation counter, and fluorescent labels can be detected using photodetector to detect emitted light. Enzyme labels are generally detected by providing a substrate and detecting a product produced by the action of the enzyme on the substrate, and thermometric labels are detected by simple visualization of a colored label. In certain embodiments, a detectable label as described above can be attached to the recombinant protein of the present invention via linkers of various lengths to reduce potential steric hindrance.

In another aspect, the present invention provides a method for detecting the presence or level of ROR1 in a sample, which comprises a step of using the antibody or antigen-binding fragment thereof of the present invention. In a preferred embodiment, the antibody or antigen-binding fragment thereof of the present invention further bears a detectable label. In another preferred embodiment, the method further comprises detecting the antibody or antigen-binding fragment thereof of the present invention by using a reagent bearing a detectable label. The method can be used for diagnostic purposes, or non-diagnostic purposes (e.g., the sample is a sample of cells rather than a sample from a patient).

In another aspect, the present invention provides a method for detecting the presence or level of ROR1 in a sample, and the method comprises contacting the sample with the antibody or antigen-binding fragment thereof of the present invention under the conditions that allow the antibody or antigen-binding fragment thereof to form a complex with ROR1, and detecting the formation of the complex.

In another aspect, there is provided a use of the antibody or antigen-binding fragment thereof of the present invention in the manufacture of a kit, and the kit is used for detecting the presence or level of ROR1 in a sample. In another aspect, the present invention provides a diagnostic or therapeutic kit, which comprises the antibody or antigen-binding fragment thereof, vector, host cell, conjugate, chimeric antigen receptor, or multispecific antibody of the present invention, and an instruction for use.

In view of the low or no expression of ROR1 in normal tissues and the expression or high expression in some cancers, tumors and tumor metastasis can be diagnosed by detecting the presence or level of ROR1 in samples. Therefore, the antibody or antigen-binding fragment thereof, conjugate, multispecific antibody, or kit of the present invention can be used for the diagnosis of a tumor and tumor metastasis. Therefore, in another aspect, there is provided a use of the antibody or antigen-binding fragment thereof, conjugate, multispecific antibody, or kit of the present invention in the diagnosis of a tumor and tumor metastasis.

The antibody of the present invention has a high binding affinity to ROR1 and has an extremely strong specificity. Therefore, the antibody of the present invention has the potential to be used for the prevention and/or treatment of a tumor. The humanized antibody of the present invention retains the functions and properties of the parental murine antibody. Moreover, the humanized antibody of the present invention has a high degree of humanization, so it can be safely administered to a human subject without causing an immunogenic reaction. Therefore, the antibody (especially humanized antibody) of the present invention has great clinical value.

### ABBREVIATION

- CDR: Complementarity determining region in immunoglobulin variable region
- FR: Antibody framework region: amino acid residues other than CDR residues in antibody variable region
- VH: Antibody heavy chain variable region
- VL: Antibody light chain variable region
- IgG: Immunoglobulin G
- AbM: The CDR definition of AbM comes from the related research of Martin (Martin ACR, Cheetham JC, Rees AR (1989) Modeling antibody hypervariable loops: A combined algorithm. Proc Natl Acad Sci USA 86: 9268-9272).
- IMGT: IMGT is based on the numbering system of the International ImMunoGeneTics Information System ^{®} (IMGT) initiated by Lefranc et al., see Lefranc et al., Dev. Comparat. Immunol. 27: 55-77, 2003.
- mAb: monoclonal antibody
- EC50: Concentration producing 50% efficacy or binding
- IC50: Concentration producing 50% inhibition
- ELISA: enzyme-linked immunosorbent assay
- PCR: polymerase chain reaction
- HRP: horseradish peroxidase
- K_{D}: equilibrium dissociation constant
- Ka: association rate constant
- Kd: dissociation rate constant
- ADCC: Antibody-dependent cytotoxicity
- CDC: Complement-dependent cytotoxicity
- FACS: Flow cytometry
- CDR-H1: Complementarity-determining region 1 in immunoglobulin heavy chain variable region
- CDR-H2: Complementarity-determining region 2 in immunoglobulin heavy chain variable region
- CDR-H3: Complementarity-determining region 3 in immunoglobulin heavy chain variable region
- CDR-L1: Complementarity-determining region 1 in immunoglobulin light chain variable region
- CDR-L2: Complementarity-determining region 2 in immunoglobulin light chain variable region
- CDR-L3: Complementarity-determining region 3 in immunoglobulin light chain variable region
- CAR: Chimeric antigen receptor
- CFA: Complete Freund's adjuvant
- IF A: Incomplete Freund's adjuvant

### DEFINITIONS

In the present invention, unless otherwise specified, scientific and technical terms used herein have the meanings commonly understood by those skilled in the art. In addition, the procedures of cell culture, biochemistry, nucleic acid chemistry, immunology, and the like used herein are all routine steps widely used in the corresponding fields. Meanwhile, for a better understanding of the present invention, definitions and explanations of related terms are provided below.

As used herein, the term "antibody" refers to an immunoglobulin molecule generally composed of two pairs of polypeptide chains (each pair having one light chain (LC) and one heavy chain (HC)). Antibody light chains can be classified as κ (kappa) and λ (lambda) light chains. Heavy chains can be classified as µ, δ, γ, α, or ε, and the isotypes of antibody can be defined as IgM, IgD, IgG, IgA, and IgE, respectively. In the light and heavy chains, the variable and constant regions are linked by a "J" region of about 12 or more amino acids, and the heavy chain also contains a "D" region of about 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. Constant domain is not directly involved in the binding of antibody to antigen, but exhibits a variety of effector functions, such as mediating the binding of immunoglobulin with host tissue or factor, including various cells (e.g., effector cells) of immune system to the first component (Clq) of classical complement system. The VH and VL regions can also be subdivided into regions of high variability (also called as complementarity determining regions (CDRs)), which are interspersed with more conserved regions called framework regions (FRs). Each VH and VL consists of 3 CDRs and 4 FRs arranged in the following order: FR1, CDR1, FR2, CDR2, FR3, CDR3, FR4 from amino terminus to carboxy terminus. The variable regions (VH and VL) of each heavy chain/light chain pair respectively form the antigen binding site. The assignment of amino acids to each region or domain can follow the definition by Kabat, Sequences of Proteins of Immunological Interest (National Institutes of Health, Bethesda, Md. (1987 and 1991)), or Chothia & Lesk (1987) J. Mol. Biol. 196: 901-917; Chothia et al. (1989) Nature 342:878-883.

Herein, unless the context clearly dictates otherwise, when the term "antibody" is referred to, it includes not only intact antibody but also an antigen-binding fragment of antibody.

As used herein, the term "complementarity determining region" or "CDR" refers to the amino acid residues in an antibody variable region that are responsible for antigen binding. The precise boundaries of these amino acid residues can be defined according to various numbering systems known in the art, for example according to the AbM numbering system (Martin ACR, Cheetham JC, Rees AR (1989) Modeling antibody hypervariable loops: A combined algorithm. Proc Natl Acad Sci USA 86: 9268-9272) or the IMGT numbering system (Lefranc et al., Dev. Comparat. Immunol. 27: 55-77, 2003). For a given antibody, those skilled in the art will readily identify the CDRs defined by each numbering system. Also, the correspondence between different numbering systems is well known to those skilled in the art (e.g., see Lefranc et al., Dev. Comparat. Immunol. 27: 55-77, 2003).

In the present invention, the CDRs contained in the antibody or antigen-binding fragment thereof of the present invention can be determined according to various numbering systems known in the art. In certain embodiments, CDRs of the antibody or antigen-binding fragment thereof of the present invention are preferably identified by the Kabat, Chothia, AbM, IMGT numbering system.

As used herein, the term "framework region" or "FR" residues refers to those amino acid residues in an antibody variable region other than the CDR residues as defined above.

A "germline antibody gene" is an immunoglobulin sequence encoded by a non-lymphoid cell, and it has not undergone the genetic rearrangement and maturation process leading to the expression of a specific immunoglobulin. One advantage provided by various embodiments of the present invention stems from the recognition that germline antibody genes retain more important amino acid sequence structures characterizing the individual of an animal species than mature antibody genes. It is therefore less recognized as a foreign substance by the species when applied therapeutically to that species.

The term "antibody" is not limited to any particular method for producing antibodies. For example, it includes recombinant antibody, monoclonal antibody and polyclonal antibody. The antibody can be of a different isotype, for example, an IgG (e.g., IgG1, IgG2, IgG3 or IgG4 subtype), IgAl, IgA2, IgD, IgE or IgM antibody.

As used herein, the term "antigen-binding fragment" of antibody refers to a polypeptide of a fragment of an antibody, such as a polypeptide of a fragment of a full-length antibody, which retains the ability to specifically bind the same antigen to which the full-length antibody binds, and/or competes with the full-length antibody for specific binding to the antigen, which is also referred to as an "antigen-binding portion". See generally, Fundamental Immunology, Ch. 7 (Paul, W., ed., 2nd ed., Raven Press, N.Y. (1989), which is incorporated herein by reference in its entirety for all purposes. The antigen-binding fragment of antibody can generate by recombinant DNA techniques or by enzymatic or chemical cleavage of intact antibody. Non-limiting examples of antigen-binding fragment include Fab, Fab', F(ab')₂, Fd, Fv, complementarity determining region (CDR) fragment, single chain antibody (e.g., scFv), chimeric antibody, diabody, linear antibody, nanobody (technology from Domantis), domain antibody (technology from Ablynx), and such polypeptides, which comprises at least a portion of an antibody sufficient to confer specific antigen binding ability on the polypeptide. Engineered antibody variants are reviewed in Holliger et al., 2005; Nat Biotechnol, 23: 1126-1136.

As used herein, the term "full-length antibody" refers to an antibody consisting of two "full-length heavy chains" or "heavy chains" and two "full-length light chains" or "light chains", wherein "full-length heavy chain" or "heavy chain" refers to a polypeptide chain consisting of a heavy chain variable region (VH), a heavy chain constant region CH1 domain, a hinge region (HR), a heavy chain constant region CH2 domain, a heavy chain constant region CH3 domain in the direction from N-terminal to C-terminal; and optionally further comprises a heavy chain constant region CH4 domain in the case of IgE isotype. Preferably, the "full-length heavy chain" is a polypeptide chain consisting of VH, CH1, HR, CH2 and CH3 in the direction from N-terminal to C-terminal. The "full-length light chain" or "light chain" is a polypeptide chain consisting of a light chain variable region (VL) and a light chain constant region (CL) in the N-terminal to C-terminal direction. The two pairs of full-length antibody chains are linked together by a disulfide bond between CL and CH1 and a disulfide bond between the HRs of the two full-length heavy chains. The full-length antibody of the present invention can be from a single species, such as a human; it can also be a chimeric antibody or a humanized antibody. The full-length antibody of the present invention comprises two antigen-binding sites formed by VH and VL pairs, respectively, which specifically recognizes/binds to the same antigen.

As used herein, the term "Fd fragment" refers to an antibody fragment consisting of VH and CH1 domains; the term "dAb fragment" refers to an antibody fragment consisting of VH domains (Ward et al., Nature 341:544 546 (1989)); the term "Fab fragment" refers to an antibody fragment consisting of VL, VH, CL and CH1 domains; the term "F(ab')₂ fragment" refers to an antibody fragment comprising two Fab fragments linked by a disulfide bridge on the hinge region; the term "Fab' fragment" refers to a fragment obtained by reducing the disulfide bond that links two heavy chain fragments in F(ab')₂ fragment, and consisting of an intact light chain and heavy chain Fd fragment (consisting of VH and CH1 domains).

As used herein, the term "Fv fragment" refers to an antibody fragment consisting of VL and VH domains of the single arm of antibody. Fv fragment is generally considered to be the smallest antibody fragment that can form an intact antigen-binding site. It is generally believed that the six CDRs confer antigen-binding specificity to an antibody. However, even a variable region (e.g., an Fd fragment, which contains only three antigen-specific CDRs) is able to recognize and bind to an antigen, albeit with possibly lower affinity than the intact binding site.

As used herein, the term "Fc" refers to an antibody fragment formed by binding the second and third constant regions of a first heavy chain to the second and third constant regions of a second heavy chain via a disulfide bond. The Fc fragment of antibody has many different functions, but is not involved in antigen binding.

As used herein, the term "scFv" refers to a single polypeptide chain comprising VL and VH domains, wherein the VL and VH are linked by a linker (see, for example, Bird et al., Science 242:423-426 (1988); Huston et al., Proc. Natl. Acad. Sci. USA 85:5879-5883 (1988); and Pluckthun, The Pharmacology of Monoclonal Antibodies, Vol. 113, Eds. Roseburg and Moore, Springer-Verlag, New York, pp. 269-315 (1994)). Such scFv molecule can have the general structure: NH₂-VL-linker-VH-COOH or NH₂-VH-linker-VL-COOH. A suitable linker in the prior art consists of repeated GGGGS amino acid sequences or variants thereof. For example, a linker with the amino acid sequence (GGGGS)₄, as well as variants thereof (Holliger et al. (1993), Proc. Natl. Acad. Sci. USA 90: 6444-6448) can be used. Other linkers useful in the present invention are described by Alfthan et al. (1995), Protein Eng. 8:725-731, Choi et al. (2001), Eur. J. Immunol. 31:94-106, Hu et al. (1996), Cancer Res. 56:3055-3061, Kipriyanov et al. (1999), J. Mol. Biol. 293:41-56 and Roovers et al. (2001), Cancer Immunol. In some cases, a disulfide bond may also exist between the VH and VL of scFv. As used herein, the term "di-scFv" refers to an antibody fragment formed by linking two scFvs.

As used herein, the term "diabody" refers to that its VH and VL domains are expressed on a single polypeptide chain, but the linker used is too short to allow pairing between the two domains of the same chain, this forces the domains to pair with the complementary domains of the other chain and create two antigen binding sites (see, for example, Holliger P. et al., Proc. Natl. Acad. Sci. USA 90:6444-6448 (1993), and Poljak R. J. et al., Structure 2:1121-1123 (1994)).

Each of the above antibody fragments maintains the ability to specifically bind to the same antigen to which the full-length antibody binds, and/or competes with the full-length antibody for specific binding to the antigen.

As used herein, the term "multispecific antibody" refers to an antibody with multiple different antigen-binding specificities, including, for example, bispecific antibody, trispecific antibody, and tetraspecific antibody. "Bispecific antibody" refers to an antibody with two different antigen-binding specificities, which is a conjugate formed by a first antibody (or a fragment thereof) and a second antibody (or a fragment thereof) or an antibody mimetic through a conjugation arm, the conjugation methods include but not limited to chemical reaction, gene fusion and enzymatic catalysis. "Multispecific antibody" includes, for example, trispecific antibody and tetraspecific antibody, in which the trispecific antibody refers to an antibody with three different antigen-binding specificities, and the tetraspecific antibody refers to an antibody with four different antigen-binding specificities.

As used herein, "antibody mimetic" refers to a substance that is capable of specific binding to antigen like an antibody, but has not an antibody structure. Examples thereof are usually artificial peptides or proteins with a molar mass of about 3 to 20 kDa. For example, ankyrin repeat protein (DARPin) and fynomer. The designed ankyrin repeat protein (DARPin) is linked with IgG antibody, scFv-Fc antibody fragment or a combination thereof, see for example, CN104341529A. Anti-IL-17a fynomer is linked with anti-IL-6R antibody, see for example, WO2015141862A1.

As used herein, "chimeric antigen receptor (CAR)" refers to a tumor antigen binding domain fused to an intracellular signaling domain, which is capable of activating T cells. Commonly, the extracellular binding domain of CAR is derived from a mouse or humanized or human monoclonal antibody.

As used herein, "immunoglobulin" or "Ig" may refer to a class of proteins that function as antibodies. Antibodies expressed by B cells are sometimes called chimeric antigen receptors or antigen receptors. The five members included in this class of proteins are IgA, IgG, IgM, IgD, and IgE. IgG is the most common circulating antibody, the most effective immunoglobulin in agglutination, complement fixation and other antibody responses and is important in defense against bacteria and viruses.

Herein, the technology of obtaining antibodies can use conventional techniques known to those of skill in the art (e.g., recombinant DNA techniques or enzymatic or chemical fragmentation methods) to obtain an antigen-binding fragment of antibody (e.g., the antibody fragment described above) from a given antibody (e.g., the antibody provided by the present invention), and the antigen-binding fragment of antibody can be screened for specificity in the same manner as that used for the intact antibody.

As used herein, the terms "monoclonal antibody", "McAb", "mAb" have the same meaning and are used interchangeably, and refer to one antibody or one fragment of antibody from a population of highly homologous antibody molecules, that is, a population of identical antibody molecules, except for natural mutations that may occur spontaneously. Monoclonal antibody is highly specific for a single epitope on an antigen. Polyclonal antibodies are relative to monoclonal antibody, which generally comprise at least two or more different antibodies that generally recognize different epitopes on an antigen. Furthermore, the modifier "monoclonal" only indicates that the antibody is characterized as being obtained from a population of highly homologous antibodies and should not be construed as requiring any particular method to prepare the antibody.

Monoclonal antibodies of the present invention can be prepared by a variety of techniques, such as hybridoma technology (see, for example, Kohler et al. Nature, 256:495, 1975), recombinant DNA technology (see, for example, U.S. Patent Application No. 4,816,567), or bacteriophage antibody library technology (see, for example, Clackson et al. Nature 352: 624-628, 1991, or Marks et al. J. Mol. Biol. 222: 581-597, 1991).

For example, the monoclonal antibody can be prepared as follows. Firstly, mice or other suitable host animals are immunized with an immunogen (added with adjuvant if necessary). The immunogen or adjuvant is usually injected subcutaneously at multiple points or intraperitoneally. The immunogen can be pre-conjugated to certain known proteins, such as serum albumin or soybean trypsin inhibitor, to enhance the immunogenicity of the antigen in the host. The adjuvant may be Freund's adjuvant or MPL-TDM or the like. After the animal is immunized, lymphocytes that secrete antibodies that specifically bind to the immunogen will be produced. In addition, lymphocytes can also be obtained by in vitro immunization. Lymphocytes of interest are collected and fused with myeloma cells using a suitable fusion agent, such as PEG, to obtain hybridoma cells (Goding, Monoclonal Antibodies: Principles and Practice, pp. 59-103, Academic Press, 1996). The hybridoma cells prepared above can be inoculated into a suitable culture medium for growth, and the culture medium preferably contains one or more substances capable of inhibiting the growth of unfused, parental myeloma cells. For example, for parental myeloma cells lacking hypoxanthine guanine phosphotransferase (HGPRT or HPRT), the addition of substances such as hypoxanthine, aminopterin, and thymine (HAT medium) to the culture medium will inhibit the growth of HGPRT-defective cells. The preferred myeloma cells should have the characteristics of high fusion rate, stable antibody secretion ability, and sensitivity to HAT medium. Among them, preferred myeloma cells are murine myeloma cells, such as MOP-21 or MC-11 mouse tumor-derived strains (THE Salk Institute Cell Distribution Center, San Diego, Calif. USA), and SP-2/0 or X63-Ag8-653 cell strain (American Type Culture Collection, Rockville, Md. USA). In addition, there are also studies reporting that human monoclonal antibodies were prepared using human myeloma and human-murine heteromyeloma cell strains (Kozbor, J. Immunol., 133: 3001 (1984); Brodeur et al., Monoclonal Antibody Production Techniques and Applications, pp. 51-63, Marcel Dekker, Inc., New York, 1987). The culture medium of growing hybridoma cells is used to detect the production of monoclonal antibodies against specific antigens. Methods for determining the binding specificity of monoclonal antibodies produced by hybridoma cells include, for example, immunoprecipitation or in vitro binding assays, such as radioimmunoassay (RIA), enzyme-linked immunosorbent assay (ELISA). For example, the affinity of mAbs can be determined using the Scatchard assay described by Munson et al., Anal. Biochem. 107: 220 (1980). After the specificity, affinity, and reactivity of the antibody produced by the hybridoma have been determined, the cell strain of interest can undergo subcloning by the standard limited dilution method described in Goding, Monoclonal Antibodies: Principles and Practice, pp. 59-103, Academic Press, 1996. The suitable medium can be DMEM or RPMI-1640 and the like. In addition, hybridoma cells can also grow in animals in the form of ascites tumors. Using traditional immunoglobulin purification methods, such as protein A agarose gel, hydroxyapatite chromatography, gel electrophoresis, dialysis or affinity chromatography, the monoclonal antibody secreted by subcloned cells can be purified from cell culture medium, ascites or serum.

The monoclonal antibody can also be obtained by genetic engineering recombinant technology. Using nucleic acid primers that specifically bind to the heavy chain and light chain genes of the monoclonal antibody to perform PCR amplification, the DNA molecules encoding the heavy chain and light chain genes of the monoclonal antibody can be isolated from the hybridoma cells. The obtained DNA molecule is inserted into an expression vector, then used to transfect a host cell (e.g., *E. coli* cell, COS cell, CHO cell, or other myeloma cells that does not produce immunoglobulin), and after being cultured under suitable conditions, the recombinantly expressed antibody of interest can be obtained.

The antibody can be purified by well-known techniques, such as affinity chromatography using protein A or protein G. Subsequently or alternatively, the specific antigen (the target molecule recognized by the antibody) or its epitope can be immobilized on a column, and the immunospecific antibody can be purified by immunoaffinity chromatography. The purification of immunoglobulin can be referred to, for example, D. Wilkinson (The Scientist, published by The Scientist, Inc., Philadelphia Pa., Vol. 14, No. 8 (Apr. 17, 2000), pp. 25-28).

As used herein, the term "murine antibody" refers to an antibody that is prepared by fusing B cells of immunized mice with myeloma cells, selecting murine hybrid fusion cells that can proliferate indefinitely and secrete the antibody, followed by screening, antibody preparation and antibody purification; or an antibody that is secreted by plasma cells which are formed by differentiation and proliferation of B cells after antigen invades the mouse body. For the antibody produced under the stimulation of specific antigen, the production of antibody is due to the interaction of various immune cells caused by the antigen invading the human body, resulting in differentiation and proliferation of B cells into plasma cells, which can produce and secrete the antibody.

As used herein, the term "chimeric antibody" refers to an antibody in which a part of light chain or/and heavy chain is derived from an antibody (which may be derived from a particular species or belong to a specific antibody class or subclass), and another part of the light chain or/and heavy chain is derived from another antibody (which may be derived from the same or different species or belong to the same or different antibody class or subclass), but in any event, it still retains binding activity to the target antigen (US Patent No. 4,816,567 to Cabilly et al.; Morrison et al., Proc. Natl. Acad. Sci. USA, 81:6851-6855 (1984)). For example, the term "chimeric antibody" can include such an antibody (e.g., human-murine chimeric antibody) in which the heavy chain and light chain variable regions of the antibody are derived from a first antibody (e.g., a murine antibody) and the heavy chain and light chain constant regions are derived from a second antibody (e.g., a human antibody).

As used herein, the term "humanized antibody" refers to a genetically engineered non-human antibody, of which the amino acid sequence has been modified to increase homology to the sequence of a human antibody. Typically, all or part of the CDRs of a humanized antibody are derived from a non-human antibody (a donor antibody), and all or part of the non-CDR regions (e.g., variable FR and/or constant regions) are derived from a human immunoglobulin (a receptor antibody). Humanized antibodies generally retain the expected properties of the donor antibody, including, but not limited to, antigen specificity, affinity, reactivity, ability to enhance immune cell activity, ability to enhance immune response, and the like. The donor antibody can be a mouse, rat, rabbit, or non-human primate (e.g., cynomolgus monkey) antibody with the expected properties (e.g., antigen specificity, affinity, reactivity, ability to enhance immune cell activity and/or ability to enhance immune response, etc.).

Humanized antibody can retain the expected properties of a non-human donor antibody (e.g., murine antibody), and can effectively reduce the immunogenicity of the non-human donor antibody (e.g., murine antibody) in a human subject, so that it is particularly advantageous. However, due to matching issues between the CDRs of the donor antibody and the FRs of the receptor antibody, the expected properties (e.g., antigen specificity, affinity, reactivity, ability to increase immune cell activity, and/or ability to enhance the immune response) of the humanized antibody are generally lower than those of the non-human donor antibody (e.g., murine antibody).

Thus, although the humanization of antibodies has been intensively studied and some progress has been made by researchers in the art (see, for example, Jones et al., Nature, 321:522-525 (1986); Reichmann et al., Nature, 332:323-329 (1988); Presta, Curr. Op. Struct. Biol., 2:593-596 (1992); and Clark, Immunol. Today 21: 397-402 (2000)), there is still no detailed guidance in the art about how to perform a complete humanization of a certain donor antibody, so that the humanized antibody produced not only has the highest possible degree of humanization, but also retain the expected properties of the donor antibody as much as possible. Technicians have to perform exploration, investigation and modification for a specific donor antibody, and make a lot of creative efforts to obtain a humanized antibody that not only has a high degree of humanization (e.g., a humanization degree of at least 75%, at least 80%, at least 85%, at least 90%, at least 91%, at least 92%, at least 93%, at least 94%, at least 95%, at least 96%, at least 97%, at least 98%, at least 99%), but also retains the expected properties of the specific donor antibody.

In the present invention, in order for the humanized antibody to retain the properties of the donor antibody (including, for example, antigen specificity, affinity, reactivity, ability to enhance immune cell activity and/or ability to enhance immune response) as much as possible, the framework region (FR) of the humanized antibody of the present invention may contain both the amino acid residues of the human receptor antibody and the amino acid residues of the corresponding non-human donor antibody.

The chimeric antibody or humanized antibody of the present invention can be prepared by using the sequence of the mouse monoclonal antibody prepared above. The DNA encoding the heavy and light chains can be obtained from the target murine hybridoma and engineered to contain non-murine (e.g., human) immunoglobulin sequences using standard molecular biology techniques.

In order to prepare the chimeric antibody, murine immunoglobulin variable regions can be linked to human immunoglobulin constant regions using methods known in the art (see, for example, US Patent No. 4,816,567 to Cabilly et al.). For example, the DNA encoding VH is operably linked to another DNA molecule encoding the heavy chain constant region to obtain a full-length heavy chain gene. The sequences of human heavy chain constant region genes are known in the art (see, for example, Kabat, E.A. et al. (1991), Sequences of Proteins of Immunological Interest, Fifth Edition, U.S. Department of Health and Human Services, NIH Publication No. 91-3242), and the DNA fragments containing these regions can be obtained by standard PCR amplification. The heavy chain constant region may be IgG1, IgG2, IgG3, IgG4, IgA, IgE, IgM or IgD constant region, but is generally preferably IgG1 or IgG4 constant region. For example, the DNA encoding VL is operably linked to another DNA molecule encoding the light chain constant region CL to obtain a full-length light chain gene (as well as a Fab light chain gene). The sequences of human light chain constant region genes are known in the art (see, for example, Kabat, E.A. et al. (1991), Sequences of Proteins of Immunological Interest, Fifth Edition, U.S. Department of Health and Human Services, NIH Publication No. 91-3242), and the DNA fragments containing these regions can be obtained by standard PCR amplification. The light chain constant region may be κ or λ constant region, but generally and preferably κ constant region.

In order to prepare the humanized antibody, murine CDRs can be grafted into human framework sequences using methods known in the art (see U.S. Patent No. 5,225,539 to Winter; U.S. Patent No. 5,530,101; 5,585,089; 5,693,762 and 6,180,370 to Queen et al; and Lo, Benny, K.C., editor, in Antibody Engineering: Methods and Protocols, volume 248, Humana Press, New Jersey, 2004). Alternatively, transgenic animals can also be utilized, which are capable of and producing complete human antibody library without producing an endogenous immunoglobulin following immunization. For example, it has been reported that the homozygous deletion of antibody heavy chain joining region (JH) gene in chimeric and germline mutant mice could completely suppress the production of endogenous antibody, then the human germline immunoglobulin gene arrays could be transferred to the germline mutant mice, which will result in the production of human antibodies upon antigenic stimulation (see, for example, Jakobovits et al., 1993, Proc. Natl. Acad. Sci. USA 90:2551; Jakobovits et al., 1993, Nature 362:255-258; Bruggermann et al., 1993, Year in Immunology 7:33; and Duchosal et al., 1992, Nature 355:258). The non-limiting examples of the above transgenic animals include, HuMAb mice (Medarex, Inc.) that contain human immunoglobulin gene miniloci encoding unrearranged human heavy chain (µ and γ) and κ light chain immunoglobulin sequences, and targeted mutations that inactivate endogenous µ and κ chain loci (see, for example, Lonberg et al. (1994), Nature 368(6474):856-859); or "KM mouse TM" that carries a human heavy chain transgene and human light chain transchromosome (see patent application WO02/43478). Other methods for humanizing antibodies include phage display technology (Hoogenboom et al., 1991, J. Mol. Biol. 227:381; Marks et al., J. Mol. Biol. 1991, 222:581-597; Vaughan et al., 1996, Nature Biotech 14:309).

As used herein, the term "humanization degree " is an indicator used to evaluate the number of non-human amino acid residues in a humanized antibody. The degree of humanization of the humanized antibody can be used, for example, by the Domain Gap Align of IMGT website, to predict the homology of the variable region sequence to the human V domain.

As used herein, "homologous antibody" refers to a variant of an antibody, which contains heavy and light chain variable regions comprising amino acid sequences homologous with the amino acid sequences of the antibody or antigen-binding fragment thereof provided herein, and retains the desired functional properties of the anti-ROR1 antibody of the present invention.

Methods of alignment of sequences for comparison are well known in the art. Various programs and alignment algorithms are described in: Smith TF and Waterman MS, Adv. Appl. Math., 2: 482, 1981; Higgins DG and Sharp PM, CABIOS 5: 151, 1989. Altschul SF et al., Nature Genet., 6: 119, 1994 provides detailed ideas on sequence alignment methods and homology calculations.

As used herein, the term "specific binding" refers to a non-random binding reaction between two molecules, such as the reaction between an antibody and its antigen. The strength or affinity of a specific binding interaction can be expressed in terms of the equilibrium dissociation constant (KD) or half-maximal effect concentration (EC50) for that interaction.

The specific binding property between two molecules can be determined by using methods well known in the art. One of the methods involves measuring the rate of formation and dissociation of antigen binding site/antigen complex. Both the "association rate constant" (ka or kon) and the "dissociation rate constant" (kdis or koff) can be calculated from the concentrations and the actual rates of association and dissociation (see, Malmqvist M, Nature, 1993, 361 :186-187). The ratio of kdis/kon is equal to the dissociation constant KD (see, Davies et al., Annual Rev Biochem, 1990; 59:439-473). The values of KD, kon and kdis can be measured by any valid methods. In certain embodiments, the dissociation constant can be measured using bioluminescence interferometry method (e.g., ForteBio Octet method). In addition, surface plasmon resonance technology (e.g., Biacore) or Kinexa can be used to measure the dissociation constant.

As used herein, the term "vector" refers to a nucleic acid delivery vehicle into which a polynucleotide can be inserted. When the vector can express the protein encoded by the inserted polynucleotide, the vector is called an expression vector. The vector can be introduced into a host cell by transformation, transduction or transfection, so that the genetic material element carried thereby can be expressed in the host cell. Vectors are well known to those skilled in the art and include, but are not limited to: plasmids; phagemid; cosmid; artificial chromosome, such as yeast artificial chromosome (YAC), bacterial artificial chromosome (BAC) or P1 derived artificial chromosome (PAC); phage such as λ phage or M13 phage and animal virus, etc. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40). A vector may contain a variety of elements that control expression, including, but not limited to, promoter sequence, transcription initiation sequence, enhancer sequence, selection element, and reporter gene. Additionally, the vector may also contain an origin of replication site.

Expression and cloning vectors contain a nucleic acid sequence that enables the vector to replicate in one or more selected host cells. Typically, in a cloning vector, this sequence is one that enables the replication of vector independent from the host chromosomal DNA, and it includes a replication origin site or autonomously replicating sequence. The term "expression vector" as used herein refers to a vector comprising a recombinant polynucleotide, which comprises an expression control sequence operably linked to a nucleotide sequence to be expressed. Expression vector comprises sufficient cis-acting elements for expression; other elements for expression may be provided by the host cell or by an in vitro expression system. Expression vectors include all those known in the art, such as cosmid, plasmid (e.g., naked or contained in liposome), and virus (e.g., lentivirus, retrovirus, adenovirus, and adeno-associated virus).

As used herein, the term "host cell" refers to a cell into which a vector can be introduced, and includes, but is not limited to, prokaryotic cell such as *Escherichia coli* or *Bacillus subtilis*, fungal cell such as yeast cell or *Aspergillus*, insect cell such as *S2 Drosophila* cell or Sf9, or animal cell such as fibroblast, NS0 cell, Vero cell, Hela cell, COS cell, CHO cell (e.g., CHO-K1, CHO-S, CHO DXB11, ExpiCHO, CHO DG44 cell), ExpiCHO cell, HEK293 cell, Expi293 cell, BHK cell, and MDCKII cell and so on. Herein, the host cell also includes an immune cell used to construct a chimeric antigen receptor T cell (CAR-T), such as T lymphocyte, NK cell, etc.

As used herein, the term "identity" refers to the match degree between two polypeptides or between two nucleic acids. When two sequences for comparison have the same monomer sub-unit of base or amino acid at a certain site (e.g., each of two DNA molecules has an adenine at a certain site, or each of two polypeptides has a lysine at a certain site), the two molecules are identical at the site. The percent identity between two sequences is a function of the number of identical sites shared by the two sequences over the total number of sites for comparison × 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by using a computer program such as Align program (DNAstar, Inc.) which is based on the method of Needleman, et al. (J. Mol. Biol. 48:443-453, 1970). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

As used herein, the term "conservative substitution" refers to an amino acid substitution that does not adversely affect or alter the intended properties of the protein/polypeptide comprising the amino acid sequence. For example, a conservative substitution can be introduced by standard techniques known in the art such as site-directed mutagenesis and PCR-mediated mutagenesis. Conservative amino acid substitutions include substitutions of amino acid residues with amino acid residues that have similar side chains, e.g., substitution with residues that are physically or functionally similar to the corresponding amino acid residues (e.g., having similar size, shape, charge, chemical properties, including the ability to form covalent bonds or hydrogen bonds, etc.). Families of amino acid residues with similar side chains have been defined in the art. These families include those with basic side chain (e.g., lysine, arginine, and histidine), acidic side chain (e.g., aspartic acid, glutamic acid), uncharged polar side chain (e.g., glycine, asparagine, glutamine, serine, threonine, tyrosine, cysteine, tryptophan), non-polar side chain (e.g., alanine, valine, leucine, isoleucine, proline, phenylalanine, methionine), β branched side chain (e.g., threonine, valine, isoleucine), and aromatic side chain (e.g., tyrosine, phenylalanine, tryptophan, histidine). Therefore, it is preferred to replace the corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al., Protein Eng. 12(10):879-884 (1999) and Burks et al., Proc. Natl Acad. Set USA 94:412-417 (1997), which are incorporated herein by reference).

The twenty conventional amino acids referred to herein have been written following conventional usage. See, for example, Immunology-A Synthesis (2nd Edition, E. S. Golub and D. R. Gren, Eds., Sinauer Associates, Sunderland, Mass. (1991)), which are incorporated herein by reference. In the present invention, the terms "polypeptide" and "protein" have the same meaning and are used interchangeably. And in the present invention, amino acids are generally represented by one-letter and three-letter abbreviations well known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient. It is well known in the art (see, for example, Remington's Pharmaceutical Sciences. Edited by Gennaro AR, 19th ed. Pennsylvania: Mack Publishing Company, 1995) and includes, but is not limited to: pH adjuster, surfactant, adjuvant, ionic strength enhancing agent, diluent, agent for maintaining osmotic pressure, agent for delaying absorption, preservative. For example, the pH adjusting agent includes, but is not limited to, phosphate buffer. The surfactant includes, but is not limited to, cationic, anionic or nonionic surfactant, such as Tween-80. The ionic strength enhancing agent includes, but is not limited to, sodium chloride. The preservative includes, but is not limited to, various antibacterial and antifungal agent, such as paraben, chloretone, phenol, sorbic acid, and the like. The agent for maintaining osmotic pressure includes, but is not limited to, sugar, NaCl, and the like. The agent for delaying absorption includes, but is not limited to, monostearate salt and gelatin. The diluent includes, but is not limited to, water, aqueous buffer (e.g., buffered saline), alcohol and polyol (e.g., glycerol), and the like. The preservative includes, but is not limited to, various antibacterial and antifungal agent, such as thimerosal, 2-phenoxyethanol, paraben, chloretone, phenol, sorbic acid, and the like. The stabilizer has the meaning commonly understood by those skilled in the art, which is capable of stabilizing the desired activity of the active ingredient in the drug, including but not limited to sodium glutamate, gelatin, SPGA, saccharide (e.g., sorbitol, mannitol, starch, sucrose, lactose, glucan, or glucose), amino acid (e.g., glutamic acid, glycine), protein (e.g., dry whey, albumin, or casein) or degradation product thereof (e.g., lactalbumin hydrolyzate), etc.

As used herein, the term "prevention" refers to a method performed to prevent or delay the occurrence of a disease or disorder or symptom (e.g., a tumor) in a subject. As used herein, the term "treatment" refers to a method performed to obtain a beneficial or desired clinical outcome. For the purposes of the present invention, a beneficial or desired clinical outcome includes, but is not limited to, alleviation of symptom, reduction in the extent of disease, stabilization (i.e., not worsening) of disease state, delaying or slowing the progression of disease, amelioration or remission of disease state, and relief of symptom (whether in part or in whole), whether detectable or undetectable. In addition, "treatment" can also mean prolonging survival as compared to expected survival if not receiving treatment.

As used herein, the term "subject" refers to a mammal, such as a primate, such as a human. In certain embodiments, the subject (e.g., a human) has a tumor, or is at a risk for the aforementioned disease.

As used herein, the term "effective amount" refers to an amount sufficient to obtain, or at least partially obtain, the desired effect. For example, an effective amount for preventing a disease (e.g., a tumor) refers to an amount sufficient to prevent, arrest, or delay the onset of the disease (e.g., tumor); an effective amount for treating a disease refers to an amount sufficient to cure or at least partially prevent the disease and complication thereof in a patient with the disease. Determining such effective amounts is well within the ability of those skilled in the art. For example, an effective amount for therapeutic use will depend on the severity of disease to be treated, the general state of patient's own immune system, the patient's general condition such as age, weight and sex, the mode of administration of drug, and other concurrently administered treatments and so on.

As used herein, the term "effector function" refers to a biological activity that can be attributed to Fc region of an antibody (Fc region with natural sequence or amino acid sequence variant), and varies with isotype of antibody. Examples of antibody effector function include, but are not limited to: Fc receptor binding affinity, antibody-dependent cell-mediated cytotoxicity (ADCC), complement-dependent cytotoxicity (CDC), antibody-dependent cellular phagocytosis (ADCP), downregulation of cell surface receptor (e.g., B cell receptor), B cell activation, cytokine secretion, half-life/clearance rate of antibody and antigen-antibody complex, etc. Methods for altering the effector function of antibody are known in the art, for example by introducing a mutation into the Fc region.

As used herein, the term "antibody-dependent cell-mediated cytotoxicity (ADCC)" refers to a form of cytotoxicity in which cytotoxic effector cells specifically bind to the target cells to which the antigen is attached, through the binding of Ig to an Fc receptor (FcR) presented on cytotoxic cells (e.g., natural killer (NK) cells, neutrophils or macrophages), and then kills the target cells by secreting cytotoxins. Methods for detecting the ADCC activity of antibody are known in the art, for example, it can be evaluated by measuring the binding activity between the antibody to be tested and an Fc receptor (e.g., CD16a).

As used herein, the term "complement-dependent cytotoxicity (CDC)" refers to a form of cytotoxicity in which the complement cascade is activated by binding of a complement component C1q to Fc of an antibody. Methods for detecting the CDC activity of an antibody are known in the art, for example, it can be evaluated by measuring the binding activity between the antibody to be tested and an Fc receptor (e.g., C1q).

As used herein, the term "pharmaceutically acceptable" means that when molecular entities, molecular fragments or compositions are properly administered to animals or humans, they do not produce unfavorable, allergic or other adverse reactions. Specific examples of some substances that can be used as pharmaceutically acceptable carriers or components thereof include sugar (e.g., lactose), starch, cellulose and derivative thereof, vegetable oil, gelatin, polyol (e.g., propylene glycol), alginic acid and the like.

As used herein, the combination therapy comprises a combination use of the anti-ROR1 antibody or antigen-binding fragment thereof encompassed by the present invention with one or more additional active therapeutic agents (e.g., chemotherapeutic agents) of a second therapy or other modes of prophylaxis or treatment (e.g., radiation therapy).

In such combination therapies, the various active agents often have different complementary mechanisms of action, and the combination therapies may result in a synergistic effect. The combination therapy includes a therapeutic agent that affects the immune response (e.g., enhances or activates the response) and a therapeutic agent that affects (e.g., inhibits or kills) tumor/cancer cells. The combination therapy reduces the likelihood of occurrence of drug-resistant cancer cells. The combination therapy may allow dose reduction of one or more agents, thereby reducing or eliminating adverse effects associated with the one or more agents. Such combination therapies may have a synergistic therapeutic or prophylactic effect on the potential disease, disorder or condition.

As used herein, "combination" comprises therapies that may be administered separately, for example, formulated separately for separate administration (e.g., may be provided in a kit), as well as therapies that may be administered together in a single formulation (i.e., "co-formulation"). In certain embodiments, the anti-ROR1 antibody or antigen-binding fragment thereof of the present invention may be administered sequentially. In other embodiments, the anti-ROR1 antibody or antigen-binding fragment thereof may be administered concurrently. The anti-ROR1 antibody or antigen-binding fragment thereof of the present invention may be used in any combination with at least one other (active) agent.

In this context, ROR1-positive is obtained by immunohistochemistry and evaluation of staining intensity by professional clinical pathologists.

The terms "cancer" and "tumor" are used interchangeably and refer to a broad group of diseases characterized by the uncontrolled growth of abnormal cells in the body. Unregulated cell division may lead to the formation of malignant tumors or cells that invade adjacent tissues and may metastasize to distant sites in the body through the lymphatic system or bloodstream. The cancer includes benign and malignant cancers as well as dormant tumors or micrometastases. The cancer also includes hematological malignancies.

The term "hematologic malignancies" includes lymphomas, leukemia, myeloma or lymphoid malignancies, as well as spleen cancer and lymph node tumors. Exemplary lymphomas include B-cell lymphoma and T-cell lymphoma. B-cell lymphomas include, for example, Hodgkin's lymphoma. T-cell lymphomas include, for example, cutaneous T-cell lymphoma. Hematological malignancies further include leukemia, such as secondary leukemia or acute lymphoblastic leukemia. Hematological malignancies further include myeloma (e.g., multiple myeloma) and other hematological and/or B-cell or T-cell related cancers.

Embodiments of the present invention will be described in detail below with reference to the drawings and examples, but those skilled in the art will understand that the following drawings and examples are only for illustrating the present invention, rather than limiting the scope of the present invention. Various objects and advantages of the present invention will become apparent to those skilled in the art from the accompanying drawings and the following detailed description of preferred embodiments.

### BRIEF DESCRIPTION OF THE DRAWINGS

FIG. 1 shows the detection of overexpression of human ROR1 protein of Ba/F3 cells.
FIG. 2 shows the detection of mouse-anti-human ROR1 antibody binding to ROR1-positive cells.
FIG. 3 shows the detection of mouse-anti-human ROR1 antibody binding to human ROR2 extracellular domain protein.
FIG. 4 shows the detection of anti-human ROR1 chimeric antibody binding to cells.
FIG. 5 shows the detection of ADCC activity of anti-human ROR1 humanized candidate antibody.
FIG. 6 shows the detection of anti-human ROR1 candidate antibody-induced internalization activity (Mab-ZAP method).
FIG. 7A to 7B show the detection of internalization activity of anti-human ROR1 candidate antibody (acid wash method).
FIG. 8 shows the detection of internalization activity of anti-human ROR1 candidate antibody (pHrodo dye (Thermo) method).

### SEQUENCE INFORMATION

Information on the sequences involved in the present invention is described in the following table:

| SEQ ID NO | Description | SEQ ID NO | Description |
|---|---|---|---|
| 1 | Murine antibody 3D8.2.3 heavy chain variable region | 83 | Chothia 38F8.1.1/38F8-Hu14/ 38F8-Hul6/ 38F8-Hul7/ 38F8-Hu18/ 38F8-Hu24/ 38F8-Hu26/ 38F8-Hu27/ 38F8-Hu28/ 38F8-Hu34/ 38F8-Hu36/ 38F8-Hu37/ 38F8-Hu38/ 38F8-Hu54/ 38F8-Hu56/ 38F8-Hu57/ 38F8-Hu58/ 38F8-Hu64/ 38F8-Hu66/ 38F8-Hu67/ 38F8-Hu68 CDR-H2 |
| 2 | Murine antibody 3D8.2.3 light chain variable region | 84 | Humanized antibody 19F6-Hu 11 / 19F6-Hu 12/ 19F6-Hu13/19F6-Hu14/19F6-Hu15 heavy chain variable region |
| 3 | Murine antibody 19F6.2.3 heavy chain variable region | 85 | Humanized antibody 19F6-Hu21 / 19F6-Hu22/ 19F6-Hu23/19F6-Hu24/19F6-Hu25 heavy chain |
| 4 | Murine antibody 19F6.2.3 light chain variable region | 86 | Humanized antibody 19F6-Hu31/19F6-Hu32/19F6-Hu33/19F6-Hu34/19F6-Hu35/19F6-Hu35V1/19F6-Hu35V2/19F6-Hu35 V3/19F6-Hu3 5V4/19F6-Hu35V5/19F6-Hu35V6/19F6-Hu35 V7/19F6-Hu3 5V8/19F6-Hu35V9/19F6-Hu35V10 heavy chain variable region |
| 5 | Murine antibody 38F8.1.1 heavy chain variable region | 87 | Humanized antibody 19F6-Hu11/19F6-Hu21/19F6-Hu31 light chain variable region |
| 6 | Murine antibody 38F8.1.1 light chain variable region | 88 | Humanized antibody 19F6-Hu12/19F6-Hu22/19F6-Hu32 light chain variable region |
| 7 | IMGT 3D8.2.3/3D8-HuC24/ 3D8-Hu25/ 3D8-Hu26/ 3D8-Hu27/ 3D8-HuC44/3D8-Hu45/ 3D8-Hu46/3D8-Hu47/3D8-HuC54/3D8-Hu55/3D8-Hu56/3D8-Hu57/3D8-HuC84/3D8-Hu85/3D8-Hu86/3D8-Hu87/3D8-HuC94/3D8-Hu95/3D8-Hu96/3D8-Hu97 CDR-H1 | 89 | Humanized antibody 19F6-Hu13/19F6-Hu23/19F6-Hu33 light chain variable region |
| 8 | IMGT 3D8.2.3/ 3D8-HuC24/ 3D8-Hu25/ 3D8-Hu26/ 3D8-Hu27/ 3D8-HuC44/ 3D8-Hu45/ 3D8-Hu46/ 3D8-Hu47/3D8-HuC54/ 3D8-Hu55/ 3D8-Hu56/ 3D8-Hu57 CDR-H2 | 90 | Humanized antibody 19F6-Hu14/19F6-Hu24/19F6-Hu34 light chain variable region |
| 9 | IMGT 3D8-HuC84/ 3D8-Hu85/ 3D8-Hu86/ 3D8-Hu87 CDR-H2 | 91 | Humanized antibody 19F6-Hu15/19F6-Hu25/19F6-Hu35 light chain variable region |
| 10 | IMGT 3D8-HuC94/ 3D8-Hu95/ 3D8-Hu96/ 3D8-Hu97 CDR-H2 | 92 | Humanized antibody 19F6-Hu35Vl light chain variable region |
| 11 | IMGT 3D8.2.3/3D8-HuC24/ 3D8-Hu25/ 3D8-Hu26/ 3D8-Hu27/ 3D8-HuC44/3D8-Hu45/ 3D8-Hu46/3D8-Hu47/3D8-HuC54/3D8-Hu55/3D8-Hu56/3D8-Hu57/3D8-HuC84/3D8-Hu85/3D8-Hu86/3D8-Hu87/3D8-HuC94/3D8-Hu95/3D8-Hu96/3D8-Hu97 CDR-H3 | 93 | Humanized antibody 19F6-Hu35V2 light chain variable region |
| 12 | IMGT 3D8.2.3/3D8-Hu25/ 3D8-Hu26/3D8-Hu45/ 3D8-Hu46/3D8-Hu55/3D8-Hu56/3D8-Hu85/3D8-Hu86/3D8-Hu95/3D8-Hu96 CDR-L1 | 94 | Humanized antibody 19F6-Hu35V3 light chain variable region |
| 13 | IMGT 3D8-HuC24/ 3D8-HuC44 /3D8-HuC54/3D8-HuC84/3D8-HuC94/3D8-Hu27/3D8-Hu47/3D8-Hu57/3D8-Hu87/3D8-Hu97 CDR-L1 | 95 | Humanized antibody 19F6-Hu35V4 light chain variable region |
| 14 | IMGT 3D8.2.3/3D8-HuC24/ 3D8-Hu25/ 3D8-Hu26/ 3D8-Hu27/ 3D8-HuC44/3D8-Hu45/ 3D8-Hu46/3D8-Hu47/3D8-HuC54/3D8-Hu55/3D8-Hu56/3D8-Hu57/3D8-HuC84/3D8-Hu85/3D8-Hu86/3D8-Hu87/3D8-HuC94/3D8-Hu95/3D8-Hu96/3D8-Hu97 CDR-L2 | 96 | Humanized antibody 19F6-Hu35V5 light chain variable region |
| 15 | IMGT/Abm/Kabat/Chothia 3D8.2.3/3D8-HuC24/ 3D8-Hu25/ 3D8-Hu26/ 3D8-Hu27/ 3D8-HuC44/3D8-Hu45/ 3D8-Hu46/3D8-Hu47/3D8-HuC54/3D8-Hu55/3D8-Hu56/3D8-Hu57/3D8-HuC84/3D8-Hu85/3D8-Hu86/3D8-Hu87/3D8-HuC94/3D8-Hu95/3D8-Hu96/3D8-Hu97/38F8.1.1/38F8-Hul4/ 38F8-Hu16/ 38F8-Hu17/ 38F8-Hu18/ 38F8-Hu24/ 38F8-Hu26/ 38F8-Hu27/ 38F8-Hu28/ 38F8-Hu34/ 38F8-Hu36/ 38F8-Hu37/ 38F8-Hu38/ 38F8-Hu54/ 38F8-Hu56/ 38F8-Hu57/ 38F8-Hu58/ 38F8-Hu64/ 38F8-Hu66/ 38F8-Hu67/ 38F8-Hu68 CDR-L3 | 97 | Humanized antibody 19F6-Hu35V6 light chain variable region |
| 16 | AbM 3D8.2.3/3D8-HuC24/ 3D8-Hu25/ 3D8-Hu26/ 3D8-Hu27/ 3D8-HuC44/3D8-Hu45/ 3D8-Hu46/3D8- | 98 | Humanized antibody 19F6-Hu35V7 light chain variable region |
| | Hu47/3D8-HuC54/3D8-Hu55/3D8-Hu56/3D8-Hu57/3D8-HuC84/3D8-Hu85/3D8-Hu86/3D8-Hu87/3D8-HuC94/3D8-Hu95/3D8-Hu96/3D8-Hu97 CDR-H1 | | |
| 17 | AbM 3D8.2.3/3D8-HuC24/ 3D8-Hu25/ 3D8-Hu26/ 3D8-Hu27/ 3D8-HuC44/3D8-Hu45/ 3D8-Hu46/3D8-Hu47/3D8-HuC54/3D8-Hu55/3D8-Hu56/3D8-Hu57 CDR-H2 | 99 | Humanized antibody 19F6-Hu35V8 light chain variable region |
| 18 | AbM 3D8-HuC84/3D8-Hu85/3D8-Hu86/3D8-Hu87 CDR-H2 | 100 | Humanized antibody 19F6-Hu35V9 light chain variable region |
| 19 | AbM 3D8-HuC94/3D8-Hu95/3D8-Hu96/3D8-Hu97 CDR-H2 | 101 | Humanized antibody 19F6-Hu35V10 light chain variable region |
| 20 | AbM/Kabat/Chothia 3D8.2.3/3D8-HuC24/ 3D8-Hu25/ 3D8-Hu26/ 3D8-Hu27/ 3D8-HuC44/3D8-Hu45/ 3D8-Hu46/3D8-Hu47/3D8-HuC54/3D8-Hu55/3D8-Hu56/3D8-Hu57/3D8-HuC84/3D8-Hu85/3D8-Hu86/3D8-Hu87/3D8-HuC94/3D8-Hu95/3D8-Hu96/3D8-Hu97 CDR-H3 | 102 | Humanized antibody 3D8-HuC24/3D8-Hu25/3D8-Hu26 /3D8-Hu27 heavy chain variable region |
| 21 | AbM/Kabat/Chothia 3D8.2.3/ CDR-L1 | 103 | Humanized antibody 3D8-HuC44/3D8-Hu45/3D8-Hu46 /3D8-Hu47 heavy chain variable region |
| 22 | AbM/Kabat/Chothia 3D8-Hu25/3D8-Hu26/3D8-Hu45/3D8-Hu46/3D8-Hu55/3D8-Hu56/3D8-Hu85/3D8-Hu86/3D8-Hu95/3D8-Hu96 CDR-L1 | 104 | Humanized antibody 3D8-HuC 54/3D8-Hu55/3D8-Hu56 /3D8-Hu57 heavy chain variable region |
| 23 | AbM/Kabat/Chothia 3D8-Hu27/3D8-Hu47/3D8-Hu57/3D8-Hu87/3D8-Hu97 CDR-L1 | 105 | Humanized antibody 3D8-HuC84/3D8-Hu85/3D8-Hu86 /3D8-Hu87 heavy chain variable region |
| 24 | AbM/Kabat/Chothia 3D8.2.3/38F8.1.1/ CDR-L2 | 106 | Humanized antibody 3D8-HuC94/3D8-Hu95/3D8-Hu96 /3D8-Hu97 heavy chain variable region |
| 25 | AbM/Kabat/Chothia 3D8-HuC24/ 3D8-HuC44 /3D8-HuC54/3D8-HuC84/3D8-HuC94/3D8-Hu25/3D8-Hu26/3D8-Hu27/3D8-Hu45/3D8-Hu46/3D8-Hu47/3D8-Hu55/3D8-Hu56/3D8-Hu57/3D8-Hu85/3D8-Hu86/3D8-Hu87/3D8-Hu95/3D8-Hu96/3D8-Hu97 CDR-L2 | 107 | Humanized antibody 3D8-HuC24/3D8-HuC44/3D8-HuC54 /3D8-HuC84/3D8-HuC94/38F8-Hul4/38F8-Hu24/38F8-Hu34/38F8-Hu54/38F8-Hu64 light chain variable region |
| 26 | Nucleotide sequence of human κ light chain constant region | 108 | Humanized antibody 3D8-Hu25/3D8-Hu45/3D8-Hu55/3D8-Hu85/3D8-Hu95 light chain variable region |
| 27 | Kabat 3D8.2.3/3D8-HuC24/ 3D8-Hu25/ 3D8-Hu26/ 3D8-Hu27/ 3D8-HuC44/3D8-Hu45/ 3D8-Hu46/3D8-Hu47/3D8-HuC54/3D8-Hu55/3D8-Hu56/3D8-Hu57/3D8-HuC84/3D8-Hu85/3D8-Hu86/3D8-Hu87/3D8-HuC94/3D8-Hu95/3D8-Hu96/3D8-Hu97 CDR-H1 | 109 | Humanized antibody 3D8-Hu26/3D8-Hu46/3D8-Hu56/3D8-Hu86/3D8-Hu96 light chain variable region |
| 28 | Kabat 3D8.2.3/3D8-HuC24/ 3D8-Hu25/ 3D8-Hu26/ 3D8-Hu27/ 3D8-HuC44/3D8-Hu45/ 3D8-Hu46/3D8-Hu47/3D8-HuC54/3D8-Hu55/3D8-Hu56/3D8-Hu57 CDR-H2 | 110 | Humanized antibody 3D8-Hu27/3D8-Hu47/3D8-Hu57/3D8-Hu87/3D8-Hu97 light chain variable region |
| 29 | Kabat 3D8-HuC84/3D8-Hu85/3D8-Hu86/3D8-Hu87 CDR-H2 | 111 | Humanized antibody 38F8-Hul4/38F8-Hul6/38F8-Hul7 /38F8-Hu18 heavy chain variable region |
| 30 | Kabat 3D8-HuC94/3D8-Hu95/3D8-Hu96/3D8-Hu97 CDR-H2 | 112 | Humanized antibody 38F8-Hu24/38F8-Hu26/38F8-Hu27 /38F8-Hu28 heavy chain variable region |
| 31 | Chothia 3D8.2.3/3D8-HuC24/ 3D8-Hu25/ 3D8-Hu26/ 3D8-Hu27/ 3D8-HuC44/3D8-Hu45/ 3D8-Hu46/3D8-Hu47/3D8-HuC54/3D8-Hu55/3D8-Hu56/3D8-Hu57/3D8-HuC84/3D8-Hu85/3D8-Hu86/3D8-Hu87/3D8-HuC94/3D8-Hu95/3D8-Hu96/3D8-Hu97 CDR-H1 | 113 | Humanized antibody 38F8-Hu34/38F8-Hu36/38F8-Hu37 /38F8-Hu38 heavy chain variable region |
| 32 | Chothia 3D8.2.3/3D8-HuC24/ 3D8-Hu25/ 3D8-Hu26/ 3D8-Hu27/ 3D8-HuC44/3D8-Hu45/ 3D8-Hu46/3D8-Hu47/3D8-HuC54/3D8-Hu55/3D8-Hu56/3D8-Hu57 CDR-H2 | 114 | Humanized antibody 38F8-Hu54/38F8-Hu56/38F8-Hu57 /38F8-Hu58 heavy chain variable region |
| 33 | Chothia 3D8-HuC84/3D8-Hu85/3D8-Hu86/3D8-Hu87 CDR-H2 | 115 | Humanized antibody 38F8-Hu64/38F8-Hu66/38F8-Hu67 /38F8-Hu68 heavy chain variable region |
| 34 | Chothia 3D8-HuC94/3D8-Hu95/3D8-Hu96/3D8-Hu97 CDR-H2 | 116 | Humanized antibody 38F8-Hul6/38F8-Hu26/38F8-Hu36 /38F8-Hu56/38F8-Hu66 light chain variable region |
| 35 | IMGT 19F6.2.3/19F6-Hull/ 19F6-Hu12/19F6-Hu13/19F6-Hu14/19F6-Hu 15/ 19F6-Hu21 / 19F6-Hu22/ 19F6-Hu23/19F6-Hu24/19F6-Hu25/19F6-Hu31/19F6-Hu32/19F6-Hu33/19F6-Hu34/19F6-Hu35/19F6-Hu35V1/19F6-Hu35V2/19F6-Hu35V3/19F6-Hu35V4/19F6-Hu35V5/19F6-Hu35 V6/19F6-Hu3 5V7/19F6-Hu3 5 V8/19F6 -Hu3 5 V9/19F6-Hu3 5 V10 CDR-H1 | 117 | Humanized antibody 38F8-Hu17/38F8-Hu27/38F8-Hu37 /38F8-Hu57/38F8-Hu67 light chain variable region |
| 36 | IMGT 19F6.2.3/19F6-Hull/ 19F6-Hu12/19F6-Hu13/19F6-Hu14/19F6-Hu 15/ 19F6-Hu21 / 19F6-Hu22/ 19F6-Hu23/19F6-Hu24/19F6-Hu25/19F6-Hu31/19F6-Hu32/19F6-Hu33/19F6-Hu34/19F6-Hu35/19F6-Hu35V1/19F6-Hu35V2/19F6-Hu35V3/19F6-Hu35V4/19F6-Hu35V5/19F6-Hu35 V6/19F6-Hu3 5V7/19F6-Hu3 5 V8/19F6 -Hu3 5 V9/19F6-Hu3 5 V10 CDR-H2 | 118 | Humanized antibody 38F8-Hul8/38F8-Hu28/38F8-Hu38/38F8-Hu58/38F8-Hu68 light chain variable region |
| 37 | IMGT 19F6.2.3/19F6-Hull/ 19F6-Hu12/19F6-Hu13/19F6-Hu14/19F6-Hu 15/ 19F6-Hu21 / 19F6-Hu22/ 19F6-Hu23/19F6-Hu24/19F6-Hu25/19F6-Hu31/19F6-Hu32/19F6-Hu33/19F6-Hu34/19F6-Hu35/19F6-Hu35V1/19F6-Hu35V2/19F6-Hu35V3/19F6-Hu35V4/19F6-Hu35V5/19F6-Hu35 V6/19F6-Hu3 5V7/19F6-Hu3 5 V8/19F6 -Hu3 5 V9/19F6-Hu3 5 V10 CDR-H3 | 119 | Human IgG1 heavy chain constant region |
| 38 | IMGT 19F6.2.3/19F6-Hull/ 19F6-Hu12/19F6-Hu13/19F6-Hu14/19F6- | 120 | Human κ light chain constant region |
| | Hu 15/ 19F6-Hu21 / 19F6-Hu22/ 19F6-Hu23/19F6-Hu24/19F6-Hu25/19F6-Hu31/19F6-Hu32/19F6-Hu33/19F6-Hu34/19F6-Hu35/19F6-Hu35V5 CDR-L1 | | |
| 39 | IMGT 19F6-Hu35V1/19F6-Hu35V3/19F6-Hu35V7/19F6-Hu35V9 CDR-L1 | 121 | Murine antibody 3D8.2.3 heavy chain variable region nucleotide sequence |
| 40 | IMGT 19F6-Hu35V2/19F6-Hu35V10 CDR-L1 | 122 | Murine antibody 3D8.2.3 light chain variable region nucleotide sequence |
| 41 | IMGT 19F6-Hu35V4 CDR-L1 | 123 | Murine antibody 19F6.2.3 heavy chain variable region nucleotide sequence |
| 42 | IMGT 19F6-Hu35V6/19F6-Hu35V8 CDR-L1 | 124 | Murine antibody 19F6.2.3 light chain variable region nucleotide sequence |
| 43 | IMGT 19F6.2.3/19F6-Hull/ 19F6-Hu12/19F6-Hu13/19F6-Hu14/19F6-Hu 15/ 19F6-Hu21 / 19F6-Hu22/ 19F6-Hu23/19F6-Hu24/19F6-Hu25/19F6-Hu31/19F6-Hu32/19F6-Hu33/19F6-Hu34/19F6-Hu35/19F6-Hu35V1/19F6-Hu35V2/19F6-Hu35V3/19F6-Hu35V4/19F6-Hu35V5/19F6-Hu35 V6/19F6-Hu3 5V7/19F6-Hu3 5 V8/19F6 -Hu3 5 V9/19F6-Hu3 5 V10 CDR-L2 | 125 | Murine antibody 38F8.1.1 heavy chain variable region nucleotide sequence |
| 44 | IMGT/Abm/Kabat/Chothia 19F6.2.3/19F6-Hu11/ 19F6-Hu12/19F6-Hul3/19F6-Hul4/19F6-Hul5/19F6-Hu21/19F6-Hu22/19F6-Hu23/19F6-Hu24/19F6-Hu25/19F6-Hu31/19F6-Hu32/19F6-Hu33/19F6-Hu34/19F6-Hu35/19F6-Hu35V1/19F6-Hu35V2/19F6-Hu35V3/19F6-Hu35V4/19F6-Hu35V5/19F6-Hu35 V6/19F6-Hu3 5V7/19F6-Hu3 5 V8/19F6 -Hu3 5 V9/19F6-Hu3 5 V10 CDR-L3 | 126 | Murine antibody 38F8.1.1 light chain variable region nucleotide sequence |
| 45 | AbM 19F6.2.3/19F6-Hull/ 19F6-Hu12/19F6-Hu13/19F6-Hu14/19F6-Hu 15/ 19F6-Hu21 / 19F6-Hu22/ 19F6-Hu23/19F6-Hu24/19F6-Hu25/19F6-Hu31/19F6-Hu32/19F6-Hu33/19F6-Hu34/19F6-Hu35/19F6-Hu35V1/19F6-Hu35V2/19F6-Hu35V3/19F6-Hu35V4/19F6-Hu35V5/19F6-Hu35 V6/19F6-Hu3 5V7/19F6-Hu3 5 V8/19F6 -Hu3 5 V9/19F6-Hu3 5 V10 CDR-H1 | 127 | Humanized antibody 19F6-Hu11/19F6-Hu12/19F6-Hu13/19F6-Hu14/19F6-Hu15 heavy chain variable region nucleotide sequence |
| 46 | AbM 19F6.2.3/19F6-Hull/ 19F6-Hu12/19F6-Hu13/19F6-Hu14/19F6-Hu 15/ 19F6-Hu21 / 19F6-Hu22/ 19F6-Hu23/19F6-Hu24/19F6-Hu25/19F6-Hu31/19F6-Hu32/19F6-Hu33/19F6-Hu34/19F6-Hu35/19F6-Hu35V1/19F6-Hu35V2/19F6-Hu35V3/19F6-Hu35V4/19F6-Hu35V5/19F6-Hu35 V6/19F6-Hu3 5V7/19F6-Hu3 5 V8/19F6 -Hu3 5 V9/19F6-Hu3 5 V10 CDR-H2 | 128 | Humanized antibody 19F6-Hu21/19F6-Hu22/19F6-Hu23/19F6-Hu24/19F6-Hu25 heavy chain variable region nucleotide sequence |
| 47 | AbM/Kabat/Chothia 19F6.2.3/19F6-Hu11/19F6-Hu12/19F6-Hu13/19F6-Hu14/19F6-Hu15/19F6-Hu21/19F6-Hu22/19F6-Hu23/19F6-Hu24/19F6-Hu25/19F6-Hu31/19F6-Hu32/19F6-Hu33/19F6-Hu34/19F6-Hu35/19F6-Hu35 V1/19F6-Hu3 5V2/19F6-Hu35 V3/19F6-Hu3 5V4/19F6-Hu35V5/19F6-Hu35V6/19F6-Hu35 V7/19F6-Hu3 5V8/19F6-Hu35V9/19F6-Hu35V10 CDR-H3 | 129 | Humanized antibody 19F6-Hu31/19F6-Hu32/19F6-Hu33/19F6-Hu34/19F6-Hu35/19F6-Hu35V1/19F6-Hu35V2/19F6-Hu35 V3/19F6-Hu3 5V4/19F6-Hu35V5/19F6-Hu35V6/19F6-Hu35 V7/19F6-Hu3 5V8/19F6-Hu35V9/19F6-Hu35V10 heavy chain variable region nucleotide sequence |
| 48 | AbM/Kabat/Chothia 19F6.2.3/19F6-Hu11/19F6-Hu12/19F6-Hu21/19F6-Hu22/19F6-Hu31/19F6-Hu32 CDR-L1 | 130 | Humanized antibody 19F6-Hu11/19F6-Hu21/19F6-Hu31 light chain variable region nucleotide sequence |
| 49 | AbM/Kabat/Chothia 19F6-Hu13/19F6-Hu23/19F6-Hu33 CDR-L1 | 131 | Humanized antibody 19F6-Hu12/19F6-Hu22/19F6-Hu32 light chain variable region nucleotide sequence |
| 50 | AbM/Kabat/Chothia 19F6-Hu14/19F6-Hu24/19F6-Hu34 CDR-L1 | 132 | Humanized antibody 19F6-Hu13/19F6-Hu23/19F6-Hu33 light chain variable region nucleotide sequence |
| 51 | AbM/Kabat/Chothia 19F6-Hu15/19F6-Hu25/19F6-Hu35/19F6-Hu35V5 CDR-L1 | 133 | Humanized antibody 19F6-Hu14/19F6-Hu24/19F6-Hu34 light chain variable region nucleotide sequence |
| 52 | AbM/Kabat/Chothia 19F6-Hu35V1/19F6-Hu35V3/19F6-Hu35V7/19F6-Hu35V9 CDR-L1 | 134 | Humanized antibody 19F6-Hu15/19F6-Hu25/19F6-Hu35 light chain variable region nucleotide sequence |
| 53 | AbM/Kabat/Chothia 19F6-Hu35V2/19F6-Hu35V10 CDR-L1 | 135 | Humanized antibody 19F6-Hu35V1 light chain variable region nucleotide sequence |
| 54 | AbM/Kabat/Chothia 19F6-Hu35V4 CDR-L1 | 136 | Humanized antibody 19F6-Hu35V2 light chain variable region nucleotide sequence |
| 55 | AbM/Kabat/Chothia 19F6-Hu35V6/19F6-Hu35V8 CDR-L1 | 137 | Humanized antibody 19F6-Hu35V3 light chain variable region nucleotide sequence |
| 56 | AbM/Kabat/Chothia 19F6.2.3/19F6-Hull/19F6-Hul5/19F6-Hu21/19F6-Hu25/19F6-Hu31/19F6-Hu35/19F6-Hu35 V1/19F6-Hu3 5V2/19F6-Hu35 V3/19F6-Hu3 5V4/19F6-Hu35V5/19F6-Hu35V6/19F6-Hu35 V7/19F6-Hu3 5V8/19F6-Hu35V9/19F6-Hu35V10 CDR-L2 | 138 | Humanized antibody 19F6-Hu35V4 light chain variable region nucleotide sequence |
| 57 | AbM/Kabat/Chothia 19F6-Hu12/19F6-Hu13/19F6-Hu22/19F6-Hu23/19F6-Hu32/19F6-Hu33 CDR-L2 | 139 | Humanized antibody 19F6-Hu35V5 light chain variable region nucleotide sequence |
| 58 | AbM/Kabat/Chothia 19F6-Hu14/19F6-Hu24/19F6-Hu34 CDR-L2 | 140 | Humanized antibody 19F6-Hu35V6 light chain variable region nucleotide sequence |
| 59 | Human IgG1 heavy chain constant region nucleotide sequence | 141 | Humanized antibody 19F6-Hu35V7 light chain variable region nucleotide sequence |
| 60 | Kabat 19F6.2.3/19F6-Hul 1/ 19F6-Hu12/19F6-Hu13/19F6-Hu14/19F6-Hu 15/ 19F6-Hu21 / 19F6-Hu22/ 19F6- | 142 | Humanized antibody 19F6-Hu35V8 light chain variable region nucleotide sequence |
| | Hu23/19F6-Hu24/19F6-Hu25/19F6-Hu31/19F6-Hu32/19F6-Hu33/19F6-Hu34/19F6-Hu35/19F6-Hu35V1/19F6-Hu35V2/19F6-Hu35V3/19F6-Hu35V4/19F6-Hu35V5/19F6-Hu35 V6/19F6-Hu3 5V7/19F6-Hu3 5 V8/19F6 -Hu3 5 V9/19F6-Hu3 5 V10 CDR-H1 | | |
| 61 | Kabat 19F6.2.3/19F6-Hu31/19F6-Hu32/19F6-Hu33/19F6-Hu34/19F6-Hu35/19F6-Hu35V1/19F6-Hu35V2/19F6-Hu35V3/19F6-Hu35V4/19F6-Hu35V5/19F6-Hu35 V6/19F6-Hu3 5V7/19F6-Hu3 5 V8/19F6 -Hu3 5 V9/19F6-Hu3 5 V10 CDR-H2 | 143 | Humanized antibody 19F6-Hu35V9 light chain variable region nucleotide sequence |
| 62 | Kabat 19F6-Hu11/19F6-Hu12/19F6-Hu13/19F6-Hu14/19F6-Hu15 CDR-H2 | 144 | Humanized antibody 19F6-Hu35V10 light chain variable region nucleotide sequence |
| 63 | Kabat 19F6-Hu21/19F6-Hu22/19F6-Hu23/19F6-Hu24/19F6-Hu25 CDR-H2 | 145 | Humanized antibody 3D8-HuC24/3D8-Hu25/3D8-Hu26 /3D8-Hu27 heavy chain variable region nucleotide sequence |
| 64 | Chothia 19F6.2.3/19F6-Hull/ 19F6-Hu12/19F6-Hu13/19F6-Hu14/19F6-Hu 15/ 19F6-Hu21 / 19F6-Hu22/ 19F6-Hu23/19F6-Hu24/19F6-Hu25/19F6-Hu31/19F6-Hu32/19F6-Hu33/19F6-Hu34/19F6-Hu35/19F6-Hu35V1/19F6-Hu35V2/19F6-Hu35V3/19F6-Hu35V4/19F6-Hu35V5/19F6-Hu35 V6/19F6-Hu3 5V7/19F6-Hu3 5 V8/19F6 -Hu3 5 V9/19F6-Hu3 5 V10 CDR-H1 | 146 | Humanized antibody 3D8-HuC44/3D8-Hu45/3D8-Hu46 /3D8-Hu47 heavy chain variable region nucleotide sequence |
| 65 | Chothia 19F6.2.3/19F6-Hull/ 19F6-Hu12/19F6-Hu13/19F6-Hu14/19F6-Hu 15/ 19F6-Hu21 / 19F6-Hu22/ 19F6-Hu23/19F6-Hu24/19F6-Hu25/19F6-Hu31/19F6-Hu32/19F6-Hu33/19F6-Hu34/19F6-Hu35/19F6-Hu35V1/19F6-Hu35V2/19F6-Hu35V3/19F6-Hu35V4/19F6-Hu35V5/19F6-Hu35 V6/19F6-Hu3 5V7/19F6-Hu3 5 V8/19F6 -Hu3 5 V9/19F6-Hu3 5 V10 CDR-H2 | 147 | Humanized antibody 3D8-HuC 54/3D8-Hu55/3D8-Hu56 /3D8-Hu57 heavy chain variable region nucleotide sequence |
| 66 | IMGT 38F8.1.1/38F8-Hul4/ 38F8-Hu16/ 38F8-Hu17/ 38F8-Hu18/ 38F8-Hu24/ 38F8-Hu26/ 38F8-Hu27/ 38F8-Hu28/ 38F8-Hu34/ 38F8-Hu36/ 38F8-Hu37/ 38F8-Hu38/ 38F8-Hu54/ 38F8-Hu56/ 38F8-Hu57/ 38F8-Hu58/ 38F8-Hu64/ 38F8-Hu66/ 38F8-Hu67/ 38F8-Hu68 CDR-H1 | 148 | Humanized antibody 3D8-HuC84/3D8-Hu85/3D8-Hu86 /3D8-Hu87 heavy chain variable region nucleotide sequence |
| 67 | IMGT 38F8.1.1/38F8-Hul4/ 38F8-Hu16/ 38F8-Hu17/ 38F8-Hu18/ 38F8-Hu24/ 38F8-Hu26/ 38F8-Hu27/ 38F8-Hu28/ 38F8-Hu34/ 38F8-Hu36/ 38F8-Hu37/ 38F8-Hu38/ 38F8-Hu54/ 38F8-Hu56/ 38F8-Hu57/ 38F8-Hu58/ 38F8-Hu64/ 38F8-Hu66/ 38F8-Hu67/ 38F8-Hu68 CDR-H2 | 149 | Humanized antibody 3D8-HuC94/3D8-Hu95/3D8-Hu96 /3D8-Hu97 heavy chain variable region nucleotide sequence |
| 68 | IMGT 38F8.1.1/38F8-Hu14/ 38F8-Hu16/ 38F8-Hu17/ 38F8-Hu18/ 38F8-Hu24/ 38F8-Hu26/ 38F8-Hu27/ 38F8-Hu28/ 38F8-Hu34/ 38F8-Hu36/ 38F8-Hu37/ 38F8-Hu38/ 38F8-Hu54/ 38F8-Hu56/ 38F8-Hu57/ 38F8-Hu58/ 38F8-Hu64/ 38F8-Hu66/ 38F8-Hu67/ 38F8-Hu68 CDR-H3 | 150 | Humanized antibody 3D8-HuC24/3D8-HuC44/3D8-HuC54 /3D8-HuC84/3D8-HuC94/38F8-Hul4/38F8-Hu24/38F8-Hu34/38F8-Hu54/38F8-Hu64 light chain variable region nucleotide sequence |
| 69 | IMGT 38F8.1.1/38F8-Hu16/38F8-Hul8/38F8-Hu26/38F8-Hu28/38F8-Hu36/38F8-Hu38/38F8-Hu56/38F8-Hu58/38F8-Hu66/38F8-Hu68 CDR-L1 | 151 | Humanized antibody 3D8-Hu25/3D8-Hu45/3D8-Hu55/3D8-Hu85/3D8-Hu95 light chain variable region nucleotide sequence |
| 70 | IMGT 38F8-Hul4/38F8-Hu24/ 38F8-Hu34/38F8-Hu54/38F8-Hu64/38F8-Hu17/38F8-Hu27/ 38F8-Hu37/38F8-Hu57/38F8-Hu67 CDR-L1 | 152 | Humanized antibody 3D8-Hu26/3D8-Hu46/3D8-Hu56/3D8-Hu86/3D8-Hu96 light chain variable region nucleotide sequence |
| 71 | IMGT 38F8.1.138F8.1.1/38F8-Hu14/ 38F8-Hu16/ 38F8-Hu17/ 38F8-Hu18/ 38F8-Hu24/ 38F8-Hu26/ 38F8-Hu27/ 38F8-Hu28/ 38F8-Hu34/ 38F8-Hu36/ 38F8-Hu37/ 38F8-Hu38/ 38F8-Hu54/ 38F8-Hu56/ 38F8-Hu57/ 38F8-Hu58/ 38F8-Hu64/ 38F8-Hu66/ 38F8-Hu67/ 38F8-Hu68 CDR-L2 | 153 | Humanized antibody 3D8-Hu27/3D8-Hu47/3D8-Hu57/3D8-Hu87/3D8-Hu97 light chain variable region nucleotide sequence |
| 72 | AbM 38F8.1.1/38F8-Hul4/ 38F8-Hu16/ 38F8-Hu17/ 38F8-Hu18/ 38F8-Hu24/ 38F8-Hu26/ 38F8-Hu27/ 38F8-Hu28/ 38F8-Hu34/ 38F8-Hu36/ 38F8-Hu37/ 38F8-Hu38/ 38F8-Hu54/ 38F8-Hu56/ 38F8-Hu57/ 38F8-Hu58/ 38F8-Hu64/ 38F8-Hu66/ 38F8-Hu67/ 38F8-Hu68 CDR-H1 | 154 | Humanized antibody 38F8-Hul4/38F8-Hul6/38F8-Hul7 /38F8-Hu18 heavy chain variable region nucleotide sequence |
| 73 | AbM 38F8.1.1/38F8-Hu14/ 38F8-Hul6/ 38F8-Hu17/ 38F8-Hu18/ 38F8-Hu24/ 38F8-Hu26/ 38F8-Hu27/ 38F8-Hu28/ 38F8-Hu34/ 38F8-Hu36/ 38F8-Hu37/ 38F8-Hu38/ 38F8-Hu54/ 38F8-Hu56/ 38F8-Hu57/ 38F8-Hu58/ 38F8-Hu64/ 38F8-Hu66/ 38F8-Hu67/ 38F8-Hu68 CDR-H2 | 155 | Humanized antibody 38F8-Hu24/38F8-Hu26/38F8-Hu27 /38F8-Hu28 heavy chain variable region nucleotide sequence |
| 74 | AbM/Kabat/Chothia 38F8.1.1/38F8-Hul4/ 38F8-Hul6/ 38F8-Hul7/ 38F8-Hu18/ 38F8-Hu24/ 38F8-Hu26/ 38F8-Hu27/ 38F8-Hu28/ 38F8-Hu34/ 38F8-Hu36/ 38F8-Hu37/ 38F8-Hu38/ 38F8-Hu54/ 38F8-Hu56/ 38F8-Hu57/ 38F8-Hu58/ 38F8-Hu64/ 38F8-Hu66/ 38F8-Hu67/ 38F8-Hu68 CDR-H3 | 156 | Humanized antibody 38F8-Hu34/38F8-Hu36/38F8-Hu37 /38F8-Hu38 heavy chain variable region nucleotide sequence |
| 75 | AbM/Kabat/Chothia 38F8.1.1 CDR-L1 | 157 | Humanized antibody 38F8-Hu54/38F8-Hu56/38F8-Hu57 /38F8-Hu58 heavy chain variable region nucleotide sequence |
| 76 | AbM/Kabat/Chothia 38F8-Hu16/38F8-Hul8/38F8-Hu26/38F8-Hu28/38F8-Hu36/38F8-Hu38/38F8-Hu56/38F8-Hu58/38F8-Hu66/38F8-Hu68 CDR-L1 | 158 | Humanized antibody 38F8-Hu64/38F8-Hu66/38F8-Hu67 /38F8-Hu68 heavy chain variable region nucleotide sequence |
| 77 | AbM/Kabat/Chothia 3D8-HuC24/ 3D8-HuC44 /3D8-HuC54/3D8-HuC84/3D8-HuC94/38F8-Hul4/38F8-Hu24/ 38F8-Hu34 /38F8-Hu54/38F8-Hu64/38F8- | 159 | Humanized antibody 38F8-Hul6/38F8-Hu26/38F8-Hu36 /38F8-Hu56/38F8-Hu66 light chain variable region nucleotide sequence |
| | Hul7/38F8-Hu27/38F8-Hu37/38F8-Hu57/38F8-Hu67 CDR-L1 | | |
| 78 | AbM/Kabat/Chothia 38F8-Hul4/38F8-Hu24/ 38F8-Hu34 /38F8-Hu54/38F8-Hu64/38F8-Hul6/ 38F8-Hul7/ 38F8-Hul8/ 38F8-Hu26/ 38F8-Hu27/ 38F8-Hu28/38F8-Hu36/ 38F8-Hu37/ 38F8-Hu38/38F8-Hu56/ 38F8-Hu57/ 38F8-Hu58/ 38F8-Hu66/ 38F8-Hu67/ 38F8-Hu68 CDR-L2 | 160 | Humanized antibody 38F8-Hu17/38F8-Hu27/38F8-Hu37 /38F8-Hu57/38F8-Hu67 light chain variable region nucleotide sequence |
| 79 | Kabat 38F8.1.1/38F8-Hu14/ 38F8-Hu16/ 38F8-Hu17/ 38F8-Hu18/ 38F8-Hu24/ 38F8-Hu26/ 38F8-Hu27/ 38F8-Hu28/ 38F8-Hu34/ 38F8-Hu36/ 38F8-Hu37/ 38F8-Hu38/ 38F8-Hu54/ 38F8-Hu56/ 38F8-Hu57/ 38F8-Hu58/ 38F8-Hu64/ 38F8-Hu66/ 38F8-Hu67/ 38F8-Hu68 CDR-H1 | 161 | Humanized antibody 38F8-Hul8/38F8-Hu28/38F8-Hu38/38F8-Hu58/38F8-Hu68 light chain variable region nucleotide sequence |
| 80 | Kabat 38F8.1.1/38F8-Hu24/ 38F8-Hu26/ 38F8-Hu27/ 38F8-Hu28/38F8-Hu54/ 38F8-Hu56/ 38F8-Hu57/38F8-Hu58/38F8-Hu64/ 38F8-Hu66/ 38F8-Hu67/38F8-Hu68 CDR-H2 | | |
| 81 | Kabat 38F8-Hu14/ 38F8-Hu16/ 38F8-Hu17/ 38F8-Hu18/38F8-Hu34/ 38F8-Hu36/ 38F8-Hu37/38F8-Hu38 CDR-H2 | | |
| 82 | Chothia 38F8.1.1/38F8-Hu14/ 38F8-Hu16/ 38F8-Hu17/ 38F8-Hu18/ 38F8-Hu24/ 38F8-Hu26/ 38F8-Hu27/ 38F8-Hu28/ 38F8-Hu34/ 38F8-Hu36/ 38F8-Hu37/ 38F8-Hu38/ 38F8-Hu54/ 38F8-Hu56/ 38F8-Hu57/ 38F8-Hu58/ 38F8-Hu64/ 38F8-Hu66/ 38F8-Hu67/ 38F8-Hu68 CDR-H1 | | |

### EXAMPLES

The present invention will now be described with reference to the following examples, which are intended to illustrate the present invention, but not to limit it.

Unless otherwise specified, the molecular biology experiment methods and immunoassay methods used in the present invention are performed by basically referring to the methods described in J. Sambrook et al., Molecular Cloning: A Laboratory Manual, 2nd Edition, Cold Spring Harbor Laboratory Press, 1989, and F. M. Ausubel et al., Short Protocols in Molecular Biology, 3rd Edition, John Wiley & Sons, Inc., 1995. Those skilled in the art understand that the examples describe the present invention by way of example and are not intended to limit the scope of the present invention.

### Example 1: Production of mouse-anti-human ROR1 antibody

### 1) Production of antibodies and preparation of identification materials

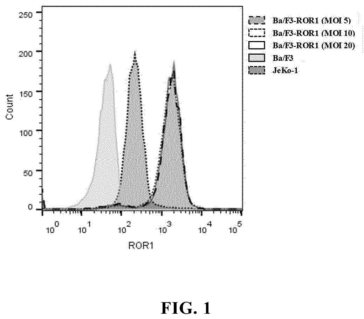

(1) Construction of cell line stably expressing human ROR1: In order to verify the specificity and function of human ROR1 antibody, Ba/F3 cells were infected with lentivirus (G&P Biosciences, Cat. No.: LVT-ROR1) containing full-length sequence of human ROR1 (gene number: Q01973), positively transduced cells were selected by puromycin, and a monoclonal BA/F3-ROR1 stable cell line was obtained by limiting dilution method for monoclonal screening. The expression of ROR1 was identified by flow cytometry (Luminex, Guava easyCyte HT). Anti-human ROR1 antibody D10 (for the sequence, see US Patent 9217040B2) was used as the detection antibody. As shown in FIG. 1, the results of flow cytometry showed that the positive rate of Ba/F3-RORl was high (nearly 100%), which could be used for subsequent experiments.
(2) Recombinant human ROR ECD-his, ECD-mouse Fc, and ECD-T cell epitope fusion proteins (ECD: extracellular domain aa30-406) were expressed in Expi293 or ExpiCHO mammalian cells and purified, and the purified proteins were identified via ELISA using the antibody D10. A pcDNA3.4 plasmid carrying the full-length human ROR1 coding sequence was constructed by gene synthesis (Genscript, Piscataway, NJ) and amplified.

### 2) Immunization of mice

The human ROR1 ECD proteins with different tags, the pcDNA3.4 plasmid and Ba/F3-ROR1 cell encoding the full-length ROR1 sequence as above-mentioned were used to immunize wild-type mice. Four mouse strains were used for immunization: Balb/c, C57B1/6, NZB and A/J. Three immunization schemes were adopted, outlined as follows: 1) firstly, 100µg of the pcDNA3.4 plasmid containing full-length sequence of human ROR1 was injected into the tail vein for 1-2 times, and then the immunization was boosted with 2-4×10⁶ Ba/F3-ROR1 cells, interval between each immunization was 2-3 weeks; 2) firstly, 100µg of the pcDNA3.4 plasmid containing full-length sequence of human ROR1 was injected into the tail vein for 1-2 times, and the immunization was boosted with 50µ to 100µg of recombinant ROR1 ECD protein mixed with incomplete Freund's adjuvant (IFA), interval between each immunization was 2-3 weeks; 3) firstly, immunization was carried out with 50 µg of recombinant ROR1 ECD protein mixed with complete Freund's adjuvant (CFA), and then the immunization was boosted with 50 µg of recombinant ROR1 ECD protein mixed with IFA, and interval between each immunization was 2-3 weeks. After the second booster immunization, the serum titer was detected by flow cytometry using Ba/F3-ROR1 cells. Mice with high titers were selected for booster immunization with ROR-1 ECD protein 3-5 days before fusion.

### 3) Preparation and screening of murine hybridoma producing anti-human ROR1 antibody

Mouse splenocytes and mouse myeloma SP2/0 cells (ATCC, Cat. No.: CRL-1581) were fused together by standard chemical fusion procedures using polyethylene glycol (molecular weight: 1500 Da, Roche, Cat. No.: 10 783 641 001) with a ratio of 5: 1. The cells were adjusted to 5×10⁵ splenocytes/mL with DMEM medium (Gibco, Cat. No.: 12430-47) containing 20% fetal bovine serum (Hyclone, Cat. No.: SH30080.03). 0.2 mL of the cells were added to each well of a 96-well plate, and cultured in an incubator at 37°C with 5% CO₂. HAT (Sigma, Cat. No.: H0262-10VL) was added to each well the day after the fusion for screening. Clones producing antibodies binding to Ba/F3-ROR1 cells were selected by a high-throughput flow cytometer (Sartorius, model: iQue Screener Plus). Positive clones that only bound to Ba/F3-ROR1 cells but not Ba/F3 wild-type cells were further selected by a flow cytometry (Luminex, model: Guava easyCyte HT). Finally, monoclonal hybridomas were obtained by limiting dilution, and their binding to ROR1 cells was confirmed by FACS. Small-scale antibody purification was performed for each of the selected positive monoclonal hybridomas. Hybridoma cells were cultured with 50-100 mL of serum-free medium (Thermo Fisher, Hybridoma-SFM, Cat. No.: 12045084), and the antibodies in the supernatant were purified and eluted by protein A (GE Healthcare, MabSelect SuRe, Cat. No.: 17543802), and the eluted monoclonal antibodies were dialyzed against 150 mM NaCl. The dialyzed antibodies were filtered and sterilized through a 0.2 µm filter. The binding of the purified antibodies to Ba/F3-ROR1 cells was detected by flow cytometry, and 3 mouse-anti-human ROR1 monoclonal antibodies (3D8.2.3, 19F6.2.3, 38F8.1.1) were selected as candidate molecules for further characterization.

### Example 2: Detection of affinity of mouse-anti-human ROR1 monoclonal antibody

The affinity of the candidate antibodies to ROR1 on the surface of human cells was detected by flow cytometry. In addition to the Ba/F3-ROR1 overexpression cells, human mantle cell lymphoma cell line Jeko-1 (ATCC, Cat. No.: CRL-3006), human colon cancer cell line HT-29 (ATCC, Cat. No.: HTB-38), human lung cancer cell line A549 (ATCC, Cat. No.: CCL-185) were also used for the binding assay. The anti-human ROR1 antibody UC961 (for sequence, see WO2018237335A1) with mouse or human IgG1 Fc was used as a positive control, and the anti-2,4,6-trinitrophenol antibody (ATCC) with the corresponding mouse or human IgG1 Fc was used as a negative control. Mouse IgG isotype control (Thermo Fisher, Cat. No.: 31903) was also used as a negative control. The Ba/F3-ROR1, Jeko-1, HT-29 and A549 cells were incubated with different concentrations of purified murine antibodies and control antibodies on ice for 30 minutes, then washed with flow cytometry buffer (PBS+2% FBS) twice, and then incubated with goat-anti-human or mouse fluorescent secondary antibody (Jackson ImmunoResearch, Cat. No.: 109-605-098 or 115-605-071) on ice for 30 minutes, and finally detected on the machine (Luminex, model: Guava easyCyte HT).

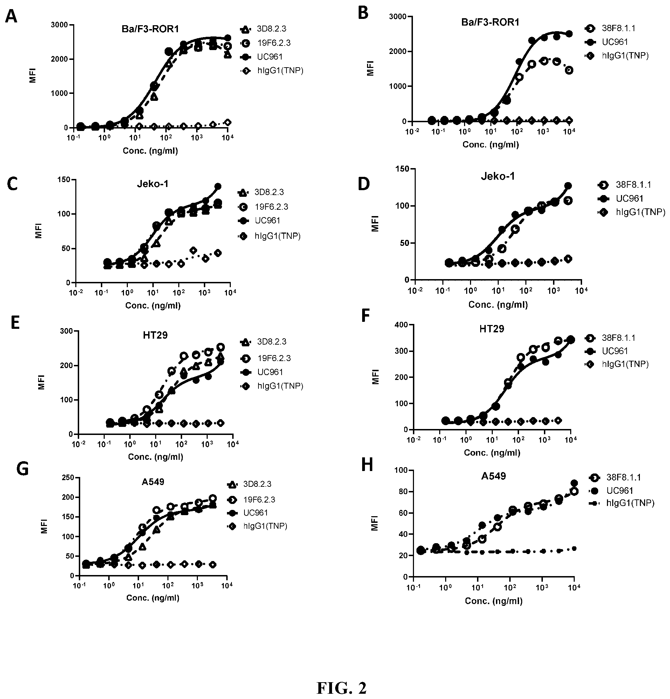

The test results were shown in FIG. 2. All three anti-human ROR1 mouse antibodies could specifically recognize human ROR1 on the surface of Ba/F3-RORl, Jeko-1, HT-29 and A549 cells.

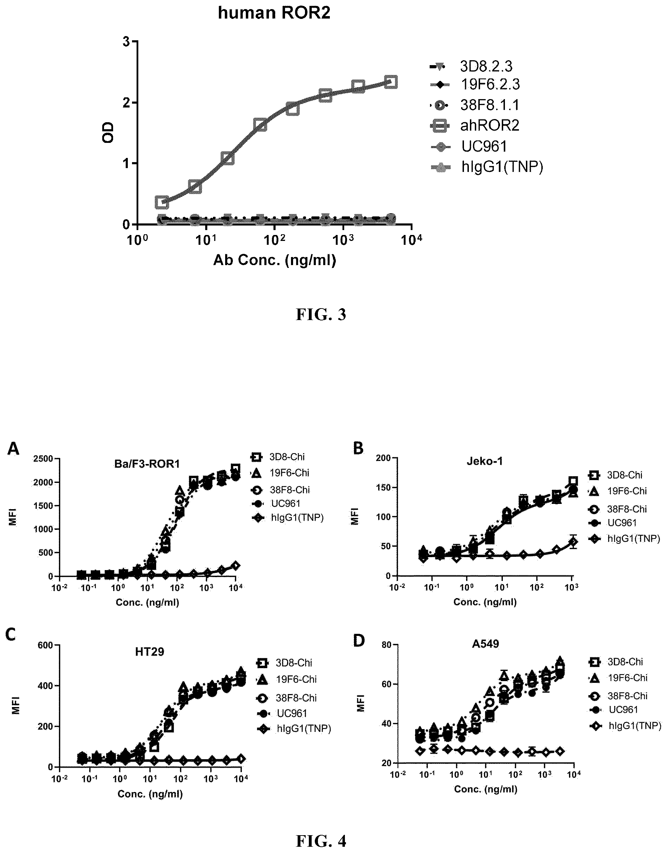

It was confirmed by ELISA using extracellular domain (ECD) protein of human ROR2 that the binding specificity of the candidate antibodies to human ROR1 was higher than that of human ROR2. The specific method was as follows: 1 µg/mL human ROR2 extracellular domain (ECD) protein (Sino Biologicals, Cat. No.: 16133-H08H) was added in a 96-well ELISA plate (Thermo Fisher, Cat. No.: 5129) at 100 µL/well, for coating overnight at 4°C. 200 µL of washing buffer (1×TBS containing 0.05% Tween-20) was used to wash 3 times, different concentrations of eight purified mouse-anti-human ROR1 candidate antibodies and UC961 were added at 100 µL, incubated at 37°C for 1 hour, and mouse-anti-human ROR2 antibody (R&D systems, Cat. No.: MAB2064) was used as a positive control. Washing was performed 3 times with 200 µL of washing buffer, 100 µL of anti-human IgG (Rockland Inc, Cat. No. 609-1304) or anti-mouse IgG secondary antibody (Rockland Inc, Cat. No. 610-1304) diluted at a ratio of 1:10000 were added to each well, incubated at 37°C for 1 hour. Washing was performed 3 times with 200 µL of washing buffer, 100 µL of TMB (Thermo Fisher, Cat. No.: TMBW-1000-01) was added for color development in the dark for 10 minutes, then 100 µL of stop solution (Thermo Fisher, Cat. No.: 13361-100-10) was added to stop the color development, and reading was carried out by microplate reader at 450nm. As shown in FIG. 3, all three antibodies tested did not bind to human ROR2 ECD protein.

### Example 3: Isotype and variable region amplification of anti-human ROR1 murine antibody

The isotypes of all mouse-anti-human ROR1 candidate antibodies (3D8.2.3, 19F6.2.3, 38F8.1.1) were identified using Pierce Rapid Isotyping Kit (purchased from Thermo Fisher, Cat. No.: 26179). The isotyping results showed that the heavy chain of the candidate clones was IgG1, and the light chain was Kappa.

In order to clone the variable region gene, total RNA was extracted from lysed hybridoma cells, and the first-strand cDNA was synthesized using a cDNA reverse transcription kit (purchased from Thermo Fisher, Cat. No.: 18080-200), the VH and VK genes were amplified by PCR using antibody-specific primers and Platinum Taq DNA polymerase (purchased from Thermo Fisher, Cat. No.: 1304011), the PCR products were purified by a DNA purification kit (purchased from Qiagen, Cat. No.: 28104), and ligated to TOPO TA cloning vector (purchased from Thermo Fisher, Cat. No.: K457540) for *E.coli* transformation. For each ligation reaction, about 6-12 *E. coli* clones were picked for sequencing. The sequencing results were analyzed by Vector NTI 11.5 (purchased from Thermo Fisher) and Sequencer 5.4.6 (purchased from Genecodes), and the variable region sequences and CDR sequences of the anti-human ROR1 murine candidate antibodies obtained were shown in the sequence information of Table 1.

**Table 1. Amino acid sequences of variable regions and CDRs of anti-human ROR1 murine antibodies**

| Clone No. | Heavy chain variable region | Light chain variable region | Numbering system | Heavy chain CDR1 | Heavy chain CDR2 | Heavy chain CDR3 | Light chain CDR1 | Light chain CDR2 | Light chain CDR3 |
|---|---|---|---|---|---|---|---|---|---|
| | (SEQ ID NO:) | (SEQ ID NO:) | | (SEQ ID NO:) | (SEQ ID NO:) | (SEQ ID NO:) | (SEQ ID NO:) | (SEQ ID NO:) | (SEQ ID NO:) |
| 3D8.2.3 | 1 | 2 | Chothia | 31 | 32 | 20 | 21 | 24 | 15 |
| | | | AbM | 16 | 17 | 20 | 21 | 24 | 15 |
| | | | Kabat | 27 | 28 | 20 | 21 | 24 | 15 |
| | | | IMGT | 7 | 8 | 11 | 12 | 14 | 15 |
| 19F6.2.3 | 3 | 4 | Chothia | 64 | 65 | 47 | 48 | 56 | 44 |
| | | | AbM | 45 | 46 | 47 | 48 | 56 | 44 |
| | | | Kabat | 60 | 61 | 47 | 48 | 56 | 44 |
| | | | IMGT | 35 | 36 | 37 | 38 | 43 | 44 |
| 38F8.1.1 | 5 | 6 | Chothia | 82 | 83 | 74 | 75 | 24 | 15 |
| | | | AbM | 72 | 73 | 74 | 75 | 24 | 15 |
| | | | Kabat | 79 | 80 | 74 | 75 | 24 | 15 |
| | | | IMGT | 66 | 67 | 68 | 69 | 71 | 15 |

### Example 4: Affinity detection of anti-human ROR1 chimeric antibody

Chimeric antibodies were constructed via engineering by ligating the light chain and heavy chain variable regions of 3D8.2.3, 19F6.2.3 and 38F8.1.1 to human light chain κ constant region (SEQ ID NO: 120) and human IgG1 heavy chain constant region (SEQ ID NO: 119), respectively. The coding DNA sequences of heavy and light chains of each antibody were synthesized and codon-optimized, and then cloned into the pcDNA3.4 plasmid (entrusted to Suzhou Hong Xun Technology Co., Ltd.). The pcDNA3.4 plasmid corresponding to the heavy and light chains of each chimeric antibody was simultaneously transfected into Expi293F cells (Thermo Fisher, Cat. No.: A14527), and the antibodies expressed in supernatant were purified by protein A, and the purified chimeric antibodies were named as 3D8-Chi, 19F6-Chi and 38F8-Chi, respectively.

The affinities of the purified anti-human ROR1 chimeric antibodies binding to the Ba/F3-ROR1, Jeko-1, HT-29 and A549 cells were analyzed by flow cytometry. The specific experimental steps were as follows: the Ba/F3-ROR1, Jeko-1, HT-29 and A549 cells were incubated with the purified chimeric antibodies of different concentration gradients on ice for 30 minutes, and the anti-ROR1 antibody UC961 with human IgG1 and the anti-TNP antibody were used as positive and negative controls respectively. Washing was performed twice with flow cytometry buffer (PBS+2% FBS), then incubation was carried out on ice for 30 minutes with goat-anti-human fluorescent secondary antibody (Jackson ImmunoResearch, Cat. No.: 109-605-098), and then the detection was performed by flow cytometry.

The results were shown in FIG. 4 and Table 2, in which the affinity EC50 of 19F6-Chi on cells was lower than that of the positive control antibody UC961, indicating that the anti-human ROR1 chimeric antibody had stronger affinity than UC961; while the affinity EC50 values of 38F8-Chi and 3D8-Chi were comparable to that of the control antibody UC961.

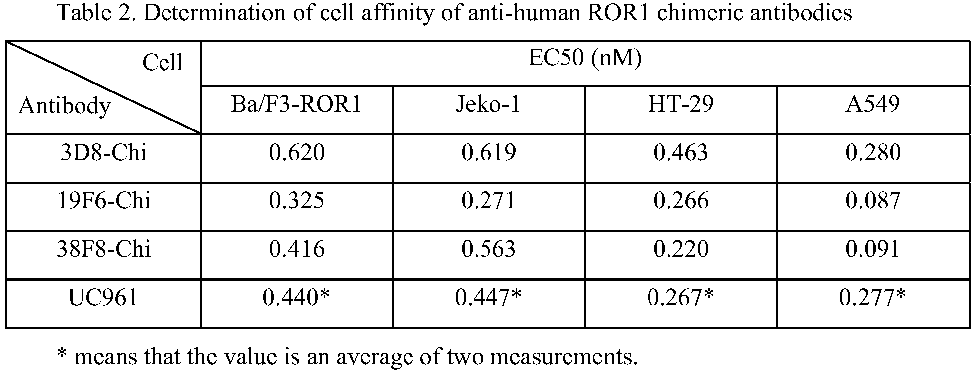

Octet ForteBio is widely used in the detection of antibody-antigen dynamic affinity. This method was used to determine the dynamic affinities of the candidate chimeric antibodies 3D8-Chi, 19F6-Chi and 38F8-Chi and the positive control antibody UC961 to human ROR1. The specific experimental steps were as follows: the antibody to be tested is first bound to anti-human IgG Fc AHC biosensor (ForteBio, Cat. No.: 18-5060) to reach a response signal value of 0.8-1.0nm, and then the antibody-coated biosensor was immersed into wells containing different concentrations (4, 2, 1, 0.5, 0.25, 0.13, 0.06 and 0 µg/mL) of human ROR1 ECD protein to measure dynamic affinity, the binding was performed for 5 minutes, then dissociation was performed for 10 minutes, and 1:1 kinetic binding model was used for all fitting analysis. As shown in Table 3, the dissociation rates of 3D8-Chi, 19F6-Chi and 38F8-Chi were significantly slower than that of the control antibody UC961 (as indicated by Kdis values), and the affinities of 3D8-Chi, 19F6-Chi and 38F8-Chi were stronger than that of the control antibody UC961 by 2.0, 34.6 and 5.0 times (as indicated by KD values), respectively.

**Table 3. Determination of dynamic affinity of anti-human ROR1 chimeric antibody to human ROR1**

| Antibody | KD (M) | kon(1/Ms) | kdis(1/s) |
|---|---|---|---|
| 3D8-Chi | 8.09E-09 | 4.71E+05 | 3.81E-03 |
| 19F6-Chi | 4.56E-10 | 1.13E+06 | 5.14E-04 |
| 38F8-Chi | 3.17E-09 | 9.42E+05 | 2.99E-03 |
| UC961 | 1.58E-08* | 9.81E+5* | 1.37E-02* |

| | | | |
|---|---|---|---|
| * means that the value is an average of two measurements. | | | |

In summary, compared with the control antibody, the antibodies of the present invention showed stronger antigen-binding abilities.

### Example 5: Humanization of anti-human ROR1 murine antibody

The mouse-anti-human ROR1 monoclonal antibodies 3D8.2.3, 19F6.2.3 and 38F8.1.1 were humanized following the CDR grafting method described in the literatures (Winter and Milstein, Nature, 1991, 349: 293-299; Rader et al., Proc. Nat. Acad. Sci. USA, 1998, 95: 8910-8915; Steinberger et al., J. Biol. Chem., 2000, 275: 36073-36078; Queen et al., Proc. Natl. Acad. Sci. USA, 1989, 86: 10029-10033). Briefly, humanized antibodies were produced by replacing most or all of the framework sequences of murine antibody with the corresponding human germline antibody framework sequences, and retaining the antigen-specific binding variable regions or CDRs of the murine antibody. From this, hybrid molecules were generated in which only CDRs consisted of non-human sequences. Usually, some key murine amino acids were transplanted into the human germline antibody framework by back mutation, and in order to maintain maximally the antigen binding affinity of the parental antibody, the potential deamidation, isomerization and glycosylation sites in the CDR were mutated to improve the biophysical properties.

The sequences of the humanized heavy and light chain variants of the above three murine monoclonal antibody clones (3D8.2.3, 19F6.2.3 and 38F8.1.1) were shown in Table 4 below:

**Table 4-1. Amino acid sequences of variable and constant regions of 19F6 anti-human ROR1 humanized antibody**

| Name | Numbering system | Heavy chain CDR1 | Heavy chain CDR2 | Heavy chain CDR3 | Light chain CDR1 | Light chain CDR2 | Light chain CDR3 | VH | VL | CH | CL |
|---|---|---|---|---|---|---|---|---|---|---|---|
| | | (SEQ ID NO:) | (SEQ ID NO:) | (SEQ ID NO:) | (SEQ ID NO:) | (SEQ ID NO:) | (SEQ ID NO:) | (SEQ ID NO:) | (SEQ ID NO:) | (SEQ ID NO:) | (SEQ ID NO:) |
| 19F6-Hu11 | Chothia | 64 | 65 | 47 | 48 | 56 | 44 | 84 | 87 | 119 | 120 |
| | AbM | 45 | 46 | 47 | 48 | 56 | 44 | | | | |
| | Kabat | 60 | 62 | 47 | 48 | 56 | 44 | | | | |
| | IMGT | 35 | 36 | 37 | 38 | 43 | 44 | | | | |
| 19F6-Hu12 | Chothia | 64 | 65 | 47 | 48 | 57 | 44 | 84 | 88 | | |
| | AbM | 45 | 46 | 47 | 48 | 57 | 44 | | | | |
| | Kabat | 60 | 62 | 47 | 48 | 57 | 44 | | | | |
| | IMGT | 35 | 36 | 37 | 38 | 43 | 44 | | | | |
| 19F6-Hul3 | Chothia | 64 | 65 | 47 | 49 | 57 | 44 | 84 | 89 | | |
| | AbM | 45 | 46 | 47 | 49 | 57 | 44 | | | | |
| | Kabat | 60 | 62 | 47 | 49 | 57 | 44 | | | | |
| | IMGT | 35 | 36 | 37 | 38 | 43 | 44 | | | | |
| 19F6-Hul4 | Chothia | 64 | 65 | 47 | 50 | 58 | 44 | 84 | 90 | | |
| | AbM | 45 | 46 | 47 | 50 | 58 | 44 | | | | |
| | Kabat | 60 | 62 | 47 | 50 | 58 | 44 | | | | |
| | IMGT | 35 | 36 | 37 | 38 | 43 | 44 | | | | |
| 19F6-Hul5 | Chothia | 64 | 65 | 47 | 51 | 56 | 44 | 84 | 91 | | |
| | AbM | 45 | 46 | 47 | 51 | 56 | 44 | | | | |
| | Kabat | 60 | 62 | 47 | 51 | 56 | 44 | | | | |
| | IMGT | 35 | 36 | 37 | 38 | 43 | 44 | | | | |
| 19F6-Hu21 | Chothia | 64 | 65 | 47 | 48 | 56 | 44 | 85 | 87 | | |
| | AbM | 45 | 46 | 47 | 48 | 56 | 44 | | | | |
| | Kabat | 60 | 63 | 47 | 48 | 56 | 44 | | | | |
| | IMGT | 35 | 36 | 37 | 38 | 43 | 44 | | | | |
| 19F6-Hu22 | Chothia | 64 | 65 | 47 | 48 | 57 | 44 | 85 | 88 | | |
| | AbM | 45 | 46 | 47 | 48 | 57 | 44 | | | | |
| | Kabat | 60 | 63 | 47 | 48 | 57 | 44 | | | | |
| | IMGT | 35 | 36 | 37 | 38 | 43 | 44 | | | | |
| 19F6-Hu23 | Chothia | 64 | 65 | 47 | 49 | 57 | 44 | 85 | 92 | | |
| | AbM | 45 | 46 | 47 | 49 | 57 | 44 | | | | |
| | Kabat | 60 | 63 | 47 | 49 | 57 | 44 | | | | |
| | IMGT | 35 | 36 | 37 | 38 | 43 | 44 | | | | |
| 19F6-Hu24 | Chothia | 64 | 65 | 47 | 50 | 58 | 44 | 85 | 90 | | |
| | AbM | 45 | 46 | 47 | 50 | 58 | 44 | | | | |
| | Kabat | 60 | 63 | 47 | 50 | 58 | 44 | | | | |
| | IMGT | 35 | 36 | 37 | 38 | 43 | 44 | | | | |
| 19F6-Hu25 | Chothia | 64 | 65 | 47 | 51 | 56 | 44 | 85 | 91 | | |
| | AbM | 45 | 46 | 47 | 51 | 56 | 44 | | | | |
| | Kabat | 60 | 63 | 47 | 51 | 56 | 44 | | | | |
| | IMGT | 35 | 36 | 37 | 38 | 43 | 44 | | | | |
| 19F6-Hu31 | Chothia | 64 | 65 | 47 | 48 | 56 | 44 | 86 | 87 | | |
| | AbM | 45 | 46 | 47 | 48 | 56 | 44 | | | | |
| | Kabat | 60 | 61 | 47 | 48 | 56 | 44 | | | | |
| | IMGT | 35 | 36 | 37 | 38 | 43 | 44 | | | | |
| 19F6-Hu32 | Chothia | 64 | 65 | 47 | 48 | 57 | 44 | 86 | 88 | | |
| | AbM | 45 | 46 | 47 | 48 | 57 | 44 | | | | |
| | Kabat | 60 | 61 | 47 | 48 | 57 | 44 | | | | |
| | IMGT | 35 | 36 | 37 | 38 | 43 | 44 | | | | |
| 19F6-Hu33 | Chothia | 64 | 65 | 47 | 49 | 57 | 44 | 86 | 89 | | |
| | AbM | 45 | 46 | 47 | 49 | 57 | 44 | | | | |
| | Kabat | 60 | 61 | 47 | 49 | 57 | 44 | | | | |
| | IMGT | 35 | 36 | 37 | 38 | 43 | 44 | | | | |
| 19F6-Hu34 | Chothia | 64 | 65 | 47 | 50 | 58 | 44 | 86 | 90 | | |
| | AbM | 45 | 46 | 47 | 50 | 58 | 44 | | | | |
| | Kabat | 60 | 61 | 47 | 50 | 58 | 44 | | | | |
| | IMGT | 35 | 36 | 37 | 38 | 43 | 44 | | | | |
| 19F6-Hu35 | Chothia | 64 | 65 | 47 | 51 | 56 | 44 | 86 | 91 | | |
| | AbM | 45 | 46 | 47 | 51 | 56 | 44 | | | | |
| | Kabat | 60 | 61 | 47 | 51 | 56 | 44 | | | | |
| | IMGT | 35 | 36 | 37 | 38 | 43 | 44 | | | | |
| 19F6-Hu35V1 | Chothia | 64 | 65 | 47 | 52 | 56 | 44 | 86 | 92 | | |
| | AbM | 45 | 46 | 47 | 52 | 56 | 44 | | | | |
| | Kabat | 60 | 61 | 47 | 52 | 56 | 44 | | | | |
| | IMGT | 35 | 36 | 37 | 39 | 43 | 44 | | | | |
| 19F6-Hu35V2 | Chothia | 64 | 65 | 47 | 53 | 56 | 44 | 86 | 93 | | |
| | AbM | 45 | 46 | 47 | 53 | 56 | 44 | | | | |
| | Kabat | 60 | 61 | 47 | 53 | 56 | 44 | | | | |
| | IMGT | 35 | 36 | 37 | 40 | 43 | 44 | | | | |
| 19F6-Hu35V3 | Chothia | 64 | 65 | 47 | 52 | 56 | 44 | 86 | 94 | | |
| | AbM | 45 | 46 | 47 | 52 | 56 | 44 | | | | |
| | Kabat | 60 | 61 | 47 | 52 | 56 | 44 | | | | |
| | IMGT | 35 | 36 | 37 | 39 | 43 | 44 | | | | |
| 19F6-Hu35V4 | Chothia | 64 | 65 | 47 | 54 | 56 | 44 | 86 | 95 | | |
| | AbM | 45 | 46 | 47 | 54 | 56 | 44 | | | | |
| | Kabat | 60 | 61 | 47 | 54 | 56 | 44 | | | | |
| | IMGT | 35 | 36 | 37 | 41 | 43 | 44 | | | | |
| 19F6-Hu35V5 | Chothia | 64 | 65 | 47 | 51 | 56 | 44 | 86 | 96 | | |
| | AbM | 45 | 46 | 47 | 51 | 56 | 44 | | | | |
| | Kabat | 60 | 61 | 47 | 51 | 56 | 44 | | | | |
| | IMGT | 35 | 36 | 37 | 38 | 43 | 44 | | | | |
| 19F6-Hu35V6 | Chothia | 64 | 65 | 47 | 55 | 56 | 44 | 86 | 97 | | |
| | AbM | 45 | 46 | 47 | 55 | 56 | 44 | | | | |
| | Kabat | 60 | 61 | 47 | 55 | 56 | 44 | | | | |
| | IMGT | 35 | 36 | 37 | 42 | 43 | 44 | | | | |
| 19F6-Hu35V7 | Chothia | 64 | 65 | 47 | 52 | 56 | 44 | 86 | 98 | | |
| | AbM | 45 | 46 | 47 | 52 | 56 | 44 | | | | |
| | Kabat | 60 | 61 | 47 | 52 | 56 | 44 | | | | |
| | IMGT | 35 | 36 | 37 | 39 | 43 | 44 | | | | |
| 19F6-Hu35V8 | Chothia | 64 | 65 | 47 | 55 | 56 | 44 | 86 | 99 | | |
| | AbM | 45 | 46 | 47 | 55 | 56 | 44 | | | | |
| | Kabat | 60 | 61 | 47 | 55 | 56 | 44 | | | | |
| | IMGT | 35 | 36 | 37 | 42 | 43 | 44 | | | | |
| 19F6-Hu35V9 | Chothia | 64 | 65 | 47 | 52 | 56 | 44 | 86 | 100 | | |
| | AbM | 45 | 46 | 47 | 52 | 56 | 44 | | | | |
| | Kabat | 60 | 61 | 47 | 52 | 56 | 44 | | | | |
| | IMGT | 35 | 36 | 37 | 39 | 43 | 44 | | | | |
| 19F6-Hu35V10 | Chothia | 64 | 65 | 47 | 53 | 56 | 44 | 86 | 101 | | |
| | AbM | 45 | 46 | 47 | 53 | 56 | 44 | | | | |
| | Kabat | 60 | 61 | 47 | 53 | 56 | 44 | | | | |
| | IMGT | 35 | 36 | 37 | 40 | 43 | 44 | | | | |

**Table 4-2. Amino acid sequences of variable and constant regions of 3D8 anti-human ROR1 humanized antibody**

| Name | Numbering system | Heavy chain CDR1 | Heavy chain CDR2 | Heavy chain CDR3 | Light chain CDR1 | Light chain CDR2 | Light chain CDR3 | VH | VL | CH | CL |
|---|---|---|---|---|---|---|---|---|---|---|---|
| | | (SEQ ID NO:) | (SEQ ID NO:) | (SEQ ID NO:) | (SEQ ID NO:) | (SEQ ID NO:) | (SEQ ID NO:) | (SEQ ID NO:) | (SEQ ID NO:) | (SEQ ID NO:) | (SEQ ID NO:) |
| 3D8-HuC24 | Chothia | 31 | 32 | 20 | 77 | 25 | 15 | 102 | 107 | 119 | 120 |
| | AbM | 16 | 17 | 20 | 77 | 25 | 15 | | | | |
| | Kabat | 27 | 28 | 20 | 77 | 25 | 15 | | | | |
| | IMGT | 7 | 8 | 11 | 13 | 14 | 15 | | | | |
| 3D8-Hu25 | Chothia | 31 | 32 | 20 | 22 | 25 | 15 | 102 | 108 | | |
| | AbM | 16 | 17 | 20 | 22 | 25 | 15 | | | | |
| | Kabat | 27 | 28 | 20 | 22 | 25 | 15 | | | | |
| | IMGT | 7 | 8 | 11 | 12 | 14 | 15 | | | | |
| 3D8-Hu26 | Chothia | 31 | 32 | 20 | 22 | 25 | 15 | 102 | 109 | | |
| | AbM | 16 | 17 | 20 | 22 | 25 | 15 | | | | |
| | Kabat | 27 | 28 | 20 | 22 | 25 | 15 | | | | |
| | IMGT | 7 | 8 | 11 | 12 | 14 | 15 | | | | |
| 3D8-Hu27 | Chothia | 31 | 32 | 20 | 23 | 25 | 15 | 102 | 110 | | |
| | AbM | 16 | 17 | 20 | 23 | 25 | 15 | | | | |
| | Kabat | 27 | 28 | 20 | 23 | 25 | 15 | | | | |
| | IMGT | 7 | 8 | 11 | 13 | 14 | 15 | | | | |
| 3D8-HuC44 | Chothia | 31 | 32 | 20 | 77 | 25 | 15 | 103 | 107 | | |
| | AbM | 16 | 17 | 20 | 77 | 25 | 15 | | | | |
| | Kabat | 27 | 28 | 20 | 77 | 25 | 15 | | | | |
| | IMGT | 7 | 8 | 11 | 13 | 14 | 15 | | | | |
| 3D8-Hu45 | Chothia | 31 | 32 | 20 | 22 | 25 | 15 | 103 | 108 | | |
| | AbM | 16 | 17 | 20 | 22 | 25 | 15 | | | | |
| | Kabat | 27 | 28 | 20 | 22 | 25 | 15 | | | | |
| | IMGT | 7 | 8 | 11 | 12 | 14 | 15 | | | | |
| 3D8-Hu46 | Chothia | 31 | 32 | 20 | 22 | 25 | 15 | 103 | 109 | | |
| | AbM | 16 | 17 | 20 | 22 | 25 | 15 | | | | |
| | Kabat | 27 | 28 | 20 | 22 | 25 | 15 | | | | |
| | IMGT | 7 | 8 | 11 | 12 | 14 | 15 | | | | |
| 3D8-Hu47 | Chothia | 31 | 32 | 20 | 23 | 25 | 15 | 103 | 110 | | |
| | AbM | 16 | 17 | 20 | 23 | 25 | 15 | | | | |
| | Kabat | 27 | 28 | 20 | 23 | 25 | 15 | | | | |
| | IMGT | 7 | 8 | 11 | 13 | 14 | 15 | | | | |
| 3D8-HuC54 | Chothia | 31 | 32 | 20 | 77 | 25 | 15 | 104 | 107 | | |
| | AbM | 16 | 17 | 20 | 77 | 25 | 15 | | | | |
| | Kabat | 27 | 28 | 20 | 77 | 25 | 15 | | | | |
| | IMGT | 7 | 8 | 11 | 13 | 14 | 15 | | | | |
| 3D8-Hu55 | Chothia | 31 | 32 | 20 | 22 | 25 | 15 | 104 | 108 | | |
| | AbM | 16 | 17 | 20 | 22 | 25 | 15 | | | | |
| | Kabat | 27 | 28 | 20 | 22 | 25 | 15 | | | | |
| | IMGT | 7 | 8 | 11 | 12 | 14 | 15 | | | | |
| 3D8-Hu56 | Chothia | 31 | 32 | 20 | 22 | 25 | 15 | 104 | 109 | | |
| | AbM | 16 | 17 | 20 | 22 | 25 | 15 | | | | |
| | Kabat | 27 | 28 | 20 | 22 | 25 | 15 | | | | |
| | IMGT | 7 | 8 | 11 | 12 | 14 | 15 | | | | |
| 3D8-Hu57 | Chothia | 31 | 32 | 20 | 23 | 25 | 15 | 104 | 110 | | |
| | AbM | 16 | 17 | 20 | 23 | 25 | 15 | | | | |
| | Kabat | 27 | 28 | 20 | 23 | 25 | 15 | | | | |
| | IMGT | 7 | 8 | 11 | 13 | 14 | 15 | | | | |
| 3D8-HuC84 | Chothia | 31 | 33 | 20 | 77 | 25 | 15 | 105 | 107 | | |
| | AbM | 16 | 18 | 20 | 77 | 25 | 15 | | | | |
| | Kabat | 27 | 29 | 20 | 77 | 25 | 15 | | | | |
| | IMGT | 7 | 9 | 11 | 13 | 14 | 15 | | | | |
| 3D8-Hu85 | Chothia | 31 | 33 | 20 | 22 | 25 | 15 | 105 | 108 | | |
| | AbM | 16 | 18 | 20 | 22 | 25 | 15 | | | | |
| | Kabat | 27 | 29 | 20 | 22 | 25 | 15 | | | | |
| | IMGT | 7 | 9 | 11 | 12 | 14 | 15 | | | | |
| 3D8-Hu86 | Chothia | 31 | 33 | 20 | 22 | 25 | 15 | 105 | 109 | | |
| | AbM | 16 | 18 | 20 | 22 | 25 | 15 | | | | |
| | Kabat | 27 | 29 | 20 | 22 | 25 | 15 | | | | |
| | IMGT | 7 | 9 | 11 | 12 | 14 | 15 | | | | |
| 3D8-Hu87 | Chothia | 31 | 33 | 20 | 23 | 25 | 15 | 105 | 110 | | |
| | AbM | 16 | 18 | 20 | 23 | 25 | 15 | | | | |
| | Kabat | 27 | 29 | 20 | 23 | 25 | 15 | | | | |
| | IMGT | 7 | 9 | 11 | 13 | 14 | 15 | | | | |
| 3D8-HuC94 | Chothia | 31 | 34 | 20 | 77 | 25 | 15 | 106 | 107 | | |
| | AbM | 16 | 19 | 20 | 77 | 25 | 15 | | | | |
| | Kabat | 27 | 30 | 20 | 77 | 25 | 15 | | | | |
| | IMGT | 7 | 10 | 11 | 13 | 14 | 15 | | | | |
| 3D8-Hu95 | Chothia | 31 | 34 | 20 | 22 | 25 | 15 | 106 | 108 | | |
| | AbM | 16 | 19 | 20 | 22 | 25 | 15 | | | | |
| | Kabat | 27 | 30 | 20 | 22 | 25 | 15 | | | | |
| | IMGT | 7 | 10 | 11 | 12 | 14 | 15 | | | | |
| 3D8-Hu96 | Chothia | 31 | 34 | 20 | 22 | 25 | 15 | 106 | 109 | | |
| | AbM | 16 | 19 | 20 | 22 | 25 | 15 | | | | |
| | Kabat | 27 | 30 | 20 | 22 | 25 | 15 | | | | |
| | IMGT | 7 | 10 | 11 | 12 | 14 | 15 | | | | |
| 3D8-Hu97 | Chothia | 31 | 34 | 20 | 23 | 25 | 15 | 106 | 110 | | |
| | AbM | 16 | 19 | 20 | 23 | 25 | 15 | | | | |
| | Kabat | 27 | 30 | 20 | 23 | 25 | 15 | | | | |
| | IMGT | 7 | 10 | 11 | 13 | 14 | 15 | | | | |

**Table 4-3. Amino acid sequences of variable and constant regions of 38F8 anti-human ROR1 humanized antibody**

| Name | Numbering system | Heavy chain CDR1 | Heavy chain CDR2 | Heavy chain CDR3 | Light chain CDR1 | Light chain CDR2 | Light chain CDR3 | VH | VL | CH | CL |
|---|---|---|---|---|---|---|---|---|---|---|---|
| | | (SEQ ID NO:) | (SEQ ID NO:) | (SEQ ID NO:) | (SEQ ID NO:) | (SEQ ID NO:) | (SEQ ID NO:) | (SEQ ID NO:) | (SEQ ID NO:) | (SEQ ID NO:) | (SEQ ID NO:) |
| 38F8-Hu14 | Chothia | 82 | 83 | 74 | 77 | 78 | 15 | 111 | 107 | 119 | 120 |
| | AbM | 72 | 73 | 74 | 77 | 78 | 15 | | | | |
| | Kabat | 79 | 81 | 74 | 77 | 78 | 15 | | | | |
| | IMGT | 66 | 67 | 68 | 70 | 71 | 15 | | | | |
| 38F8-Hu16 | Chothia | 82 | 83 | 74 | 76 | 78 | 15 | 111 | 116 | | |
| | AbM | 72 | 73 | 74 | 76 | 78 | 15 | | | | |
| | Kabat | 79 | 81 | 74 | 76 | 78 | 15 | | | | |
| | IMGT | 66 | 67 | 68 | 69 | 71 | 15 | | | | |
| 38F8-Hu17 | Chothia | 82 | 83 | 74 | 77 | 78 | 15 | 111 | 117 | | |
| | AbM | 72 | 73 | 74 | 77 | 78 | 15 | | | | |
| | Kabat | 79 | 81 | 74 | 77 | 78 | 15 | | | | |
| | IMGT | 66 | 67 | 68 | 70 | 71 | 15 | | | | |
| 38F8-Hu18 | Chothia | 82 | 83 | 74 | 76 | 78 | 15 | 111 | 118 | | |
| | AbM | 72 | 73 | 74 | 76 | 78 | 15 | | | | |
| | Kabat | 79 | 81 | 74 | 76 | 78 | 15 | | | | |
| | IMGT | 66 | 67 | 68 | 69 | 71 | 15 | | | | |
| 38F8-Hu24 | Chothia | 82 | 83 | 74 | 77 | 78 | 15 | 112 | 107 | | |
| | AbM | 72 | 73 | 74 | 77 | 78 | 15 | | | | |
| | Kabat | 79 | 80 | 74 | 77 | 78 | 15 | | | | |
| | IMGT | 66 | 67 | 68 | 70 | 71 | 15 | | | | |
| 38F8-Hu26 | Chothia | 82 | 83 | 74 | 76 | 78 | 15 | 112 | 116 | | |
| | AbM | 72 | 73 | 74 | 76 | 78 | 15 | | | | |
| | Kabat | 79 | 80 | 74 | 76 | 78 | 15 | | | | |
| | IMGT | 66 | 67 | 68 | 69 | 71 | 15 | | | | |
| 38F8-Hu27 | Chothia | 82 | 83 | 74 | 77 | 78 | 15 | 112 | 117 | | |
| | AbM | 72 | 73 | 74 | 77 | 78 | 15 | | | | |
| | Kabat | 79 | 80 | 74 | 77 | 78 | 15 | | | | |
| | IMGT | 66 | 67 | 68 | 70 | 71 | 15 | | | | |
| 38F8-Hu28 | Chothia | 82 | 83 | 74 | 76 | 78 | 15 | 112 | 118 | | |
| | AbM | 72 | 73 | 74 | 76 | 78 | 15 | | | | |
| | Kabat | 79 | 80 | 74 | 76 | 78 | 15 | | | | |
| | IMGT | 66 | 67 | 68 | 69 | 71 | 15 | | | | |
| | Chothia | 82 | 83 | 74 | 77 | 78 | 15 | 113 | 107 | | |
| 38F8-Hu34 | AbM | 72 | 73 | 74 | 77 | 78 | 15 | | | | |
| | Kabat | 79 | 81 | 74 | 77 | 78 | 15 | | | | |
| | IMGT | 66 | 67 | 68 | 70 | 71 | 15 | | | | |
| 38F8-Hu36 | Chothia | 82 | 83 | 74 | 76 | 78 | 15 | 113 | 116 | | |
| | AbM | 72 | 73 | 74 | 76 | 78 | 15 | | | | |
| | Kabat | 79 | 81 | 74 | 76 | 78 | 15 | | | | |
| | IMGT | 66 | 67 | 68 | 69 | 71 | 15 | | | | |
| 38F8-Hu37 | Chothia | 82 | 83 | 74 | 77 | 78 | 15 | 113 | 117 | | |
| | AbM | 72 | 73 | 74 | 77 | 78 | 15 | | | | |
| | Kabat | 79 | 81 | 74 | 77 | 78 | 15 | | | | |
| | IMGT | 66 | 67 | 68 | 70 | 71 | 15 | | | | |
| 38F8-Hu38 | Chothia | 82 | 83 | 74 | 76 | 78 | 15 | 113 | 118 | | |
| | AbM | 72 | 73 | 74 | 76 | 78 | 15 | | | | |
| | Kabat | 79 | 81 | 74 | 76 | 78 | 15 | | | | |
| | IMGT | 66 | 67 | 68 | 69 | 71 | 15 | | | | |
| 38F8-Hu54 | Chothia | 82 | 83 | 74 | 77 | 78 | 15 | 114 | 107 | | |
| | AbM | 72 | 73 | 74 | 77 | 78 | 15 | | | | |
| | Kabat | 79 | 80 | 74 | 77 | 78 | 15 | | | | |
| | IMGT | 66 | 67 | 68 | 70 | 71 | 15 | | | | |
| 38F8-Hu56 | Chothia | 82 | 83 | 74 | 76 | 78 | 15 | 114 | 116 | | |
| | AbM | 72 | 73 | 74 | 76 | 78 | 15 | | | | |
| | Kabat | 79 | 80 | 74 | 76 | 78 | 15 | | | | |
| | IMGT | 66 | 67 | 68 | 69 | 71 | 15 | | | | |
| 38F8-Hu57 | Chothia | 82 | 83 | 74 | 77 | 78 | 15 | 114 | 117 | | |
| | AbM | 72 | 73 | 74 | 77 | 78 | 15 | | | | |
| | Kabat | 79 | 80 | 74 | 77 | 78 | 15 | | | | |
| | IMGT | 66 | 67 | 68 | 70 | 71 | 15 | | | | |
| 38F8-Hu58 | Chothia | 82 | 83 | 74 | 76 | 78 | 15 | 114 | 118 | | |
| | AbM | 72 | 73 | 74 | 76 | 78 | 15 | | | | |
| | Kabat | 79 | 80 | 74 | 76 | 78 | 15 | | | | |
| | IMGT | 66 | 67 | 68 | 69 | 71 | 15 | | | | |
| 38F8-Hu64 | Chothia | 82 | 83 | 74 | 77 | 78 | 15 | 115 | 107 | | |
| | AbM | 72 | 73 | 74 | 77 | 78 | 15 | | | | |
| | Kabat | 79 | 80 | 74 | 77 | 78 | 15 | | | | |
| | IMGT | 66 | 67 | 68 | 70 | 71 | 15 | | | | |
| 38F8-Hu66 | Chothia | 82 | 83 | 74 | 76 | 78 | 15 | 115 | 116 | | |
| | AbM | 72 | 73 | 74 | 76 | 78 | 15 | | | | |
| | Kabat | 79 | 80 | 74 | 76 | 78 | 15 | | | | |
| | IMGT | 66 | 67 | 68 | 69 | 71 | 15 | | | | |
| 38F8-Hu67 | Chothia | 82 | 83 | 74 | 77 | 78 | 15 | 115 | 117 | | |
| | AbM | 72 | 73 | 74 | 77 | 78 | 15 | | | | |
| | Kabat | 79 | 80 | 74 | 77 | 78 | 15 | | | | |
| | IMGT | 66 | 67 | 68 | 70 | 71 | 15 | | | | |
| 38F8-Hu68 | Chothia | 82 | 83 | 74 | 76 | 78 | 15 | 115 | 118 | | |
| | AbM | 72 | 73 | 74 | 76 | 78 | 15 | | | | |
| | Kabat | 79 | 80 | 74 | 76 | 78 | 15 | | | | |
| | IMGT | 66 | 67 | 68 | 69 | 71 | 15 | | | | |

### Example 6: Determination of affinity of anti-human ROR1 humanized candidate antibodies

In order to evaluate the anti-human ROR1 humanized candidate antibodies, the coding DNA sequences of the candidate humanized antibodies (3D8, 38F8, 19F6) in Example 5 were synthesized and codon-optimized, and then cloned into the pcDNA3.4 plasmid (entrusted to Suzhou Hong Xun Technology Co., Ltd.). The pcDNA3.4 plasmids corresponding to the heavy and light chains of each humanized antibody were simultaneously transfected into Expi293F cells, and protein A was used to purify the expressed antibodies in the supernatant.

The affinities of the anti-human ROR1 humanized antibodies to Ba/F3-ROR1, Jeko-1, HT-29 and A549 cells were detected by flow cytometry. The specific experimental steps were as follows: the Ba/F3-ROR1, Jeko-1, HT-29 and A549 cells were incubated with the purified humanized antibodies of different concentration gradients on ice for 30 minutes, and the anti-ROR1 antibody UC961 with human IgG1 and the anti-TNP antibody were used as positive and negative controls, respectively. Washing was performed twice with flow cytometry buffer (PBS+2% FBS), then incubation was performed on ice for 30 minutes with goat-anti-human fluorescent secondary antibody (Jackson ImmunoResearch, Cat. No.: 109-605-098), and finally the detection was carried out on the machine.

The results were shown in Table 5. The affinity EC50 values of the humanized molecules 19F6-Hu35V1, 3D8-HuC24 and 38F8-Hu57 produced by the three murine antibodies on all four cells was comparable to or lower than that of the positive control antibody UC961, indicating that the above anti-human ROR1 humanized antibodies had comparable or stronger affinity as compared to UC961.

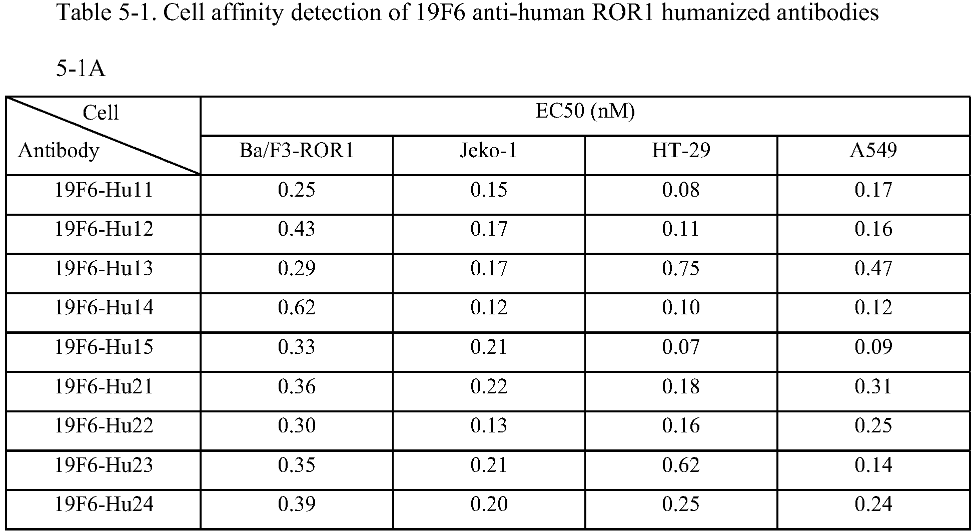

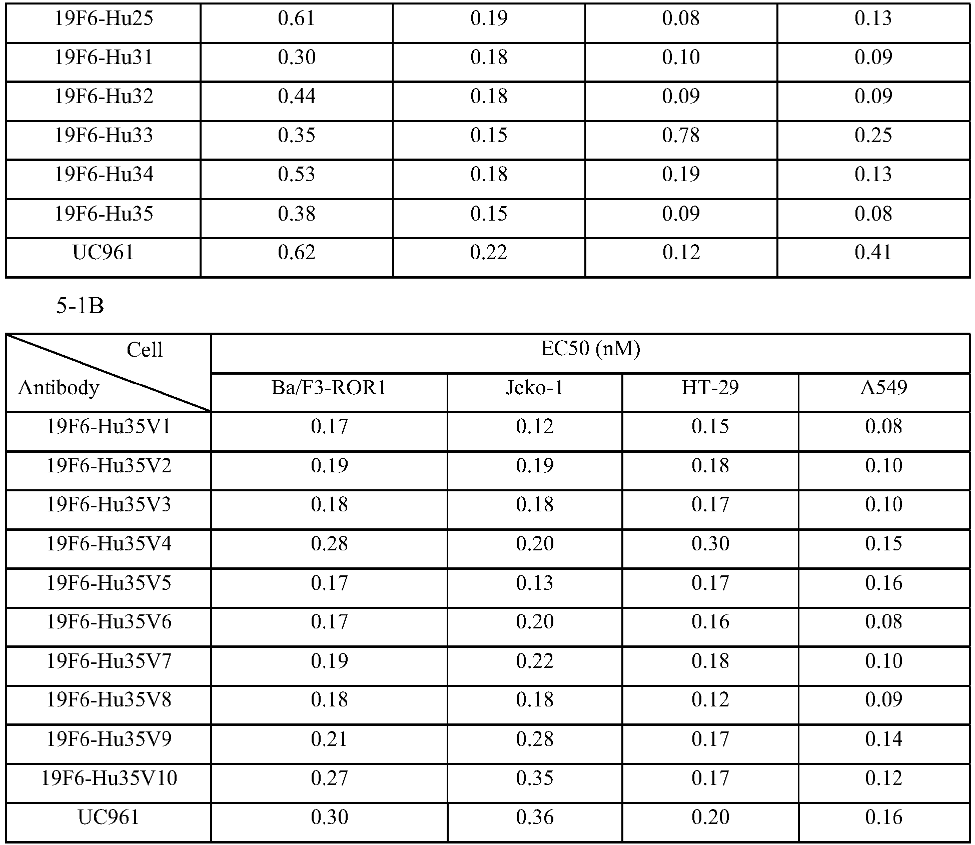

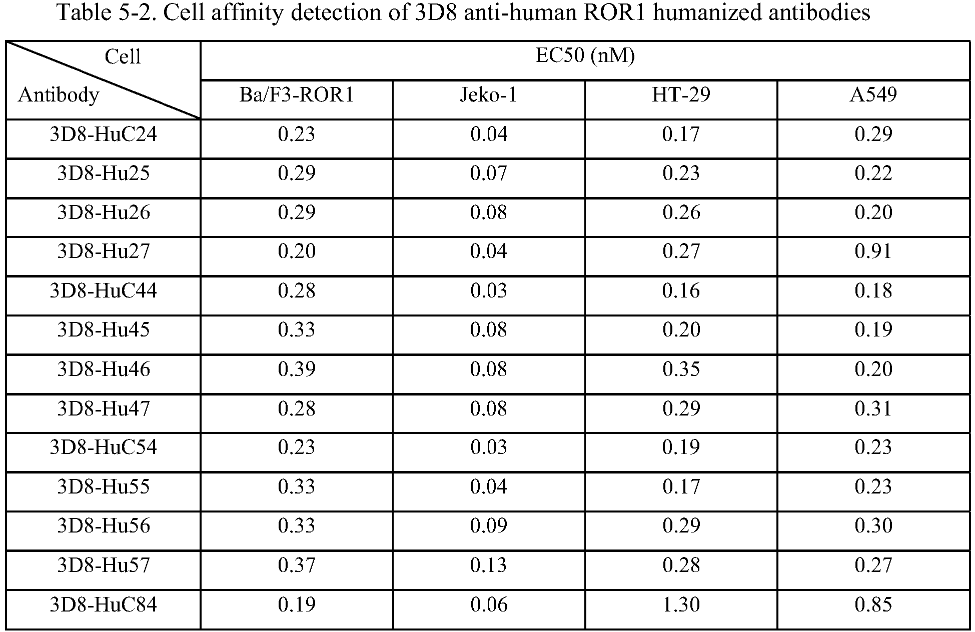

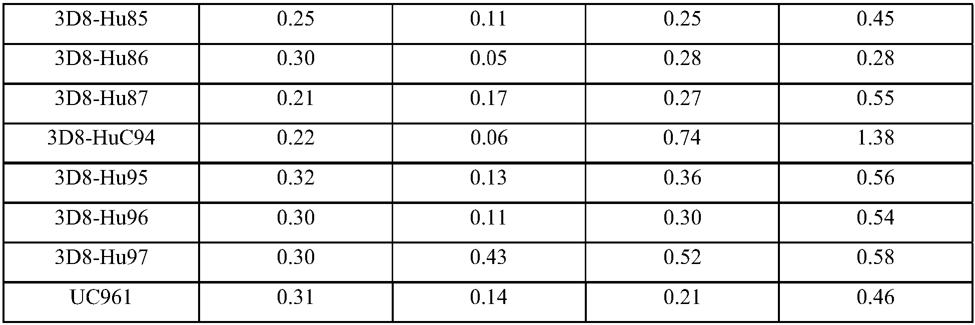

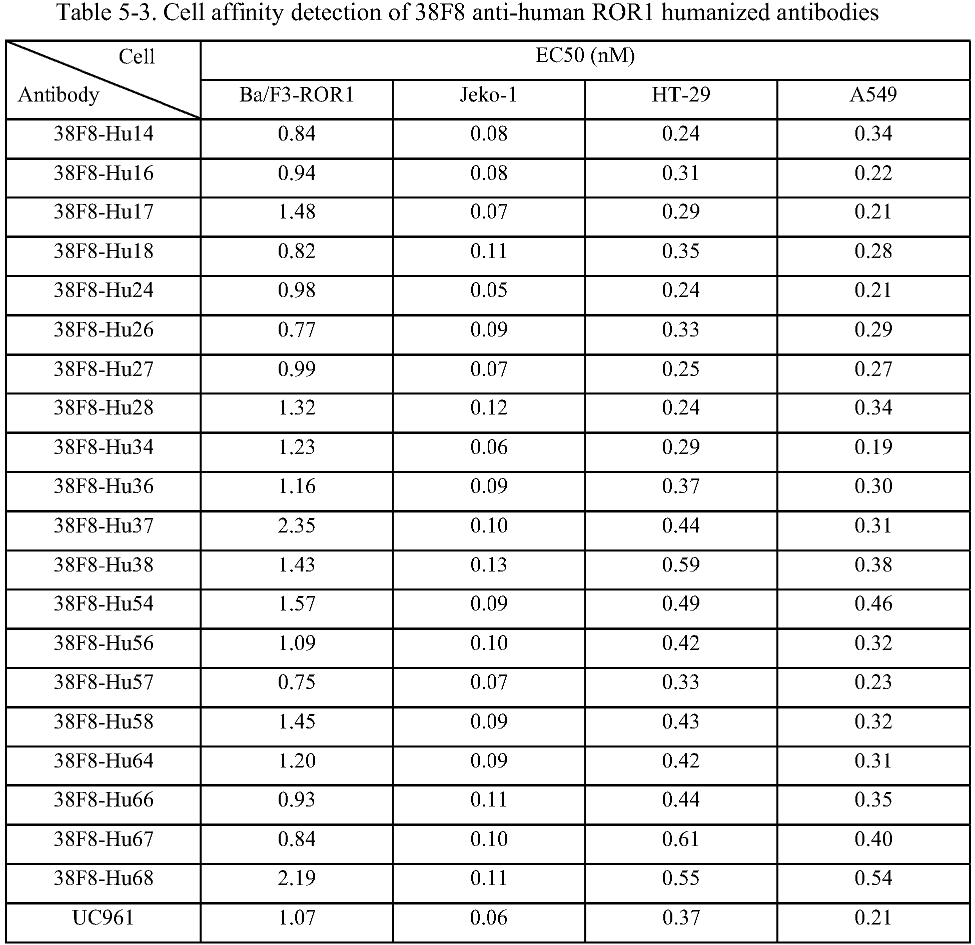

The dynamic affinities of humanized molecules 19F6-Hu35V1, 3D8-HuC24 and 38F8-Hu57 and positive control antibody UC961 to human ROR1 ECD protein were determined by Octet ForteBio method. The specific experimental steps were as follows: the antibody to be tested was first bound to anti-human IgG Fc AHC biosensor (ForteBio, Cat. No.: 18-5060) to reach a response signal value of 0.8-1.2nm, then the biosensor coated with the antibody was immersed in wells containing different concentrations (16, 8, 4, 2, 1, 0.5, 0.25 and 0 µg/mL) of human ROR1 ECD protein to measure dynamic affinity, the binding was performed for 2-4 minutes, then dissociation was carried out for 4-6 minutes, and 1: 1 kinetic binding model was used for all fitting analysis.

As shown in Table 6, the humanized molecule 19F6-Hu35V1 showed a dissociation rate significantly slower than that of the control antibody UC961 (as indicated by Kdis value), and an affinity 5.4 times stronger than that of the control antibody UC961 (as indicated by KD value), while the 3D8-HuC24 and 38F8-Hu57 showed comparable affinity to the control antibody UC961.

**Table 6. Determination of dynamic affinity of anti-human ROR1 humanized antibodies to human ROR1**

| Antibody | KD (M) | kon(1/Ms) | kdis(1/s) |
|---|---|---|---|
| 19F6-Hu35v1 | 1.61E-09 | 5.38E+05 | 8.63E-04 |
| 3D8-HuC24 | 2.61E-08 | 5.86E+05 | 1.53E-02 |
| 38F8-Hu57 | 1.00E-08 | 2.41E+05 | 2.42E-03 |
| UC961 | 8.73E-09* | 1.26E+06* | 1.12E-02* |

| | | | |
|---|---|---|---|
| * means that the value is an average of three determinations. | | | |

### Example 7: Determination of ADCC activity of anti-human ROR1 humanized candidate antibodies

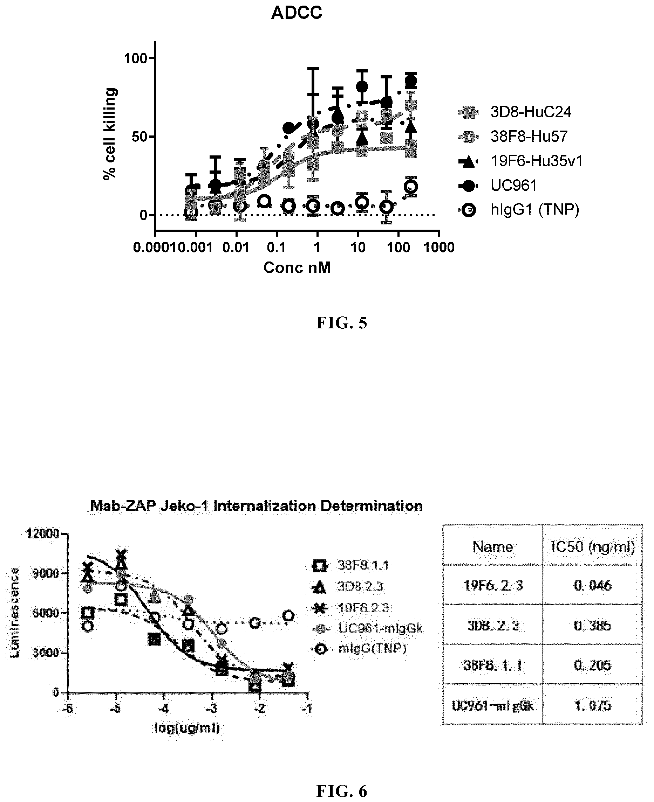

Humanized anti-human ROR1 candidate antibodies 19F6-Hu35V1, 3D8-HuC24 and 38F8-Hu57 were of the IgG1 subtype, and have strong antibody-dependent cell-mediated cytotoxicity (ADCC) activity. In order to measure the ADCC activities of 19F6-Hu35V1, 3D8-HuC24 and 38F8-Hu57, the method of NK cells killing was adopted for detection. The specific experimental steps were as follows: the Ba/F3-ROR1 cells were centrifuged, resuspended in culture medium, adjusted to have a cell density of 1×10⁴ cells/40 µL/well, and added to the corresponding wells of a 96-well plate; the NK92MI-mCD16a-hFceRI cells were centrifuged, resuspended with MEMA culture medium, adjusted to have a cell density of 5×10⁴/50 µL/well, and added to the corresponding wells; the 19F6-Hu35V1, 3D8-HuC24, 38F8-Hu57 and UC961 antibodies were diluted with MEMA culture medium by 4-fold dilution starting from 1000nM for a total of 10 concentration points. The diluted antibody was added to the corresponding well at 10 µL/well, placed in a 37°C, 5% CO₂ cell culture incubator and co-cultured with the Ba/F3-ROR1 and NK92MI-mCD16a-hFceRI for 5.5 hours. In the positive control well, 5 µL of 4% Triton-X 100 (Fisher Scientific, Cat. No. BP151-500) was added, allowed to stand for 5-10 minutes, then added with CytoTox-Glo Cytotoxicity detection reagent (Promega, Cat. No. G9291) with 30 µL/well , and after 15 min, the luminescence readings were obtained by a microplate reader (MD, SpectraMax M2), and the results were imported into Graphpad Prism for curve fitting. As shown in FIG. 5 and Table 7, the EC50 values of ADCC killing activity of the humanized antibodies 19F6-Hu35V1, 3D8-HuC24 and 38F8-Hu57 on Ba/F3-ROR1 cells were comparable to that of the control antibody UC961.

**Table 7. Determination of ADCC activity of anti-human ROR1 humanized candidate antibodies**

| Antibody | EC50 (nM) |
|---|---|
| 19F6-Hu35V1 | 0.31 |
| 3D8-HuC24 | 0.28 |
| 38F8-Hu57 | 0.19 |
| UC961 | 0.33 |

### Example 8: Determination of internalization induced by anti-human ROR1 candidate antibodies

Binding of antibodies to target proteins on the cell surface may induce the internalization of antibody-antigen complexes. In the present invention, Mab-ZAP kit (ATSBio, IT-04) was adopted to measure the activity of inducing internalization of mouse-anti-human ROR1 candidate antibodies (Kohls MD and Lappi DA BioTechniques 2000, 28: 162). The specific experimental steps were as follows: Jeko-1 cells were counted by a cell counter, the cell concentration was adjusted to 7500 cells/well, then 90uL of the cells were added to a 96-well plate, and incubated overnight in an incubator at 37°C and 5% CO₂. On the second day, the murine antibody 19F6.2.3, 3D8.2.3, 38F8.1.1, and UC961 control antibody were diluted 5 times with complete culture medium starting from 10ug/ml, with a total of 8 concentration points. 10ul of the antibody at different concentrations was transferred into Jeko-1 cells. Mab-zap solution with a concentration of 10ug/mL (50×) was prepared according to the kit instructions, and added at 2ul per well, and the cells were incubated at 37°C, 5% CO₂ for 3 days. CTG2.0 (Promega, Cat. No.: G9241) was pre-warmed in the dark at room temperature, and 100uL of the CTG reagent was added to each well, covered with aluminum foil, shaken on a rotary shaker for 2 minutes, and then incubated at room temperature for 10 minutes to stabilize luminescence signal. Luminescence value was read with a plate reader. As shown in FIG. 6, the activity of inducing internalization of the antibodies 19F6.2.3, 3D8.2.3 and 38F8.1.1 on Jeko-1 cells were higher than that of the control antibody UC961, that was, 23.4, 3.8 and 5.3 times stronger than that of the control antibody UC961, respectively (as indicated by IC50 values).

### Example 9: Detection of internalization activity of anti-human ROR1 candidate antibodies

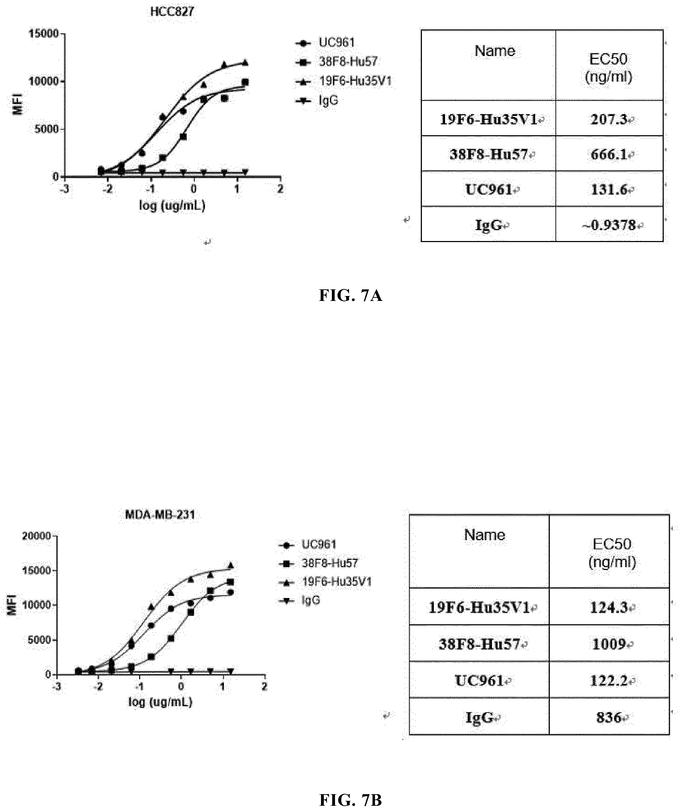

In the present invention, the acid wash method was used to measure the internalization activities of the anti-human ROR1 candidate antibodies. The specific experimental steps were as follows: HCC827 and MDA-MB-231 cells were digested by trypsin, counted and then adjusted to have a cell concentration of 200,000 cells/well, after the cells were washed twice with PBS, the cells were resuspended with 2% BSA, and then 50uL of the cells was added to a 96-well plate. At the same time, the antibodies 19F6-Hu35V1, 38F8-Hu57, UC961 control antibody, IgG antibody (chicken lysozyme antibody) were diluted by 3-fold dilution starting from 15 µg/mL for a total of 8 or 9 concentration points. 50 µL of each antibody at different concentrations was transferred into the cells and mixed well (plate 1); meanwhile, each antibody at the highest concentration point was mixed with the cells in another 96-well plate (plate 2), and the two plates were incubated at 4°C for 40 min. After incubation, washing was performed twice with PBS, and centrifugation was carried out at 500g for 3 min. Anti-human IgG secondary antibody (Alexa Fluor 488) was diluted at 1: 150, added at 50 µL per well, mixed well and incubated at 4°C for 20 min. After the incubation, the cells were washed twice with PBS, centrifuged at 500 g for 3 min, the cells were resuspended with 200 µL of the corresponding complete culture medium, the plate 1 was placed at 37°C and the plate 2 was placed at 4°C, and incubation was continued for 4 h. After the incubation, centrifugation was perform at 500g for 3min to remove the medium, 100µL of acid (150mM NaCl, 0.1M glycine, adjusted with hydrochloric acid to pH 2.0) was added to each well, resuspended and washed for 2 minutes, then 60ul of alkali (3 g/L Tris base, 9 g/L NaCl, adjusted with NaOH to pH 13) was added and mixed well to neutralize; the supernatant was discarded, PBS was added for resuspension, and then detection was performed by flow cytometry. The results were shown in FIGS. 7A to 7B. The internalization activity of the antibody 19F6-Hu35V1 in the HCC827 and MDA-MB-231 cells were comparable to those of the control antibody UC961, the maximum internalization signal of antibody 19F6-Hu35V1 was slightly stronger, and the internalization activity of 38F8-Hu57 was slightly weaker in general. The negative control antibody IgG showed no internalization in both cells.

### Example 10: Detection of internalization activity of anti-human ROR1 candidate antibodies

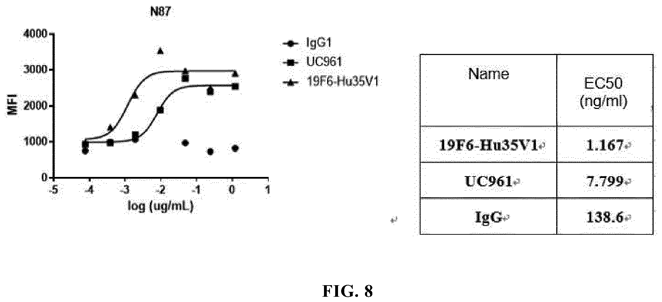

In the present invention, pHrodo dye (Thermo) was also used to determine the internalization activity of the anti-human ROR1 candidate antibodies. The specific experimental steps were as follows: N87 cells were digested with trypsin, the cells were counted with a cell counter, adjusted to have a cell concentration of 10,000 cells/well, 100 µL of the cells were coated on a 96-well plate, and incubated overnight in an incubator at 37°C and 5% CO₂. On the second day, the antibody 19F6-Hu35V1, UC961 control antibody, and IgG antibody were diluted with 1640 complete culture medium by 5-fold dilution starting from 2.4 µg/ml for a total of 7 concentration points. At the same time, pHrodo^{™} Red dye was diluted with 1640 complete culture medium to 12 µg/mL, 30 µL of each antibody at different concentrations were mixed well with 30 µL of the diluted dye, and incubated at room temperature in the dark for 20 min. After the incubation, the cell culture medium was discarded, the antibody-dye mixture solution was added to the cells of each well, and incubated overnight at 37°C in an incubator with 5% CO₂. On the next day, the cells were digested with trypsin and detected by flow cytometry. The results were shown in FIG. 8. The internalization activity of the antibody 19F6-Hu35V1 in N87 cells was stronger than that of the control antibody UC961, that was, about 7 times of that of UC961. The negative control antibody IgG showed no internalization activity in N87 cells.

## Claims

1. An antibody or antigen-binding fragment thereof that specifically binds to ROR1, wherein the antibody or antigen-binding fragment thereof comprises the following complementarity determining regions (CDRs):
(a) CDR-H1, CDR-H2 and CDR-H3 contained in a heavy chain variable region (VH) as set forth in any one of SEQ ID NOs: 1, and 102-106; and/or
CDR-L1, CDR-L2 and CDR-L3 contained in a light chain variable region (VL) as set forth in any one of SEQ ID NOs: 2, and 107-110;
or
(b) CDR-H1, CDR-H2 and CDR-H3 contained in a heavy chain variable region (VH) as set forth in any one of SEQ ID NOs: 3, and 84-86; and/or
CDR-L1, CDR-L2 and CDR-L3 contained in a light chain variable region (VL) as set forth in any one of SEQ ID NOs: 4, and 87-101;
or
(c) CDR-H1, CDR-H2 and CDR-H3 contained in a heavy chain variable region (VH) as set forth in any one of SEQ ID NOs: 5, and 111-115; and/or
CDR-L1, CDR-L2 and CDR-L3 contained in a light chain variable region (VL) as set forth in any one of SEQ ID NOs: 6, 107, and 116-118;
or
(d) CDR-H1, CDR-H2 and CDR-H3 contained in the following heavy chain variable region (VH), and/or CDR-L1, CDR-L2 and CDR-L3 contained in the following light chain variable region (VL), wherein the heavy chain variable region (VH) and/or the light chain variable region (VL) has at least one CDR that contains a mutation as compared with the heavy chain variable region and/or light chain variable region as described in any one of (a) to (c), and the mutation is a substitution, deletion or addition of one or several amino acids (e.g., a substitution, deletion or addition of 1, 2 or 3 amino acids); preferably, the substitution is a conservative substitution;
preferably, the CDRs are defined according to the Chothia, AbM, Kabat or IMGT numbering system;
preferably, the antibody or antigen-binding fragment thereof binds to human ROR1.

2. The antibody or antigen-binding fragment thereof according to claim 1, wherein the antibody or antigen-binding fragment thereof comprises:
(1) the following heavy chain variable region (VH) and/or light chain variable region (VL), wherein CDRs are defined by the Chothia numbering system:
(a) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
a light chain variable region (VL) comprising the following 3 CDRs: CDR-L1 having a sequence as set forth in any one of SEQ ID NOs: 48-55, CDR-L2 having a sequence as set forth in any one of SEQ ID NOs: 56-58, and CDR-L3 having a sequence as set forth in SEQ ID NO: 44;
or
(b) a heavy chain variable region (VH) comprising the following 3 CDRs: CDR-H1 having a sequence as set forth in SEQ ID NO: 31, CDR-H2 having a sequence as set forth in any one of SEQ ID NOs: 32-34, and CDR-H3 having a sequence as set forth in SEQ ID NO: 20; and/or,
a light chain variable region (VL) comprising the following 3 CDRs: CDR-L1 having a sequence as set forth in any one of SEQ ID NOs: 21-23 and 77, CDR-L2 having a sequence as set forth in SEQ ID NO: 24 or 25, and CDR-L3 having a sequence as set forth in SEQ ID NO: 15;
or
(c) a heavy chain variable region (VH) comprising the following 3 CDRs: CDR-H1 having a sequence as set forth in SEQ ID NO: 82, CDR-H2 having a sequence as set forth in SEQ ID NO: 83, and CDR-H3 having a sequence as set forth in SEQ ID NO: 74; and/or,
a light chain variable region (VL) comprising the following 3 CDRs: CDR-L1 having a sequence as set forth in any one of SEQ ID NOs: 75-77, CDR-L2 having a sequence as set forth in SEQ ID NO: 24 or 78, and CDR-L3 having a sequence as set forth in SEQ ID NO: 15;
(2) the following heavy chain variable region (VH) and/or light chain variable region (VL), wherein CDRs are defined by the AbM numbering system:
(a) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
a light chain variable region (VL) comprising the following 3 CDRs: CDR-L1 having a sequence as set forth in any one of SEQ ID NOs: 48-55, CDR-L2 having a sequence as set forth in any one of SEQ ID NOs: 56-58, and CDR-L3 having a sequence as set forth in SEQ ID NO: 44;
or
(b) a heavy chain variable region (VH) comprising the following 3 CDRs: CDR-H1 having a sequence as set forth in SEQ ID NO: 16, CDR-H2 having a sequence as set forth in any one of SEQ ID NOs: 17-19, and CDR-H3 having a sequence as set forth in SEQ ID NO: 20; and/or,
a light chain variable region (VL) comprising the following 3 CDRs: CDR-L1 having a sequence as set forth in any one of SEQ ID NOs: 21-23, and 77, CDR-L2 having a sequence as set forth in SEQ ID NO: 24 or 25, and CDR-L3 having a sequence as set forth in SEQ ID NO: 15;
or
(c) a heavy chain variable region (VH) comprising the following 3 CDRs: CDR-H1 having a sequence as set forth in SEQ ID NO: 72, CDR-H2 having a sequence as set forth in SEQ ID NO: 73, and CDR-H3 having a sequence as set forth in SEQ ID NO: 74; and/or,
a light chain variable region (VL) comprising the following 3 CDRs: CDR-L1 having a sequence as set forth in any one of SEQ ID NOs: 75-77, CDR-L2 having a sequence as set forth in SEQ ID NO: 24 or 78, CDR-L3 having a sequence as set forth in SEQ ID NO: 15;
or
(3) the following heavy chain variable region (VH) and/or light chain variable region (VL), wherein CDRs are defined by the Kabat numbering system:
(a) a heavy chain variable region (VH) comprising the following 3 CDRs: CDR-H1 having a sequence as set forth in SEQ ID NO: 60, CDR-H2 having a sequence as set forth in any one of SEQ ID NOs: 61-63, and CDR-H3 having a sequence as set forth in SEQ ID NO: 47; and/or,
a light chain variable region (VL) comprising the following 3 CDRs: CDR-L1 having a sequence as set forth in any one of SEQ ID NOs: 48-55, CDR-L2 having a sequence as set forth in any one of SEQ ID NOs: 56-58, and CDR-L3 having a sequence as set forth in SEQ ID NO: 44;
or
(b) a heavy chain variable region (VH) comprising the following 3 CDRs: CDR-H1 having a sequence as set forth in SEQ ID NO: 27, CDR-H2 having a sequence as set forth in any one of SEQ ID NOs: 28-30, and CDR-H3 having a sequence as set forth in SEQ ID NO: 20; and/or,
a light chain variable region (VL) comprising the following 3 CDRs: CDR-L1 having a sequence as set forth in any one of SEQ ID NOs: 21-23, and 77, CDR-L2 having a sequence as set forth in any one of SEQ ID NOs: 24-25, and CDR-L3 having a sequence as set forth in SEQ ID NO: 15;
or
(c) a heavy chain variable region (VH) comprising the following 3 CDRs: CDR-H1 having a sequence as set forth in SEQ ID NO: 79, CDR-H2 having a sequence as set forth in SEQ ID NO: 80 or 81, and CDR-H3 having a sequence as set forth in SEQ ID NO: 74; and/or, a light chain variable region (VL) comprising the following 3 CDRs: CDR-L1 having a sequence as set forth in any one of SEQ ID NOs: 75-77, CDR-L2 having a sequence as set forth in SEQ ID NO: 24 or 78, and CDR-L3 having a sequence as set forth in SEQ ID NO: 15;
or
(4) the following heavy chain variable region (VH) and/or light chain variable region (VL), wherein CDRs are defined by the IMGT numbering system:
(a) a heavy chain variable region (VH) comprising the following 3 CDRs: CDR-H1 having a sequence as set forth in SEQ ID NO: 35, CDR-H2 having a sequence as set forth in SEQ ID NO: 36, and CDR-H3 having a sequence as set forth in SEQ ID NO: 37; and/or,
a light chain variable region (VL) comprising the following 3 CDRs: CDR-L1 having a sequence as set forth in any one of SEQ ID NOs: 38-42, CDR-L2 having a sequence as set forth in SEQ ID NO: 43, and CDR-L3 having a sequence as set forth in SEQ ID NO: 44;
or
(b) a heavy chain variable region (VH) comprising the following 3 CDRs: CDR-H1 having a sequence as set forth in SEQ ID NO: 7, CDR-H2 having a sequence as set forth in any one of SEQ ID NOs: 8-10, and CDR-H3 having a sequence as set forth in SEQ ID NO: 11; and/or,
a light chain variable region (VL) comprising the following 3 CDRs: CDR-L1 having a sequence as set forth in SEQ ID NO: 12 or 13, CDR-L2 having a sequence as set forth in SEQ ID NO: 14, and CDR-L3 having a sequence as set forth in SEQ ID NO: 15;
or
(c) a heavy chain variable region (VH) comprising the following 3 CDRs: CDR-H1 having a sequence as set forth in SEQ ID NO: 66, CDR-H2 having a sequence as set forth in SEQ ID NO: 67, and CDR-H3 having a sequence as set forth in SEQ ID NO: 68; and/or,
a light chain variable region (VL) comprising the following 3 CDRs: CDR-L1 having a sequence as set forth in SEQ ID NO: 69 or 70, CDR-L2 having a sequence as set forth in SEQ ID NO: 71, and CDR-L3 having a sequence as set forth in SEQ ID NO: 15;
or
(5) the following heavy chain variable region (VH) and/or light chain variable region (VL), wherein the heavy chain variable region (VH) and/or light chain variable region (VL) comprise at least one CDR that contains a mutation as compared with the heavy chain variable region and/or light chain variable region described in any one of (a) to (c) in (1) to (4), and the mutation is a substitution, deletion or addition of one or several amino acids (e.g., a substitution, deletion or addition of 1, 2 or 3 amino acids); preferably, the substitution is a conservative substitution;
preferably, the antibody or antigen-binding fragment thereof binds to human ROR1.

3. The antibody or antigen-binding fragment thereof according to claim 1 or 2, wherein the antibody or antigen-binding fragment thereof comprises:
(i) the following heavy chain variable region (VH) and/or light chain variable region (VL), wherein CDRs are defined by the Chothia numbering system:
(1) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
a light chain variable region (VL) comprising the following 3 CDRs: CDR-L1 having a sequence as set forth in SEQ ID NO: 48, CDR-L2 having a sequence as set forth in SEQ ID NO: 56, and CDR-L3 having a sequence as set forth in SEQ ID NO: 44;
or
(2) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
a light chain variable region (VL) comprising the following 3 CDRs: CDR-L1 having a sequence as set forth in SEQ ID NO: 48, CDR-L2 having a sequence as set forth in SEQ ID NO: 57, and CDR-L3 having a sequence as set forth in SEQ ID NO: 44;
or
(3) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
a light chain variable region (VL) comprising the following 3 CDRs: CDR-L1 having a sequence as set forth in SEQ ID NO: 49, CDR-L2 having a sequence as set forth in SEQ ID NO: 57, and CDR-L3 having a sequence as set forth in SEQ ID NO: 44;
or
(4) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
a light chain variable region (VL) comprising the following 3 CDRs: CDR-L1 having a sequence as set forth in SEQ ID NO: 50, CDR-L2 having a sequence as set forth in SEQ ID NO: 58, and CDR-L3 having a sequence as set forth in SEQ ID NO: 44;
or
(5) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
a light chain variable region (VL) comprising the following 3 CDRs: CDR-L1 having a sequence as set forth in SEQ ID NO: 51, CDR-L2 having a sequence as set forth in SEQ ID NO: 56, and CDR-L3 having a sequence as set forth in SEQ ID NO: 44;
or
(6) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
a light chain variable region (VL) comprising the following 3 CDRs: CDR-L1 having a sequence as set forth in SEQ ID NO: 52, CDR-L2 having a sequence as set forth in SEQ ID NO: 56, and CDR-L3 having a sequence as set forth in SEQ ID NO: 44;
or
(7) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
a light chain variable region (VL) comprising the following 3 CDRs: CDR-L1 having a sequence as set forth in SEQ ID NO: 53, CDR-L2 having a sequence as set forth in SEQ ID NO: 56, and CDR-L3 having a sequence as set forth in SEQ ID NO: 44;
or
(8) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
a light chain variable region (VL) comprising the following 3 CDRs: CDR-L1 having a sequence as set forth in SEQ ID NO: 54, CDR-L2 having a sequence as set forth in SEQ ID NO: 56, and CDR-L3 having a sequence as set forth in SEQ ID NO: 44;
or
(9) a heavy chain variable region (VH) comprising the following 3 CDRs: CDR-H1 having a sequence as set forth in SEQ ID NO: 64, CDR-H2 having a sequence as set forth in SEQ ID NO: 65, and CDR-H3 having a sequence as set forth in SEQ ID NO: 47; and/or,
a light chain variable region (VL) comprising the following 3 CDRs: CDR-L1 having a sequence as set forth in SEQ ID NO: 55, CDR-L2 having a sequence as set forth in SEQ ID NO: 56, and CDR-L3 having a sequence as set forth in SEQ ID NO: 44;
or
(10) a heavy chain variable region (VH) comprising the following 3 CDRs: CDR-H1 having a sequence as set forth in SEQ ID NO: 31, CDR-H2 having a sequence as set forth in SEQ ID NO: 32, and CDR-H3 having a sequence as set forth in SEQ ID NO: 20; and/or,
a light chain variable region (VL) comprising the following 3 CDRs: CDR-L1 having a sequence as set forth in SEQ ID NO: 21, CDR-L2 having a sequence as set forth in SEQ ID NO: 24, and CDR-L3 having a sequence as set forth in SEQ ID NO: 15;
or
(11) a heavy chain variable region (VH) comprising the following 3 CDRs: CDR-H1 having a sequence as set forth in SEQ ID NO: 31, CDR-H2 having a sequence as set forth in SEQ ID NO: 32, and CDR-H3 having a sequence as set forth in SEQ ID NO: 20; and/or,
a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
or
(12) a heavy chain variable region (VH) comprising the following 3 CDRs: CDR-H1 having a sequence as set forth in SEQ ID NO: 31, CDR-H2 having a sequence as set forth in SEQ ID NO: 32, and CDR-H3 having a sequence as set forth in SEQ ID NO: 20; and/or,
a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
or
(13) a heavy chain variable region (VH) comprising the following 3 CDRs: CDR-H1 having a sequence as set forth in SEQ ID NO: 31, CDR-H2 having a sequence as set forth in SEQ ID NO: 32, and CDR-H3 having a sequence as set forth in SEQ ID NO: 20; and/or,
a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
or
(14) a heavy chain variable region (VH) comprising the following 3 CDRs: CDR-H1 having a sequence as set forth in SEQ ID NO: 31, CDR-H2 having a sequence as set forth in SEQ ID NO: 33, and CDR-H3 having a sequence as set forth in SEQ ID NO: 20; and/or,
a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
or
(15) a heavy chain variable region (VH) comprising the following 3 CDRs: CDR-H1 having a sequence as set forth in SEQ ID NO: 31, CDR-H2 having a sequence as set forth in SEQ ID NO: 33, and CDR-H3 having a sequence as set forth in SEQ ID NO: 20; and/or,
a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
or
(16) a heavy chain variable region (VH) comprising the following 3 CDRs: CDR-H1 having a sequence as set forth in SEQ ID NO: 31, CDR-H2 having a sequence as set forth in SEQ ID NO: 33, and CDR-H3 having a sequence as set forth in SEQ ID NO: 20; and/or,
a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
or
(17) a heavy chain variable region (VH) comprising the following 3 CDRs: CDR-H1 having a sequence as set forth in SEQ ID NO: 31, CDR-H2 having a sequence as set forth in SEQ ID NO: 34, and CDR-H3 having a sequence as set forth in SEQ ID NO: 20; and/or,
a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
or
(18) a heavy chain variable region (VH) comprising the following 3 CDRs: CDR-H1 having a sequence as set forth in SEQ ID NO: 31, CDR-H2 having a sequence as set forth in SEQ ID NO: 34, and CDR-H3 having a sequence as set forth in SEQ ID NO: 20; and/or,
a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
or
(19) a heavy chain variable region (VH) comprising the following 3 CDRs: CDR-H1 having a sequence as set forth in SEQ ID NO: 31, CDR-H2 having a sequence as set forth in SEQ ID NO: 34, and CDR-H3 having a sequence as set forth in SEQ ID NO: 20; and/or,
a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
or
(20) a heavy chain variable region (VH) comprising the following 3 CDRs: CDR-H1 having a sequence as set forth in SEQ ID NO: 82, CDR-H2 having a sequence as set forth in SEQ ID NO: 83, and CDR-H3 having a sequence as set forth in SEQ ID NO: 74; and/or,
a light chain variable region (VL) comprising the following 3 CDRs: CDR-L1 having a sequence as set forth in SEQ ID NO: 75, CDR-L2 having a sequence as set forth in SEQ ID NO: 24, and CDR-L3 having a sequence as set forth in SEQ ID NO: 15;
or
(21) a heavy chain variable region (VH) comprising the following 3 CDRs: CDR-H1 having a sequence as set forth in SEQ ID NO: 82, CDR-H2 having a sequence as set forth in SEQ ID NO: 83, and CDR-H3 having a sequence as set forth in SEQ ID NO: 74; and/or,
a light chain variable region (VL) comprising the following 3 CDRs: CDR-L1 having a sequence as set forth in SEQ ID NO: 77, CDR-L2 having a sequence as set forth in SEQ ID NO: 78, and CDR-L3 having a sequence as set forth in SEQ ID NO: 15;
or
(22) a heavy chain variable region (VH) comprising the following 3 CDRs: CDR-H1 having a sequence as set forth in SEQ ID NO: 82, CDR-H2 having a sequence as set forth in SEQ ID NO: 83, and CDR-H3 having a sequence as set forth in SEQ ID NO: 74; and/or,
a light chain variable region (VL) comprising the following 3 CDRs: CDR-L1 having a sequence as set forth in SEQ ID NO: 76, CDR-L2 having a sequence as set forth in SEQ ID NO: 78, and CDR-L3 having a sequence as set forth in SEQ ID NO: 15;
or
(ii) the following heavy chain variable region (VH) and/or light chain variable region (VL), wherein CDRs are defined by the AbM numbering system:
(1) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
a light chain variable region (VL) comprising the following 3 CDRs: CDR-L1 having a sequence as set forth in SEQ ID NO: 48, CDR-L2 having a sequence as set forth in SEQ ID NO: 56, and CDR-L3 having a sequence as set forth in SEQ ID NO: 44;
or
(2) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
a light chain variable region (VL) comprising the following 3 CDRs: CDR-L1 having a sequence as set forth in SEQ ID NO: 48, CDR-L2 having a sequence as set forth in SEQ ID NO: 57, and CDR-L3 having a sequence as set forth in SEQ ID NO: 44;
or
(3) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
a light chain variable region (VL) comprising the following 3 CDRs: CDR-L1 having a sequence as set forth in SEQ ID NO: 49, CDR-L2 having a sequence as set forth in SEQ ID NO: 57, and CDR-L3 having a sequence as set forth in SEQ ID NO: 44;
or
(4) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
a light chain variable region (VL) comprising the following 3 CDRs: CDR-L1 having a sequence as set forth in SEQ ID NO: 50, CDR-L2 having a sequence as set forth in SEQ ID NO: 58, and CDR-L3 having a sequence as set forth in SEQ ID NO: 44;
or
(5) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
a light chain variable region (VL) comprising the following 3 CDRs: CDR-L1 having a sequence as set forth in SEQ ID NO: 51, CDR-L2 having a sequence as set forth in SEQ ID NO: 56, and CDR-L3 having a sequence as set forth in SEQ ID NO: 44;
or
(6) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
a light chain variable region (VL) comprising the following 3 CDRs: CDR-L1 having a sequence as set forth in SEQ ID NO: 52, CDR-L2 having a sequence as set forth in SEQ ID NO: 56, and CDR-L3 having a sequence as set forth in SEQ ID NO: 44;
or
(7) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
a light chain variable region (VL) comprising the following 3 CDRs: CDR-L1 having a sequence as set forth in SEQ ID NO: 53, CDR-L2 having a sequence as set forth in SEQ ID NO: 56, and CDR-L3 having a sequence as set forth in SEQ ID NO: 44;
or
(8) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
a light chain variable region (VL) comprising the following 3 CDRs: CDR-L1 having a sequence as set forth in SEQ ID NO: 54, CDR-L2 having a sequence as set forth in SEQ ID NO: 56, and CDR-L3 having a sequence as set forth in SEQ ID NO: 44;
or
(9) a heavy chain variable region (VH) comprising the following 3 CDRs: CDR-H1 having a sequence as set forth in SEQ ID NO: 45, CDR-H2 having a sequence as set forth in SEQ ID NO: 46, and CDR-H3 having a sequence as set forth in SEQ ID NO: 47; and/or,
a light chain variable region (VL) comprising the following 3 CDRs: CDR-L1 having a sequence as set forth in SEQ ID NO: 55, CDR-L2 having a sequence as set forth in SEQ ID NO: 56, and CDR-L3 having a sequence as set forth in SEQ ID NO: 44;
or
(10) a heavy chain variable region (VH) comprising the following 3 CDRs: CDR-H1 having a sequence as set forth in SEQ ID NO: 16, CDR-H2 having a sequence as set forth in SEQ ID NO: 17, and CDR-H3 having a sequence as set forth in SEQ ID NO: 20; and/or,
a light chain variable region (VL) comprising the following 3 CDRs: CDR-L1 having a sequence as set forth in SEQ ID NO: 21, CDR-L2 having a sequence as set forth in SEQ ID NO: 24, and CDR-L3 having a sequence as set forth in SEQ ID NO: 15;
or
(11) a heavy chain variable region (VH) comprising the following 3 CDRs: CDR-H1 having a sequence as set forth in SEQ ID NO: 16, CDR-H2 having a sequence as set forth in SEQ ID NO: 17, and CDR-H3 having a sequence as set forth in SEQ ID NO: 20; and/or,
a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
or
(12) a heavy chain variable region (VH) comprising the following 3 CDRs: CDR-H1 having a sequence as set forth in SEQ ID NO: 16, CDR-H2 having a sequence as set forth in SEQ ID NO: 17, and CDR-H3 having a sequence as set forth in SEQ ID NO: 20; and/or,
a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
or
(13) a heavy chain variable region (VH) comprising the following 3 CDRs: CDR-H1 having a sequence as set forth in SEQ ID NO: 16, CDR-H2 having a sequence as set forth in SEQ ID NO: 17, and CDR-H3 having a sequence as set forth in SEQ ID NO: 20; and/or,
a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
or
(14) a heavy chain variable region (VH) comprising the following 3 CDRs: CDR-H1 having a sequence as set forth in SEQ ID NO: 16, CDR-H2 having a sequence as set forth in SEQ ID NO: 18, and CDR-H3 having a sequence as set forth in SEQ ID NO: 20; and/or,
a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
or
(15) a heavy chain variable region (VH) comprising the following 3 CDRs: CDR-H1 having a sequence as set forth in SEQ ID NO: 16, CDR-H2 having a sequence as set forth in SEQ ID NO: 18, and CDR-H3 having a sequence as set forth in SEQ ID NO: 20; and/or,
a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
or
(16) a heavy chain variable region (VH) comprising the following 3 CDRs: CDR-H1 having a sequence as set forth in SEQ ID NO: 16, CDR-H2 having a sequence as set forth in SEQ ID NO: 18, and CDR-H3 having a sequence as set forth in SEQ ID NO: 20; and/or,
a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
or
(17) a heavy chain variable region (VH) comprising the following 3 CDRs: CDR-H1 having a sequence as set forth in SEQ ID NO: 16, CDR-H2 having a sequence as set forth in SEQ ID NO: 19, and CDR-H3 having a sequence as set forth in SEQ ID NO: 20; and/or,
a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
or
(18) a heavy chain variable region (VH) comprising the following 3 CDRs: CDR-H1 having a sequence as set forth in SEQ ID NO: 16, CDR-H2 having a sequence as set forth in SEQ ID NO: 19, and CDR-H3 having a sequence as set forth in SEQ ID NO: 20; and/or,
a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
or
(19) A heavy chain variable region (VH) comprising the following 3 CDRs: CDR-H1 having a sequence as set forth in SEQ ID NO: 16, CDR-H2 having a sequence as set forth in SEQ ID NO: 19, and CDR-H3 having a sequence as set forth in SEQ ID NO: 20; and/or,
a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
or
(20) a heavy chain variable region (VH) comprising the following 3 CDRs: CDR-H1 having a sequence as set forth in SEQ ID NO: 72, CDR-H2 having a sequence as set forth in SEQ ID NO: 73, and CDR-H3 having a sequence as set forth in SEQ ID NO: 74; and/or,
a light chain variable region (VL) comprising the following 3 CDRs: CDR-L1 having a sequence as set forth in SEQ ID NO: 75, CDR-L2 having a sequence as set forth in SEQ ID NO: 24, and CDR-L3 having a sequence as set forth in SEQ ID NO: 15;
or
(21) a heavy chain variable region (VH) comprising the following 3 CDRs: CDR-H1 having a sequence as set forth in SEQ ID NO: 72, CDR-H2 having a sequence as set forth in SEQ ID NO: 73, and CDR-H3 having a sequence as set forth in SEQ ID NO: 74; and/or,
a light chain variable region (VL) comprising the following 3 CDRs: CDR-L1 having a sequence as set forth in SEQ ID NO: 77, CDR-L2 having a sequence as set forth in SEQ ID NO: 78, and CDR-L3 having a sequence as set forth in SEQ ID NO: 15;
or
(22) a heavy chain variable region (VH) comprising the following 3 CDRs: CDR-H1 having a sequence as set forth in SEQ ID NO: 72, CDR-H2 having a sequence as set forth in SEQ ID NO: 73, and CDR-H3 having a sequence as set forth in SEQ ID NO: 74; and/or,
a light chain variable region (VL) comprising the following 3 CDRs: CDR-L1 having a sequence as set forth in SEQ ID NO: 76, CDR-L2 having a sequence as set forth in SEQ ID NO: 78, and CDR-L3 having a sequence as set forth in SEQ ID NO: 15;
or
(iii) the following heavy chain variable region (VH) and/or light chain variable region (VL), wherein CDRs are defined by the Kabat numbering system:
(1) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
a light chain variable region (VL) comprising the following 3 CDRs: CDR-L1 having a sequence as set forth in SEQ ID NO: 48, CDR-L2 having a sequence as set forth in SEQ ID NO: 56, and CDR-L3 having a sequence as set forth in SEQ ID NO: 44;
or
(2) a heavy chain variable region (VH) comprising the following 3 CDRs: CDR-H1 having a sequence as set forth in SEQ ID NO: 60, CDR-H2 having a sequence as set forth in SEQ ID NO: 62, and CDR-H3 having a sequence as set forth in SEQ ID NO: 47; and/or,
a light chain variable region (VL) comprising the following 3 CDRs: CDR-L1 having a sequence as set forth in SEQ ID NO: 48, CDR-L2 having a sequence as set forth in SEQ ID NO: 56, and CDR-L3 having a sequence as set forth in SEQ ID NO: 44;
or
(3) a heavy chain variable region (VH) comprising the following 3 CDRs: CDR-H1 having a sequence as set forth in SEQ ID NO: 60, CDR-H2 having a sequence as set forth in SEQ ID NO: 62, and CDR-H3 having a sequence as set forth in SEQ ID NO: 47; and/or,
a light chain variable region (VL) comprising the following 3 CDRs: CDR-L1 having a sequence as set forth in SEQ ID NO: 48, CDR-L2 having a sequence as set forth in SEQ ID NO: 57, and CDR-L3 having a sequence as set forth in SEQ ID NO: 44;
or
(4) a heavy chain variable region (VH) comprising the following 3 CDRs: CDR-H1 having a sequence as set forth in SEQ ID NO: 60, CDR-H2 having a sequence as set forth in SEQ ID NO: 62, and CDR-H3 having a sequence as set forth in SEQ ID NO: 47; and/or,
a light chain variable region (VL) comprising the following 3 CDRs: CDR-L1 having a sequence as set forth in SEQ ID NO: 49, CDR-L2 having a sequence as set forth in SEQ ID NO: 57, and CDR-L3 having a sequence as set forth in SEQ ID NO: 44;
or
(5) a heavy chain variable region (VH) comprising the following 3 CDRs: CDR-H1 having a sequence as set forth in SEQ ID NO: 60, CDR-H2 having a sequence as set forth in SEQ ID NO: 62, and CDR-H3 having a sequence as set forth in SEQ ID NO: 47; and/or,
a light chain variable region (VL) comprising the following 3 CDRs: CDR-L1 having a sequence as set forth in SEQ ID NO: 50, CDR-L2 having a sequence as set forth in SEQ ID NO: 58, and CDR-L3 having a sequence as set forth in SEQ ID NO: 44;
or
(6) a heavy chain variable region (VH) comprising the following 3 CDRs: CDR-H1 having a sequence as set forth in SEQ ID NO: 60, CDR-H2 having a sequence as set forth in SEQ ID NO: 62, and CDR-H3 having a sequence as set forth in SEQ ID NO: 47; and/or,
a light chain variable region (VL) comprising the following 3 CDRs: CDR-L1 having a sequence as set forth in SEQ ID NO: 51, CDR-L2 having a sequence as set forth in SEQ ID NO: 56, and CDR-L3 having a sequence as set forth in SEQ ID NO: 44;
or
(7) a heavy chain variable region (VH) comprising the following 3 CDRs: CDR-H1 having a sequence as set forth in SEQ ID NO: 60, CDR-H2 having a sequence as set forth in SEQ ID NO: 63, and CDR-H3 having a sequence as set forth in SEQ ID NO: 47; and/or,
a light chain variable region (VL) comprising the following 3 CDRs: CDR-L1 having a sequence as set forth in SEQ ID NO: 48, CDR-L2 having a sequence as set forth in SEQ ID NO: 56, and CDR-L3 having a sequence as set forth in SEQ ID NO: 44;
or
(8) A heavy chain variable region (VH) comprising the following 3 CDRs: CDR-H1 having a sequence as set forth in SEQ ID NO: 60, CDR-H2 having a sequence as set forth in SEQ ID NO: 63, and CDR-H3 having a sequence as set forth in SEQ ID NO: 47; and/or,
a light chain variable region (VL) comprising the following 3 CDRs: CDR-L1 having a sequence as set forth in SEQ ID NO: 48, CDR-L2 having a sequence as set forth in SEQ ID NO: 57, and CDR-L3 having a sequence as set forth in SEQ ID NO: 44;
or
(9) a heavy chain variable region (VH) comprising the following 3 CDRs: CDR-H1 having a sequence as set forth in SEQ ID NO: 60, CDR-H2 having a sequence as set forth in SEQ ID NO: 63, and CDR-H3 having a sequence as set forth in SEQ ID NO: 47; and/or,
a light chain variable region (VL) comprising the following 3 CDRs: CDR-L1 having a sequence as set forth in SEQ ID NO: 49, CDR-L2 having a sequence as set forth in SEQ ID NO: 57, and CDR-L3 having a sequence as set forth in SEQ ID NO: 44;
or
(10) a heavy chain variable region (VH) comprising the following 3 CDRs: CDR-H1 having a sequence as set forth in SEQ ID NO: 60, CDR-H2 having a sequence as set forth in SEQ ID NO: 63, and CDR-H3 having a sequence as set forth in SEQ ID NO: 47; and/or,
a light chain variable region (VL) comprising the following 3 CDRs: CDR-L1 having a sequence as set forth in SEQ ID NO: 50, CDR-L2 having a sequence as set forth in SEQ ID NO: 58, and CDR-L3 having a sequence as set forth in SEQ ID NO: 44;
or
(11) a heavy chain variable region (VH) comprising the following 3 CDRs: CDR-H1 having a sequence as set forth in SEQ ID NO: 60, CDR-H2 having a sequence as set forth in SEQ ID NO: 63, and CDR-H3 having a sequence as set forth in SEQ ID NO: 47; and/or,
a light chain variable region (VL) comprising the following 3 CDRs: CDR-L1 having a sequence as set forth in SEQ ID NO: 51, CDR-L2 having a sequence as set forth in SEQ ID NO: 56, and CDR-L3 having a sequence as set forth in SEQ ID NO: 44;
or
(12) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
a light chain variable region (VL) comprising the following 3 CDRs: CDR-L1 having a sequence as set forth in SEQ ID NO: 48, CDR-L2 having a sequence as set forth in SEQ ID NO: 57, and CDR-L3 having a sequence as set forth in SEQ ID NO: 44;
or
(13) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
a light chain variable region (VL) comprising the following 3 CDRs: CDR-L1 having a sequence as set forth in SEQ ID NO: 49, CDR-L2 having a sequence as set forth in SEQ ID NO: 57, and CDR-L3 having a sequence as set forth in SEQ ID NO: 44;
or
(14) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
a light chain variable region (VL) comprising the following 3 CDRs: CDR-L1 having a sequence as set forth in SEQ ID NO: 50, CDR-L2 having a sequence as set forth in SEQ ID NO: 58, and CDR-L3 having a sequence as set forth in SEQ ID NO: 44;
or
(15) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
a light chain variable region (VL) comprising the following 3 CDRs: CDR-L1 having a sequence as set forth in SEQ ID NO: 51, CDR-L2 having a sequence as set forth in SEQ ID NO: 56, and CDR-L3 having a sequence as set forth in SEQ ID NO: 44;
or
(16) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
a light chain variable region (VL) comprising the following 3 CDRs: CDR-L1 having a sequence as set forth in SEQ ID NO: 52, CDR-L2 having a sequence as set forth in SEQ ID NO: 56, and CDR-L3 having a sequence as set forth in SEQ ID NO: 44;
or
(17) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
a light chain variable region (VL) comprising the following 3 CDRs: CDR-L1 having a sequence as set forth in SEQ ID NO: 53, CDR-L2 having a sequence as set forth in SEQ ID NO: 56, and CDR-L3 having a sequence as set forth in SEQ ID NO: 44;
or
(18) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
a light chain variable region (VL) comprising the following 3 CDRs: CDR-L1 having a sequence as set forth in SEQ ID NO: 54, CDR-L2 having a sequence as set forth in SEQ ID NO: 56, and CDR-L3 having a sequence as set forth in SEQ ID NO: 44;
or
(19) a heavy chain variable region (VH) comprising the following 3 CDRs: CDR-H1 having a sequence as set forth in SEQ ID NO: 60, CDR-H2 having a sequence as set forth in SEQ ID NO: 61, and CDR-H3 having a sequence as set forth in SEQ ID NO: 47; and/or,
a light chain variable region (VL) comprising the following 3 CDRs: CDR-L1 having a sequence as set forth in SEQ ID NO: 55, CDR-L2 having a sequence as set forth in SEQ ID NO: 56, and CDR-L3 having a sequence as set forth in SEQ ID NO: 44;
or
(20) a heavy chain variable region (VH) comprising the following 3 CDRs: CDR-H1 having a sequence as set forth in SEQ ID NO: 27, CDR-H2 having a sequence as set forth in SEQ ID NO: 28, and CDR-H3 having a sequence as set forth in SEQ ID NO: 20; and/or,
a light chain variable region (VL) comprising the following 3 CDRs: CDR-L1 having a sequence as set forth in SEQ ID NO: 21, CDR-L2 having a sequence as set forth in SEQ ID NO: 24, and CDR-L3 having a sequence as set forth in SEQ ID NO: 15;
or
(21) a heavy chain variable region (VH) comprising the following 3 CDRs: CDR-H1 having a sequence as set forth in SEQ ID NO: 27, CDR-H2 having a sequence as set forth in SEQ ID NO: 28, and CDR-H3 having a sequence as set forth in SEQ ID NO: 20; and/or,
a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
or
(22) a heavy chain variable region (VH) comprising the following 3 CDRs: CDR-H1 having a sequence as set forth in SEQ ID NO: 27, CDR-H2 having a sequence as set forth in SEQ ID NO: 28, and CDR-H3 having a sequence as set forth in SEQ ID NO: 20; and/or,
a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
or
(23) a heavy chain variable region (VH) comprising the following 3 CDRs: CDR-H1 having a sequence as set forth in SEQ ID NO: 27, CDR-H2 having a sequence as set forth in SEQ ID NO: 28, and CDR-H3 having a sequence as set forth in SEQ ID NO: 20; and/or,
a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
or
(24) a heavy chain variable region (VH) comprising the following 3 CDRs: CDR-H1 having a sequence as set forth in SEQ ID NO: 27, CDR-H2 having a sequence as set forth in SEQ ID NO: 29, and CDR-H3 having a sequence as set forth in SEQ ID NO: 20; and/or,
a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
or
(25) a heavy chain variable region (VH) comprising the following 3 CDRs: CDR-H1 having a sequence as set forth in SEQ ID NO: 27, CDR-H2 having a sequence as set forth in SEQ ID NO: 29, and CDR-H3 having a sequence as set forth in SEQ ID NO: 20; and/or,
a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
or
(26) a heavy chain variable region (VH) comprising the following 3 CDRs: CDR-H1 having a sequence as set forth in SEQ ID NO: 27, CDR-H2 having a sequence as set forth in SEQ ID NO: 29, and CDR-H3 having a sequence as set forth in SEQ ID NO: 20; and/or,
a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
or
(27) a heavy chain variable region (VH) comprising the following 3 CDRs: CDR-H1 having a sequence as set forth in SEQ ID NO: 27, CDR-H2 having a sequence as set forth in SEQ ID NO: 30, and CDR-H3 having a sequence as set forth in SEQ ID NO: 20; and/or,
a light chain variable region (VL) comprising the following 3 CDRs: CDR-L1 having a sequence as set forth in SEQ ID NO: 77, CDR-L2 having a sequence as set forth in SEQ ID NO: 25, and CDR-L3 having a sequence as set forth in SEQ ID NO: 15;
or
(28) a heavy chain variable region (VH) comprising the following 3 CDRs: CDR-H1 having a sequence as set forth in SEQ ID NO: 27, CDR-H2 having a sequence as set forth in SEQ ID NO: 30, and CDR-H3 having a sequence as set forth in SEQ ID NO: 20; and/or,
a light chain variable region (VL) comprising the following 3 CDRs: CDR-L1 having a sequence as set forth in SEQ ID NO: 22, CDR-L2 having a sequence as set forth in SEQ ID NO: 25, and CDR-L3 having a sequence as set forth in SEQ ID NO: 15;
or
(29) a heavy chain variable region (VH) comprising the following 3 CDRs: CDR-H1 having a sequence as set forth in SEQ ID NO: 27, CDR-H2 having a sequence as set forth in SEQ ID NO: 30, and CDR-H3 having a sequence as set forth in SEQ ID NO: 20; and/or,
a light chain variable region (VL) comprising the following 3 CDRs: CDR-L1 having a sequence as set forth in SEQ ID NO: 23, CDR-L2 having a sequence as set forth in SEQ ID NO: 25, and CDR-L3 having a sequence as set forth in SEQ ID NO: 15;
or
(30) a heavy chain variable region (VH) comprising the following 3 CDRs: CDR-H1 having a sequence as set forth in SEQ ID NO: 79, CDR-H2 having a sequence as set forth in SEQ ID NO: 80, and CDR-H3 having a sequence as set forth in SEQ ID NO: 74; and/or,
a light chain variable region (VL) comprising the following 3 CDRs: CDR-L1 having a sequence as set forth in SEQ ID NO: 75, CDR-L2 having a sequence as set forth in SEQ ID NO: 24, and CDR-L3 having a sequence as set forth in SEQ ID NO: 15;
or
(31) a heavy chain variable region (VH) comprising the following 3 CDRs: CDR-H1 having a sequence as set forth in SEQ ID NO: 79, CDR-H2 having a sequence as set forth in SEQ ID NO: 81, and CDR-H3 having a sequence as set forth in SEQ ID NO: 74; and/or,
a light chain variable region (VL) comprising the following 3 CDRs: CDR-L1 having a sequence as set forth in SEQ ID NO: 77, CDR-L2 having a sequence as set forth in SEQ ID NO: 78, and CDR-L3 having a sequence as set forth in SEQ ID NO: 15;
or
(32) a heavy chain variable region (VH) comprising the following 3 CDRs: CDR-H1 having a sequence as set forth in SEQ ID NO: 79, CDR-H2 having a sequence as set forth in SEQ ID NO: 81, and CDR-H3 having a sequence as set forth in SEQ ID NO: 74; and/or,
a light chain variable region (VL) comprising the following 3 CDRs: CDR-L1 having a sequence as set forth in SEQ ID NO: 76, CDR-L2 having a sequence as set forth in SEQ ID NO: 78, and CDR-L3 having a sequence as set forth in SEQ ID NO: 15;
or
(33) a heavy chain variable region (VH) comprising the following 3 CDRs: CDR-H1 having a sequence as set forth in SEQ ID NO: 79, CDR-H2 having a sequence as set forth in SEQ ID NO: 80, and CDR-H3 having a sequence as set forth in SEQ ID NO: 74; and/or,
a light chain variable region (VL) comprising the following 3 CDRs: CDR-L1 having a sequence as set forth in SEQ ID NO: 77, CDR-L2 having a sequence as set forth in SEQ ID NO: 78, and CDR-L3 having a sequence as set forth in SEQ ID NO: 15;
or
(34) a heavy chain variable region (VH) comprising the following 3 CDRs: CDR-H1 having a sequence as set forth in SEQ ID NO: 79, CDR-H2 having a sequence as set forth in SEQ ID NO: 80, and CDR-H3 having a sequence as set forth in SEQ ID NO: 74; and/or,
a light chain variable region (VL) comprising the following 3 CDRs: CDR-L1 having a sequence as set forth in SEQ ID NO: 76, CDR-L2 having a sequence as set forth in SEQ ID NO: 78, and CDR-L3 having a sequence as set forth in SEQ ID NO: 15;
or
(iv) the following heavy chain variable region (VH) and/or light chain variable region (VL), wherein CDRs are defined by the IMGT numbering system:
(1) a heavy chain variable region (VH) comprising the following 3 CDRs: CDR-H1 having a sequence as set forth in SEQ ID NO: 35, CDR-H2 having a sequence as set forth in SEQ ID NO: 36, and CDR-H3 having a sequence as set forth in SEQ ID NO: 37; and/or,
a light chain variable region (VL) comprising the following 3 CDRs: CDR-L1 having a sequence as set forth in SEQ ID NO: 38, CDR-L2 having a sequence as set forth in SEQ ID NO: 43, and CDR-L3 having a sequence as set forth in SEQ ID NO: 44;
or
(2) a heavy chain variable region (VH) comprising the following 3 CDRs: CDR-H1 having a sequence as set forth in SEQ ID NO: 35, CDR-H2 having a sequence as set forth in SEQ ID NO: 36, and CDR-H3 having a sequence as set forth in SEQ ID NO: 37; and/or,
a light chain variable region (VL) comprising the following 3 CDRs: CDR-L1 having a sequence as set forth in SEQ ID NO: 39, CDR-L2 having a sequence as set forth in SEQ ID NO: 43, and CDR-L3 having a sequence as set forth in SEQ ID NO: 44;
or
(3) a heavy chain variable region (VH) comprising the following 3 CDRs: CDR-H1 having a sequence as set forth in SEQ ID NO: 35, CDR-H2 having a sequence as set forth in SEQ ID NO: 36, and CDR-H3 having a sequence as set forth in SEQ ID NO: 37; and/or,
a light chain variable region (VL) comprising the following 3 CDRs: CDR-L1 having a sequence as set forth in SEQ ID NO: 40, CDR-L2 having a sequence as set forth in SEQ ID NO: 43, and CDR-L3 having a sequence as set forth in SEQ ID NO: 44;
or
(4) a heavy chain variable region (VH) comprising the following 3 CDRs: CDR-H1 having a sequence as set forth in SEQ ID NO: 35, CDR-H2 having a sequence as set forth in SEQ ID NO: 36, and CDR-H3 having a sequence as set forth in SEQ ID NO: 37; and/or,
a light chain variable region (VL) comprising the following 3 CDRs: CDR-L1 having a sequence as set forth in SEQ ID NO: 41, CDR-L2 having a sequence as set forth in SEQ ID NO: 43, and CDR-L3 having a sequence as set forth in SEQ ID NO: 44;
or
(5) a heavy chain variable region (VH) comprising the following 3 CDRs: CDR-H1 having a sequence as set forth in SEQ ID NO: 35, CDR-H2 having a sequence as set forth in SEQ ID NO: 36, and CDR-H3 having a sequence as set forth in SEQ ID NO: 37; and/or,
a light chain variable region (VL) comprising the following 3 CDRs: CDR-L1 having a sequence as set forth in SEQ ID NO: 42, CDR-L2 having a sequence as set forth in SEQ ID NO: 43, and CDR-L3 having a sequence as set forth in SEQ ID NO: 44;
or
(6) a heavy chain variable region (VH) comprising the following 3 CDRs: CDR-H1 having a sequence as set forth in SEQ ID NO: 7, CDR-H2 having a sequence as set forth in SEQ ID NO: 8, and CDR-H3 having a sequence as set forth in SEQ ID NO: 11; and/or,
a light chain variable region (VL) comprising the following 3 CDRs: CDR-L1 having a sequence as set forth in SEQ ID NO: 12, CDR-L2 having a sequence as set forth in SEQ ID NO: 14, and CDR-L3 having a sequence as set forth in SEQ ID NO: 15;
or
(7) a heavy chain variable region (VH) comprising the following 3 CDRs: CDR-H1 having a sequence as set forth in SEQ ID NO: 7, CDR-H2 having a sequence as set forth in SEQ ID NO: 8, and CDR-H3 having a sequence as set forth in SEQ ID NO: 11; and/or,
a light chain variable region (VL) comprising the following 3 CDRs: CDR-L1 having a sequence as set forth in SEQ ID NO: 13, CDR-L2 having a sequence as set forth in SEQ ID NO: 14, and CDR-L3 having a sequence as set forth in SEQ ID NO: 15;
or
(8) a heavy chain variable region (VH) comprising the following 3 CDRs: CDR-H1 having a sequence as set forth in SEQ ID NO: 7, CDR-H2 having a sequence as set forth in SEQ ID NO: 9, and CDR-H3 having a sequence as set forth in SEQ ID NO: 11; and/or,
a light chain variable region (VL) comprising the following 3 CDRs: CDR-L1 having a sequence as set forth in SEQ ID NO: 12, CDR-L2 having a sequence as set forth in SEQ ID NO: 14, and CDR-L3 having a sequence as set forth in SEQ ID NO: 15;
or
(9) a heavy chain variable region (VH) comprising the following 3 CDRs: CDR-H1 having a sequence as set forth in SEQ ID NO: 7, CDR-H2 having a sequence as set forth in SEQ ID NO: 9, and CDR-H3 having a sequence as set forth in SEQ ID NO: 11; and/or,
a light chain variable region (VL) comprising the following 3 CDRs: CDR-L1 having a sequence as set forth in SEQ ID NO: 13, CDR-L2 having a sequence as set forth in SEQ ID NO: 14, and CDR-L3 having a sequence as set forth in SEQ ID NO: 15;
or
(10) a heavy chain variable region (VH) comprising the following 3 CDRs: CDR-H1 having a sequence as set forth in SEQ ID NO: 7, CDR-H2 having a sequence as set forth in SEQ ID NO: 10, and CDR-H3 having a sequence as set forth in SEQ ID NO: 11; and/or,
a light chain variable region (VL) comprising the following 3 CDRs: CDR-L1 having a sequence as set forth in SEQ ID NO: 12, CDR-L2 having a sequence as set forth in SEQ ID NO: 14, and CDR-L3 having a sequence as set forth in SEQ ID NO: 15;
or
(11) a heavy chain variable region (VH) comprising the following 3 CDRs: CDR-H1 having a sequence as set forth in SEQ ID NO: 7, CDR-H2 having a sequence as set forth in SEQ ID NO: 10, and CDR-H3 having a sequence as set forth in SEQ ID NO: 11; and/or,
a light chain variable region (VL) comprising the following 3 CDRs: CDR-L1 having a sequence as set forth in SEQ ID NO: 13, CDR-L2 having a sequence as set forth in SEQ ID NO: 14, and CDR-L3 having a sequence as set forth in SEQ ID NO: 15;
or
(12) a heavy chain variable region (VH) comprising the following 3 CDRs: CDR-H1 having a sequence as set forth in SEQ ID NO: 66, CDR-H2 having a sequence as set forth in SEQ ID NO: 67, and CDR-H3 having a sequence as set forth in SEQ ID NO: 68; and/or,
a light chain variable region (VL) comprising the following 3 CDRs: CDR-L1 having a sequence as set forth in SEQ ID NO: 69, CDR-L2 having a sequence as set forth in SEQ ID NO: 71, and CDR-L3 having a sequence as set forth in SEQ ID NO: 15;
or
(13) a heavy chain variable region (VH) comprising the following 3 CDRs: CDR-H1 having a sequence as set forth in SEQ ID NO: 66, CDR-H2 having a sequence as set forth in SEQ ID NO: 67, and CDR-H3 having a sequence as set forth in SEQ ID NO: 68; and/or,
a light chain variable region (VL) comprising the following 3 CDRs: CDR-L1 having a sequence as set forth in SEQ ID NO: 70, CDR-L2 having a sequence as set forth in SEQ ID NO: 71, and CDR-L3 having a sequence as set forth in SEQ ID NO: 15;
or
(v) the following heavy chain variable region (VH) and/or light chain variable region (VL), wherein the heavy chain variable region (VH) and/or light chain variable region (VL) comprises at least one CDR that contains a mutation as compared with the CDR regions described in any one of (i) to (iv), and the mutation is a substitution, deletion or addition of one or several amino acids (e.g., a substitution, deletion or addition of 1, 2 or 3 amino acids); preferably, the substitution is a conservative substitution;
preferably, the antibody or antigen-binding fragment thereof binds to human ROR1.

4. The antibody or antigen-binding fragment thereof according to any one of claims 1-3, wherein the antibody or antigen-binding fragment thereof comprises a heavy chain variable region (VH) and/or light chain variable region (VL) selected from any one of the following groups:
(a) a VH as set forth in any one of SEQ ID NOs: 1 and 102-106, or a variant thereof, and/or, a VL as set forth in any one of SEQ ID NOs: 2 and 107-110, or a variant thereof;
(b) a VH as set forth in any one of SEQ ID NOs: 3 and 84-86, or a variant thereof, and/or, a VL as set forth in any one of SEQ ID NOs: 4 and 87-101, or a variant thereof;
(c) a VH as set forth in any one of SEQ ID NOs: 5 and 111-115, or a variant thereof, and/or, a VL as set forth in any one of SEQ ID NOs: 6, 107 and 116-118, or a variant thereof;
wherein the variant has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletion or addition of one or several amino acids (e.g., a substitution, deletion or addition of 1, 2, 3, 4 or 5 amino acids) as compared to the sequence from which it is derived; preferably, the substitution is a conservative substitution.

5. The antibody or antigen-binding fragment thereof according to any one of claims 1-4, wherein the antibody or antigen-binding fragment thereof comprises a heavy chain variable region (VH) and/or light chain variable region (VL) selected from any one of the following groups:
(1) a VH as set forth in SEQ ID NO: 1 or a variant thereof and a VL as set forth in SEQ ID NO: 2 or a variant thereof;
(2) a VH as set forth in SEQ ID NO: 3 or a variant thereof and a VL as set forth in SEQ ID NO: 4 or a variant thereof;
(3) a VH as set forth in SEQ ID NO: 5 or a variant thereof and a VL as set forth in SEQ ID NO: 6 or a variant thereof;
(4) a VH as set forth in SEQ ID NO: 84 or a variant thereof and a VL as set forth in SEQ ID NO: 87 or a variant thereof;
(5) a VH as set forth in SEQ ID NO: 84 or a variant thereof and a VL as set forth in SEQ ID NO: 88 or a variant thereof;
(6) a VH as set forth in SEQ ID NO: 84 or a variant thereof and a VL as set forth in SEQ ID NO: 89 or a variant thereof;
(7) a VH as set forth in SEQ ID NO: 84 or a variant thereof and a VL as set forth in SEQ ID NO: 90 or a variant thereof;
(8) a VH as set forth in SEQ ID NO: 84 or a variant thereof and a VL as set forth in SEQ ID NO: 91 or a variant thereof;
(9) a VH as set forth in SEQ ID NO: 85 or a variant thereof and a VL as set forth in SEQ ID NO: 87 or a variant thereof;
(10) a VH as set forth in SEQ ID NO: 85 or a variant thereof and a VL as set forth in SEQ ID NO: 88 or a variant thereof;
(11) a VH as set forth in SEQ ID NO: 85 or a variant thereof and a VL as set forth in SEQ ID NO: 92 or a variant thereof;
(12) a VH as set forth in SEQ ID NO: 85 or a variant thereof and a VL as set forth in SEQ ID NO: 90 or a variant thereof;
(13) a VH as set forth in SEQ ID NO: 85 or a variant thereof and a VL as set forth in SEQ ID NO: 91 or a variant thereof;
(14) a VH as set forth in SEQ ID NO: 86 or a variant thereof and a VL as set forth in SEQ ID NO: 87 or a variant thereof;
(15) a VH as set forth in SEQ ID NO: 86 or a variant thereof and a VL as set forth in SEQ ID NO: 88 or a variant thereof;
(16) a VH as set forth in SEQ ID NO: 86 or a variant thereof and a VL as set forth in SEQ ID NO: 89 or a variant thereof;
(17) a VH as set forth in SEQ ID NO: 86 or a variant thereof and a VL as set forth in SEQ ID NO: 90 or a variant thereof;
(18) a VH as set forth in SEQ ID NO: 86 or a variant thereof and a VL as set forth in SEQ ID NO: 91 or a variant thereof;
(19) a VH as set forth in SEQ ID NO: 86 or a variant thereof and a VL as set forth in SEQ ID NO: 92 or a variant thereof;
(20) a VH as set forth in SEQ ID NO: 86 or a variant thereof and a VL as set forth in SEQ ID NO: 93 or a variant thereof;
(21) a VH as set forth in SEQ ID NO: 86 or a variant thereof and a VL as set forth in SEQ ID NO: 94 or a variant thereof;
(22) a VH as set forth in SEQ ID NO: 86 or a variant thereof and a VL as set forth in SEQ ID NO: 95 or a variant thereof;
(23) a VH as set forth in SEQ ID NO: 86 or a variant thereof and a VL as set forth in SEQ ID NO: 96 or a variant thereof;
(24) a VH as set forth in SEQ ID NO: 86 or a variant thereof and a VL as set forth in SEQ ID NO: 97 or a variant thereof;
(25) a VH as set forth in SEQ ID NO: 86 or a variant thereof and a VL as set forth in SEQ ID NO: 98 or a variant thereof;
(26) a VH as set forth in SEQ ID NO: 86 or a variant thereof and a VL as set forth in SEQ ID NO: 99 or a variant thereof;
(27) a VH as set forth in SEQ ID NO: 86 or a variant thereof and a VL as set forth in SEQ ID NO: 100 or a variant thereof;
(28) a VH as set forth in SEQ ID NO: 86 or a variant thereof and a VL as set forth in SEQ ID NO: 101 or a variant thereof;
(29) a VH as set forth in SEQ ID NO: 102 or a variant thereof and a VL as set forth in SEQ ID NO: 107 or a variant thereof;
(30) a VH as set forth in SEQ ID NO: 102 or a variant thereof and a VL as set forth in SEQ ID NO: 108 or a variant thereof;
(31) a VH as set forth in SEQ ID NO: 102 or a variant thereof and a VL as set forth in SEQ ID NO: 109 or a variant thereof;
(32) a VH as set forth in SEQ ID NO: 102 or a variant thereof and a VL as set forth in SEQ ID NO: 110 or a variant thereof;
(33) a VH as set forth in SEQ ID NO: 103 or a variant thereof and a VL as set forth in SEQ ID NO: 107 or a variant thereof;
(34) a VH as set forth in SEQ ID NO: 103 or a variant thereof and a VL as set forth in SEQ ID NO: 108 or a variant thereof;
(35) a VH as set forth in SEQ ID NO: 103 or a variant thereof and a VL as set forth in SEQ ID NO: 109 or a variant thereof;
(36) a VH as set forth in SEQ ID NO: 103 or a variant thereof and a VL as set forth in SEQ ID NO: 110 or a variant thereof;
(37) a VH as set forth in SEQ ID NO: 104 or a variant thereof and a VL as set forth in SEQ ID NO: 107 or a variant thereof;
(38) a VH as set forth in SEQ ID NO: 104 or a variant thereof and a VL as set forth in SEQ ID NO: 108 or a variant thereof;
(39) a VH as set forth in SEQ ID NO: 104 or a variant thereof and a VL as set forth in SEQ ID NO: 109 or a variant thereof;
(40) a VH as set forth in SEQ ID NO: 104 or a variant thereof and a VL as set forth in SEQ ID NO: 110 or a variant thereof;
(41) a VH as set forth in SEQ ID NO: 105 or a variant thereof and a VL as set forth in SEQ ID NO: 107 or a variant thereof;
(42) a VH as set forth in SEQ ID NO: 105 or a variant thereof and a VL as set forth in SEQ ID NO: 108 or a variant thereof;
(43) a VH as set forth in SEQ ID NO: 105 or a variant thereof and a VL as set forth in SEQ ID NO: 109 or a variant thereof;
(44) a VH as set forth in SEQ ID NO: 105 or a variant thereof and a VL as set forth in SEQ ID NO: 110 or a variant thereof;
(45) a VH as set forth in SEQ ID NO: 106 or a variant thereof and a VL as set forth in SEQ ID NO: 107 or a variant thereof;
(46) a VH as set forth in SEQ ID NO: 106 or a variant thereof and a VL as set forth in SEQ ID NO: 108 or a variant thereof;
(47) a VH as set forth in SEQ ID NO: 106 or a variant thereof and a VL as set forth in SEQ ID NO: 109 or a variant thereof;
(48) a VH as set forth in SEQ ID NO: 106 or a variant thereof and a VL as set forth in SEQ ID NO: 110 or a variant thereof;
(49) a VH as set forth in SEQ ID NO: 111 or a variant thereof and a VL as set forth in SEQ ID NO: 107 or a variant thereof;
(50) a VH as set forth in SEQ ID NO: 111 or a variant thereof and a VL as set forth in SEQ ID NO: 116 or a variant thereof;
(51) a VH as set forth in SEQ ID NO: 111 or a variant thereof and a VL as set forth in SEQ ID NO: 117 or a variant thereof;
(52) a VH as set forth in SEQ ID NO: 111 or a variant thereof and a VL as set forth in SEQ ID NO: 118 or a variant thereof;
(53) a VH as set forth in SEQ ID NO: 112 or a variant thereof and a VL as set forth in SEQ ID NO: 107 or a variant thereof;
(54) a VH as set forth in SEQ ID NO: 112 or a variant thereof and a VL as set forth in SEQ ID NO: 116 or a variant thereof;
(55) a VH as set forth in SEQ ID NO: 112 or a variant thereof and a VL as set forth in SEQ ID NO: 117 or a variant thereof;
(56) a VH as set forth in SEQ ID NO: 112 or a variant thereof and a VL as set forth in SEQ ID NO: 118 or a variant thereof;
(57) a VH as set forth in SEQ ID NO: 113 or a variant thereof and a VL as set forth in SEQ ID NO: 107 or a variant thereof;
(58) a VH as set forth in SEQ ID NO: 113 or a variant thereof and a VL as set forth in SEQ ID NO: 116 or a variant thereof;
(59) a VH as set forth in SEQ ID NO: 113 or a variant thereof and a VL as set forth in SEQ ID NO: 117 or a variant thereof;
(60) a VH as set forth in SEQ ID NO: 113 or a variant thereof and a VL as set forth in SEQ ID NO: 118 or a variant thereof;
(61) a VH as set forth in SEQ ID NO: 114 or a variant thereof and a VL as set forth in SEQ ID NO: 107 or a variant thereof;
(62) a VH as set forth in SEQ ID NO: 114 or a variant thereof and a VL as set forth in SEQ ID NO: 116 or a variant thereof;
(63) a VH as set forth in SEQ ID NO: 114 or a variant thereof and a VL as set forth in SEQ ID NO: 117 or a variant thereof;
(64) a VH as set forth in SEQ ID NO: 114 or a variant thereof and a VL as set forth in SEQ ID NO: 118 or a variant thereof;
(65) a VH as set forth in SEQ ID NO: 115 or a variant thereof and a VL as set forth in SEQ ID NO: 107 or a variant thereof;
(66) a VH as set forth in SEQ ID NO: 115 or a variant thereof and a VL as set forth in SEQ ID NO: 116 or a variant thereof;
(67) a VH as set forth in SEQ ID NO: 115 or a variant thereof and a VL as set forth in SEQ ID NO: 117 or a variant thereof;
(68) a VH as set forth in SEQ ID NO: 115 or a variant thereof and a VL as set forth in SEQ ID NO: 118 or a variant thereof;
wherein the variant has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletion, or addition of one or several amino acids (e.g., a substitution, deletion or addition of 1, 2, 3, 4 or 5 amino acids) as compared to the sequence from which it is derived; preferably, the substitution is a conservative substitution.

6. The antibody or antigen-binding fragment thereof according to any one of claims 1-5, wherein the antibody or antigen-binding fragment thereof is a murine antibody, a chimeric antibody or a humanized antibody.

7. The antibody or antigen-binding fragment thereof according to any one of claims 1-6, wherein the antibody or antigen-binding fragment thereof further comprises:
(a) a human immunoglobulin heavy chain constant region (CH) or a variant thereof, wherein the variant has a substitution, deletion or addition of one or more amino acids (e.g., a substitution, deletion or addition of up to 20, up to 15, up to 10, or up to 5 amino acids; for example, a substitution, deletion or addition of 1, 2, 3, 4 or 5 amino acids) as compared to the wild-type sequence from which it is derived; and
(b) a human immunoglobulin light chain constant region (CL) or a variant thereof, wherein the variant has a substitution, deletion or addition of one or more amino acids (e.g., a substitution, deletion or addition of up to 20, up to 15, up to 10, or up to 5 amino acids; for example, a substitution, deletion or addition of 1, 2, 3, 4 or 5 amino acids) as compared to the wild-type sequence from which it is derived;
preferably, the heavy chain constant region is an IgG heavy chain constant region, such as an IgGl, IgG2, IgG3 or IgG4 heavy chain constant region;
preferably, the antibody or antigen-binding fragment thereof comprises a heavy chain constant region selected from the group consisting of:
(1) a human IgG1 heavy chain constant region;
(2) a human IgG4 heavy chain constant region;
preferably, the antibody or antigen-binding fragment thereof comprises a heavy chain constant region (CH) as set forth in SEQ ID NO: 119 or a variant thereof, wherein the variant has a conservative substitution of at most 20 amino acids (e.g., a conservative substitution of at most 15, at most 10, or at most 5 amino acids; for example, a conservative substitution of 1, 2, 3, 4 or 5 amino acids) as compared to SEQ ID NO: 119;
preferably, the light chain constant region is a κ light chain constant region;
preferably, the antibody or antigen-binding fragment thereof comprises a light chain constant region (CL) as set forth in SEQ ID NO: 120 or a variant thereof, wherein the variant has a conservative substitution of at most 20 amino acids (e.g., a conservative substitution of at most 15, at most 10, or at most 5 amino acids; for example, a conservative substitution of 1, 2, 3, 4 or 5 amino acids) as compared to SEQ ID NO: 120;
more preferably, the antibody or antigen-binding fragment thereof comprises a heavy chain constant region (CH) as set forth in SEQ ID NO: 119 and a light chain constant region (CL) as set forth in SEQ ID NO: 120.

8. The antibody or antigen-binding fragment thereof according to any one of claims 1-7, wherein the antibody comprises any one of the following groups:
(1) a heavy chain comprising a VH as set forth in SEQ ID NO: 1 and a heavy chain constant region (CH) as set forth in SEQ ID NO: 119, and, a light chain comprising a VL as set forth in SEQ ID NO: 2 and a light chain constant region (CL) as set forth in SEQ ID NO: 120;
(2) a heavy chain comprising a VH as set forth in SEQ ID NO: 3 and a heavy chain constant region (CH) as set forth in SEQ ID NO: 119, and, a light chain comprising a VL as set forth in SEQ ID NO: 4 and a light chain constant region (CL) as set forth in SEQ ID NO: 120;
(3) a heavy chain comprising a VH as set forth in SEQ ID NO: 5 and a heavy chain constant region (CH) as set forth in SEQ ID NO: 119, and, a light chain comprising a VL as set forth in SEQ ID NO: 6 and a light chain constant region (CL) as set forth in SEQ ID NO: 120;
(4) a heavy chain comprising a VH as set forth in SEQ ID NO: 84 and a heavy chain constant region (CH) as set forth in SEQ ID NO: 119, and, a light chain comprising a VL as set forth in SEQ ID NO: 87 and a light chain constant region (CL) as set forth in SEQ ID NO: 120;
(5) a heavy chain comprising a VH as set forth in SEQ ID NO: 84 and a heavy chain constant region (CH) as set forth in SEQ ID NO: 119, and, a light chain comprising a VL as set forth in SEQ ID NO: 88 and a light chain constant region (CL) as set forth in SEQ ID NO: 120;
(6) a heavy chain comprising a VH as set forth in SEQ ID NO: 84 and a heavy chain constant region (CH) as set forth in SEQ ID NO: 119, and, a light chain comprising a VL as set forth in SEQ ID NO: 89 and a light chain constant region (CL) as set forth in SEQ ID NO: 120;
(7) a heavy chain comprising a VH as set forth in SEQ ID NO: 84 and a heavy chain constant region (CH) as set forth in SEQ ID NO: 119, and, a light chain comprising a VL as set forth in SEQ ID NO: 90 and a light chain constant region (CL) as set forth in SEQ ID NO: 120;
(8) a heavy chain comprising a VH as set forth in SEQ ID NO: 84 and a heavy chain constant region (CH) as set forth in SEQ ID NO: 119, and, a light chain comprising a VL as set forth in SEQ ID NO: 91 and a light chain constant region (CL) as set forth in SEQ ID NO: 120;
(9) a heavy chain comprising a VH as set forth in SEQ ID NO: 85 and a heavy chain constant region (CH) as set forth in SEQ ID NO: 119, and, a light chain comprising a VL as set forth in SEQ ID NO: 87 and a light chain constant region (CL) as set forth in SEQ ID NO: 120;
(10) a heavy chain comprising a VH as set forth in SEQ ID NO: 85 and a heavy chain constant region (CH) as set forth in SEQ ID NO: 119, and, a light chain comprising a VL as set forth in SEQ ID NO: 88 and a light chain constant region (CL) as set forth in SEQ ID NO: 120;
(11) a heavy chain comprising a VH as set forth in SEQ ID NO: 85 and a heavy chain constant region (CH) as set forth in SEQ ID NO: 119, and, a light chain comprising a VL as set forth in SEQ ID NO: 92 and a light chain constant region (CL) as set forth in SEQ ID NO: 120;
(12) a heavy chain comprising a VH as set forth in SEQ ID NO: 85 and a heavy chain constant region (CH) as set forth in SEQ ID NO: 119, and, a light chain comprising a VL as set forth in SEQ ID NO: 90 and a light chain constant region (CL) as set forth in SEQ ID NO: 120;
(13) a heavy chain comprising a VH as set forth in SEQ ID NO: 85 and a heavy chain constant region (CH) as set forth in SEQ ID NO: 119, and, a light chain comprising a VL as set forth in SEQ ID NO: 91 and a light chain constant region (CL) as set forth in SEQ ID NO: 120;
(14) a heavy chain comprising a VH as set forth in SEQ ID NO: 86 and a heavy chain constant region (CH) as set forth in SEQ ID NO: 119, and, a light chain comprising a VL as set forth in SEQ ID NO: 87 and a light chain constant region (CL) as set forth in SEQ ID NO: 120;
(15) a heavy chain comprising a VH as set forth in SEQ ID NO: 86 and a heavy chain constant region (CH) as set forth in SEQ ID NO: 119, and, a light chain comprising a VL as set forth in SEQ ID NO: 88 and a light chain constant region (CL) as set forth in SEQ ID NO: 120;
(16) a heavy chain comprising a VH as set forth in SEQ ID NO: 86 and a heavy chain constant region (CH) as set forth in SEQ ID NO: 119, and, a light chain comprising a VL as set forth in SEQ ID NO: 89 and a light chain constant region (CL) as set forth in SEQ ID NO: 120;
(17) a heavy chain comprising a VH as set forth in SEQ ID NO: 86 and a heavy chain constant region (CH) as set forth in SEQ ID NO: 119, and, a light chain comprising a VL as set forth in SEQ ID NO: 90 and a light chain constant region (CL) as set forth in SEQ ID NO: 120;
(18) a heavy chain comprising a VH as set forth in SEQ ID NO: 86 and a heavy chain constant region (CH) as set forth in SEQ ID NO: 119, and, a light chain comprising a VL as set forth in SEQ ID NO: 91 and a light chain constant region (CL) as set forth in SEQ ID NO: 120;
(19) a heavy chain comprising a VH as set forth in SEQ ID NO: 86 and a heavy chain constant region (CH) as set forth in SEQ ID NO: 119, and, a light chain comprising a VL as set forth in SEQ ID NO: 92 and a light chain constant region (CL) as set forth in SEQ ID NO: 120;
(20) a heavy chain comprising a VH as set forth in SEQ ID NO: 86 and a heavy chain constant region (CH) as set forth in SEQ ID NO: 119, and, a light chain comprising a VL as set forth in SEQ ID NO: 93 and a light chain constant region (CL) as set forth in SEQ ID NO: 120;
(21) a heavy chain comprising a VH as set forth in SEQ ID NO: 86 and a heavy chain constant region (CH) as set forth in SEQ ID NO: 119, and, a light chain comprising a VL as set forth in SEQ ID NO: 94 and a light chain constant region (CL) as set forth in SEQ ID NO: 120;
(22) a heavy chain comprising a VH as set forth in SEQ ID NO: 86 and a heavy chain constant region (CH) as set forth in SEQ ID NO: 119, and, a light chain comprising a VL as set forth in SEQ ID NO: 95 and a light chain constant region (CL) as set forth in SEQ ID NO: 120;
(23) a heavy chain comprising a VH as set forth in SEQ ID NO: 86 and a heavy chain constant region (CH) as set forth in SEQ ID NO: 119, and, a light chain comprising a VL as set forth in SEQ ID NO: 96 and a light chain constant region (CL) as set forth in SEQ ID NO: 120;
(24) a heavy chain comprising a VH as set forth in SEQ ID NO: 86 and a heavy chain constant region (CH) as set forth in SEQ ID NO: 119, and, a light chain comprising a VL as set forth in SEQ ID NO: 97 and a light chain constant region (CL) as set forth in SEQ ID NO: 120;
(25) a heavy chain comprising a VH as set forth in SEQ ID NO: 86 and a heavy chain constant region (CH) as set forth in SEQ ID NO: 119, and, a light chain comprising a VL as set forth in SEQ ID NO: 98 and a light chain constant region (CL) as set forth in SEQ ID NO: 120;
(26) a heavy chain comprising a VH as set forth in SEQ ID NO: 86 and a heavy chain constant region (CH) as set forth in SEQ ID NO: 119, and, a light chain comprising a VL as set forth in SEQ ID NO: 99 and a light chain constant region (CL) as set forth in SEQ ID NO: 120;
(27) a heavy chain comprising a VH as set forth in SEQ ID NO: 86 and a heavy chain constant region (CH) as set forth in SEQ ID NO: 119, and, a light chain comprising a VL as set forth in SEQ ID NO: 100 and a light chain constant region (CL) as set forth in SEQ ID NO: 120;
(28) a heavy chain comprising a VH as set forth in SEQ ID NO: 86 and a heavy chain constant region (CH) as set forth in SEQ ID NO: 119, and, a light chain comprising a VL as set forth in SEQ ID NO: 101 and a light chain constant region (CL) as set forth in SEQ ID NO: 120;
(29) a heavy chain comprising a VH as set forth in SEQ ID NO: 102 and a heavy chain constant region (CH) as set forth in SEQ ID NO: 119, and, a light chain comprising a VL as set forth in SEQ ID NO: 107 and a light chain constant region (CL) as set forth in SEQ ID NO: 120;
(30) a heavy chain comprising a VH as set forth in SEQ ID NO: 102 and a heavy chain constant region (CH) as set forth in SEQ ID NO: 119, and, a light chain comprising a VL as set forth in SEQ ID NO: 108 and a light chain constant region (CL) as set forth in SEQ ID NO: 120;
(31) a heavy chain comprising a VH as set forth in SEQ ID NO: 102 and a heavy chain constant region (CH) as set forth in SEQ ID NO: 119, and, a light chain comprising a VL as set forth in SEQ ID NO: 109 and a light chain constant region (CL) as set forth in SEQ ID NO: 120;
(32) a heavy chain comprising a VH as set forth in SEQ ID NO: 102 and a heavy chain constant region (CH) as set forth in SEQ ID NO: 119, and, a light chain comprising a VL as set forth in SEQ ID NO: 110 and a light chain constant region (CL) as set forth in SEQ ID NO: 120;
(33) a heavy chain comprising a VH as set forth in SEQ ID NO: 103 and a heavy chain constant region (CH) as set forth in SEQ ID NO: 119, and, a light chain comprising a VL as set forth in SEQ ID NO: 107 and a light chain constant region (CL) as set forth in SEQ ID NO: 120;
(34) a heavy chain comprising a VH as set forth in SEQ ID NO: 103 and a heavy chain constant region (CH) as set forth in SEQ ID NO: 119, and, a light chain comprising a VL as set forth in SEQ ID NO: 108 and a light chain constant region (CL) as set forth in SEQ ID NO: 120;
(35) a heavy chain comprising a VH as set forth in SEQ ID NO: 103 and a heavy chain constant region (CH) as set forth in SEQ ID NO: 119, and, a light chain comprising a VL as set forth in SEQ ID NO: 109 and a light chain constant region (CL) as set forth in SEQ ID NO: 120;
(36) a heavy chain comprising a VH as set forth in SEQ ID NO: 103 and a heavy chain constant region (CH) as set forth in SEQ ID NO: 119, and, a light chain comprising a VL as set forth in SEQ ID NO: 110 and a light chain constant region (CL) as set forth in SEQ ID NO: 120;
(37) a heavy chain comprising a VH as set forth in SEQ ID NO: 104 and a heavy chain constant region (CH) as set forth in SEQ ID NO: 119, and, a light chain comprising a VL as set forth in SEQ ID NO: 107 and a light chain constant region (CL) as set forth in SEQ ID NO: 120;
(38) a heavy chain comprising a VH as set forth in SEQ ID NO: 104 and a heavy chain constant region (CH) as set forth in SEQ ID NO: 119, and, a light chain comprising a VL as set forth in SEQ ID NO: 108 and a light chain constant region (CL) as set forth in SEQ ID NO: 120;
(39) a heavy chain comprising a VH as set forth in SEQ ID NO: 104 and a heavy chain constant region (CH) as set forth in SEQ ID NO: 119, and, a light chain comprising a VL as set forth in SEQ ID NO: 109 and a light chain constant region (CL) as set forth in SEQ ID NO: 120;
(40) a heavy chain comprising a VH as set forth in SEQ ID NO: 104 and a heavy chain constant region (CH) as set forth in SEQ ID NO: 119, and, a light chain comprising a VL as set forth in SEQ ID NO: 110 and a light chain constant region (CL) as set forth in SEQ ID NO: 120;
(41) a heavy chain comprising a VH as set forth in SEQ ID NO: 105 and a heavy chain constant region (CH) as set forth in SEQ ID NO: 119, and, a light chain comprising a VL as set forth in SEQ ID NO: 107 and a light chain constant region (CL) as set forth in SEQ ID NO: 120;
(42) a heavy chain comprising a VH as set forth in SEQ ID NO: 105 and a heavy chain constant region (CH) as set forth in SEQ ID NO: 119, and, a light chain comprising a VL as set forth in SEQ ID NO: 108 and a light chain constant region (CL) as set forth in SEQ ID NO: 120;
(43) a heavy chain comprising a VH as set forth in SEQ ID NO: 105 and a heavy chain constant region (CH) as set forth in SEQ ID NO: 119, and, a light chain comprising a VL as set forth in SEQ ID NO: 109 and a light chain constant region (CL) as set forth in SEQ ID NO: 120;
(44) a heavy chain comprising a VH as set forth in SEQ ID NO: 105 and a heavy chain constant region (CH) as set forth in SEQ ID NO: 119, and, a light chain comprising a VL as set forth in SEQ ID NO: 110 and a light chain constant region (CL) as set forth in SEQ ID NO: 120;
(45) a heavy chain comprising a VH as set forth in SEQ ID NO: 106 and a heavy chain constant region (CH) as set forth in SEQ ID NO: 119, and, a light chain comprising a VL as set forth in SEQ ID NO: 107 and a light chain constant region (CL) as set forth in SEQ ID NO: 120;
(46) a heavy chain comprising a VH as set forth in SEQ ID NO: 106 and a heavy chain constant region (CH) as set forth in SEQ ID NO: 119, and, a light chain comprising a VL as set forth in SEQ ID NO: 108 and a light chain constant region (CL) as set forth in SEQ ID NO: 120;
(47) a heavy chain comprising a VH as set forth in SEQ ID NO: 106 and a heavy chain constant region (CH) as set forth in SEQ ID NO: 119, and, a light chain comprising a VL as set forth in SEQ ID NO: 109 and a light chain constant region (CL) as set forth in SEQ ID NO: 120;
(48) a heavy chain comprising a VH as set forth in SEQ ID NO: 106 and a heavy chain constant region (CH) as set forth in SEQ ID NO: 119, and, a light chain comprising a VL as set forth in SEQ ID NO: 110 and a light chain constant region (CL) as set forth in SEQ ID NO: 120;
(49) a heavy chain comprising a VH as set forth in SEQ ID NO: 111 and a heavy chain constant region (CH) as set forth in SEQ ID NO: 119, and, a light chain comprising a VL as set forth in SEQ ID NO: 107 and a light chain constant region (CL) as set forth in SEQ ID NO: 120;
(50) a heavy chain comprising a VH as set forth in SEQ ID NO: 111 and a heavy chain constant region (CH) as set forth in SEQ ID NO: 119, and, a light chain comprising a VL as set forth in SEQ ID NO: 116 and a light chain constant region (CL) as set forth in SEQ ID NO: 120;
(51) a heavy chain comprising a VH as set forth in SEQ ID NO: 111 and a heavy chain constant region (CH) as set forth in SEQ ID NO: 119, and, a light chain comprising a VL as set forth in SEQ ID NO: 117 and a light chain constant region (CL) as set forth in SEQ ID NO: 120;
(52) a heavy chain comprising a VH as set forth in SEQ ID NO: 111 and a heavy chain constant region (CH) as set forth in SEQ ID NO: 119, and, a light chain comprising a VL as set forth in SEQ ID NO: 118 and a light chain constant region (CL) as set forth in SEQ ID NO: 120;
(53) a heavy chain comprising a VH as set forth in SEQ ID NO: 112 and a heavy chain constant region (CH) as set forth in SEQ ID NO: 119, and, a light chain comprising a VL as set forth in SEQ ID NO: 107 and a light chain constant region (CL) as set forth in SEQ ID NO: 120;
(54) a heavy chain comprising a VH as set forth in SEQ ID NO: 112 and a heavy chain constant region (CH) as set forth in SEQ ID NO: 119, and, a light chain comprising a VL as set forth in SEQ ID NO: 116 and a light chain constant region (CL) as set forth in SEQ ID NO: 120;
(55) a heavy chain comprising a VH as set forth in SEQ ID NO: 112 and a heavy chain constant region (CH) as set forth in SEQ ID NO: 119, and, a light chain comprising a VL as set forth in SEQ ID NO: 117 and a light chain constant region (CL) as set forth in SEQ ID NO: 120;
(56) a heavy chain comprising a VH as set forth in SEQ ID NO: 112 and a heavy chain constant region (CH) as set forth in SEQ ID NO: 119, and, a light chain comprising a VL as set forth in SEQ ID NO: 118 and a light chain constant region (CL) as set forth in SEQ ID NO: 120;
(57) a heavy chain comprising a VH as set forth in SEQ ID NO: 113 and a heavy chain constant region (CH) as set forth in SEQ ID NO: 119, and, a light chain comprising a VL as set forth in SEQ ID NO: 107 and a light chain constant region (CL) as set forth in SEQ ID NO: 120;
(58) a heavy chain comprising a VH as set forth in SEQ ID NO: 113 and a heavy chain constant region (CH) as set forth in SEQ ID NO: 119, and, a light chain comprising a VL as set forth in SEQ ID NO: 116 and a light chain constant region (CL) as set forth in SEQ ID NO: 120;
(59) a heavy chain comprising a VH as set forth in SEQ ID NO: 113 and a heavy chain constant region (CH) as set forth in SEQ ID NO: 119, and, a light chain comprising a VL as set forth in SEQ ID NO: 117 and a light chain constant region (CL) as set forth in SEQ ID NO: 120;
(60) a heavy chain comprising a VH as set forth in SEQ ID NO: 113 and a heavy chain constant region (CH) as set forth in SEQ ID NO: 119, and, a light chain comprising a VL as set forth in SEQ ID NO: 118 and a light chain constant region (CL) as set forth in SEQ ID NO: 120;
(61) a heavy chain comprising a VH as set forth in SEQ ID NO: 114 and a heavy chain constant region (CH) as set forth in SEQ ID NO: 119, and, a light chain comprising a VL as set forth in SEQ ID NO: 107 and a light chain constant region (CL) as set forth in SEQ ID NO: 120;
(62) a heavy chain comprising a VH as set forth in SEQ ID NO: 114 and a heavy chain constant region (CH) as set forth in SEQ ID NO: 119, and, a light chain comprising a VL as set forth in SEQ ID NO: 116 and a light chain constant region (CL) as set forth in SEQ ID NO: 120;
(63) a heavy chain comprising a VH as set forth in SEQ ID NO: 114 and a heavy chain constant region (CH) as set forth in SEQ ID NO: 119, and, a light chain comprising a VL as set forth in SEQ ID NO: 117 and a light chain constant region (CL) as set forth in SEQ ID NO: 120;
(64) a heavy chain comprising a VH as set forth in SEQ ID NO: 114 and a heavy chain constant region (CH) as set forth in SEQ ID NO: 119, and, a light chain comprising a VL as set forth in SEQ ID NO: 118 and a light chain constant region (CL) as set forth in SEQ ID NO: 120;
(65) a heavy chain comprising a VH as set forth in SEQ ID NO: 115 and a heavy chain constant region (CH) as set forth in SEQ ID NO: 119, and, a light chain comprising a VL as set forth in SEQ ID NO: 107 and a light chain constant region (CL) as set forth in SEQ ID NO: 120;
(66) a heavy chain comprising a VH as set forth in SEQ ID NO: 115 and a heavy chain constant region (CH) as set forth in SEQ ID NO: 119, and, a light chain comprising a VL as set forth in SEQ ID NO: 116 and a light chain constant region (CL) as set forth in SEQ ID NO: 120;
(67) a heavy chain comprising a VH as set forth in SEQ ID NO: 115 and a heavy chain constant region (CH) as set forth in SEQ ID NO: 119, and, a light chain comprising a VL as set forth in SEQ ID NO: 117 and a light chain constant region (CL) as set forth in SEQ ID NO: 120;
(68) a heavy chain comprising a VH as set forth in SEQ ID NO: 115 and a heavy chain constant region (CH) as set forth in SEQ ID NO: 119, and, a light chain comprising a VL as set forth in SEQ ID NO: 118 and a light chain constant region (CL) as set forth in SEQ ID NO: 120.

9. The antibody or antigen-binding fragment thereof according to any one of claims 1-8, wherein the antibody or antigen-binding fragment thereof is selected from the group consisting of ScFv, Fab, Fab', (Fab')₂, Fv fragment, disulfide bond-linked Fv(dsFv), diabody, bispecific antibody and multispecific antibody.

10. The antibody or antigen-binding fragment thereof according to any one of claims 1-9, wherein the antibody or antigen-binding fragment thereof bears a label; preferably, the antibody or antigen-binding fragment thereof bears a detectable label, such as enzyme (e.g., horseradish peroxidase), radionuclide, fluorescent dye, luminescent substance (e.g., chemiluminescent substance), or biotin.

11. The antibody or antigen-binding fragment thereof according to any one of claims 1-10, wherein the antibody or antigen-binding fragment thereof has a characteristic selected from the group consisting of:
binding to ROR1 (e.g., human ROR1) with an EC50 of less than about 500 nM, for example, less than about 100 nM, 10 nM, 1 nM, 0.9 nM, 0.8 nM, 0.7 nM, 0.6 nM, 0.5 nM, 0.4 nM, 0.3 nM, 0.2 nM, 0.1 nM or lower; preferably, the EC50 is determined by flow cytometry or cell-based competitive ELISA; and/or
binding to ROR1 (e.g., human ROR1) with a K_{D} of less than about 100 nM, for example, less than about 50 nM, 40 nM, 30 nM, 20 nM, 10 nM, 1 nM, 0.1 nM or lower; preferably, the K_{D} is determined by biolayer interferometry (BLI);
preferably, the antibody or antigen-binding fragment thereof does not bind to ROR2 (e.g., human ROR2);
preferably, the antibody or antigen-binding fragment thereof has an ADCC and/or CDC activity;
more preferably, the antibody or antigen-binding fragment thereof has an enhanced ADCC and/or CDC activity.

12. An isolated nucleic acid molecule, which encodes the antibody or antigen-binding fragment thereof according to any one of claims 1-11, a heavy chain and/or light chain thereof, or a heavy chain variable region and/or light chain variable region thereof; preferably, the encoded antibody or antigen-binding fragment thereof, heavy chain and/or light chain thereof, or heavy chain variable region and/or light chain variable region thereof comprises the heavy chain variable region and/or light chain variable region as defined in any one of claims 1-5.

13. The isolated nucleic acid molecule according to claim 12, which comprises a nucleic acid molecule encoding an antibody heavy chain variable region, and/or a nucleic acid molecule encoding an antibody light chain variable region, wherein, the nucleic acid molecule encoding the antibody heavy chain variable region has a sequence selected from the group consisting of:
(a) a nucleotide sequence as set forth in SEQ ID NOs: 121, 123, 125, 127-129, 145-149, and 154-158, or
(b) a sequence substantially identical to the nucleotide sequence described in (a) (e.g., a sequence having a sequence identity of at least about 85%, 90%, 95%, 99% or higher as compared to the nucleotide sequence described in (a), or a sequence having a substitution of one or more nucleotides), or
(c) a sequence that differs by no more than 3, 6, 15, 30 or 45 nucleotides from the nucleotide sequence described in (a), and/or,
the nucleic acid molecule encoding the antibody light chain variable region has a sequence selected from the group consisting of:
(d) a nucleotide sequence as set forth in SEQ ID NOs: 122, 124, 126, 130-144, 150-153, and 159-161, or
(e) a sequence substantially identical to the nucleotide sequence described in (d) (e.g., a sequence having a sequence identity of at least about 85%, 90%, 95%, 99% or higher as compared to the nucleotide sequence described in (d), or a sequence having a substitution of one or more nucleotides), or
(f) a sequence that differs by no more than 3, 6, 15, 30 or 45 nucleotides from the nucleotide sequence described in (d).

14. The isolated nucleic acid molecule according to claim 12 or 13, which comprises a nucleic acid molecule encoding an antibody heavy chain constant region, and/or a nucleic acid molecule encoding an antibody light chain constant region, wherein,
the nucleic acid molecule encoding the antibody heavy chain constant region has a sequence selected from the group consisting of:
(a) a nucleotide sequence as set forth in SEQ ID NO: 59, or
(b) a sequence substantially identical to the nucleotide sequence described in (a) (e.g., as compared to the nucleotide sequence described in (a), a sequence having a sequence identity of at least about 85%, 90%, 95%, 99% or higher, or a sequence having a substitution of one or more nucleotides), or
(c) a sequence that differs by no more than 3, 6, 15, 30 or 45 nucleotides from the nucleotide sequence described in (a), and/or,
the nucleic acid molecule encoding the antibody light chain constant region has a sequence selected from the group consisting of:
(d) a nucleotide sequence as set forth in SEQ ID NO: 26, or
(e) a sequence substantially identical to the nucleotide sequence described in (d) (e.g., as compared to the nucleotide sequence described in (d), a sequence having a sequence identity of at least about 85%, 90%, 95%, 99% or higher, or a sequence having a substitution of one or more nucleotides), or
(f) a sequence that differs by no more than 3, 6, 15, 30 or 45 nucleotides from the nucleotide sequence described in (d).

15. A vector, which comprises the nucleic acid molecule according to any one of claims 12-14; preferably, the vector is a cloning vector or an expression vector.

16. A host cell, which comprises the nucleic acid molecule according to any one of claims 12-14 or the vector according to claim 15.

17. A method for preparing the antibody or antigen-binding fragment thereof according to any one of claims 1-11, comprising culturing the host cell according to claim 16 under conditions that allow expression of the antibody or antigen-binding fragment thereof, and recovering the antibody or antigen-binding fragment thereof from a culture of the cultured host cell.

18. A conjugate, which comprises an antibody or antigen-binding fragment thereof and a coupling moiety, wherein the antibody is the antibody or antigen-binding fragment thereof according to any one of claims 1 to 11.

19. The conjugate according to claim 18, wherein the coupling moiety is selected from a detectable label or a second biologically functional fragment;
preferably, the detectable label is selected from radioisotope, fluorescent substance, luminescent substance, colored substance, enzyme or any combination thereof;
preferably, the second biologically functional fragment is selected from polypeptide, protein, receptor or ligand with binding activity, polyethylene glycol (PEG), nuclide, nucleic acid, small molecule toxin, or any combination thereof.

20. A chimeric antigen receptor, which comprises the antibody or antigen-binding fragment thereof (e.g., ScFv) according to any one of claims 1 to 11, a transmembrane domain, and one or more intracellular T cell signaling domains.

21. A multispecific antibody, which is formed by conjugating a first antibody or fragment thereof with other antibody or fragment thereof or antibody mimetic, wherein each antibody or fragment thereof or antibody mimetic maintains original binding specificity, and the first antibody or fragment thereof is the antibody or antigen-binding fragment thereof according to any one of claims 1 to 11; preferably, the multispecific antibody is a bispecific antibody, a trispecific antibody or a tetraspecific antibody.

22. A pharmaceutical composition, which comprises the antibody or antigen-binding fragment thereof according to any one of claims 1 to 11, or the vector according to claim 15, or the host cell according to claim 16, or the conjugate according to claim 18 or 19, or the chimeric antigen receptor according to claim 20, or the multispecific antibody according to claim 21, and a pharmaceutically acceptable carrier and/or excipient;
preferably, the pharmaceutical composition further comprises an additional pharmaceutically active agent;
more preferably, the additional pharmaceutically active agent is a drug with antitumor activity; more preferably, the additional pharmaceutically active agent is one or more selected from the group consisting of epidermal growth factor receptor (EGFR) inhibitor, immune checkpoint inhibitor, B cell antigen inhibitor, BTK inhibitor, and chemotherapy drug;
more preferably, the antibody or antigen-binding fragment thereof and the additional pharmaceutically active agent are provided as separate components or as components of a single composition;
more preferably, the additional pharmaceutically active agent is one or more selected from the group consisting of anti-PD1/PD-L1 antibody, anti-CD20 antibody, and anti-EGFR antibody.

23. The pharmaceutical composition according to claim 22, wherein the antibody or antigen-binding fragment thereof in the pharmaceutical composition is capable, in a subject, of
(a) inducing tumor cell apoptosis;
(b) inhibiting tumor cell growth, proliferation, differentiation, and/or angiogenesis;
(c) inducing and/or increasing complement-dependent cytotoxic activity;
(d) inducing and/or increasing antibody-dependent cytotoxic activity;
(e) inhibiting the expression and activation of ROR1;
(f) inhibiting ROR1-mediated cell signaling;
(g) preventing and/or treating a ROR1-mediated disease/disorder; or
(h) any combination of (a) to (g).

24. A diagnostic or therapeutic kit, which comprises the antibody or antigen-binding fragment thereof according to any one of claims 1-11, or the vector according to claim 15, or the host cell according to claim 16, or the conjugate according to claim 18 or 19, or the chimeric antigen receptor according to claim 20, or the multispecific antibody according to claim 21, or the pharmaceutical composition according to any one of claims 22-23, and optionally an instruction for use.

25. Use of the antibody or antigen-binding fragment thereof according to any one of claims 1-11, or the vector according to claim 15, or the host cell according to claim 16, or the conjugation according to claim 18 or 19, or the chimeric antigen receptor according to claim 20, or the multispecific antibody according to claim 21, or the pharmaceutical composition according to any one of claims 22-23, in the manufacture of a medicament for the prevention and/or treatment and/or adjuvant treatment of a tumor;
preferably, the antibody or antigen-binding fragment thereof according to any one of claims 1 to 11, or the vector according to claim 15, or the host cell according to claim 16, or the conjugate according to claim 18 or 19, or the chimeric antigen receptor according to claim 20, or the multispecific antibody according to claim 21, is administered separately, in combination, simultaneously or sequentially with an additional pharmaceutically active agent;
more preferably, the additional pharmaceutically active agent is a drug with antitumor activity;
more preferably, the additional pharmaceutically active agent is one or more selected from the group consisting of epidermal growth factor receptor (EGFR) inhibitor, immune checkpoint inhibitor, B cell antigen inhibitor, BTK inhibitor, and chemotherapy drug;
more preferably, the additional pharmaceutically active agent is one or more selected from the group consisting of anti-PD1/PD-L1 antibody, anti-CD20 antibody, and anti-EGFR antibody.

26. Use of the antibody or antigen-binding fragment thereof according to any one of claims 1-11, or the vector according to claim 15, or the host cell according to claim 16, or the conjugation according to claim 18 or 19, or the chimeric antigen receptor according to claim 20, or the multispecific antibody according to claim 21, or the pharmaceutical composition according to any one of claims 22-23, in the manufacture of a medicament which is used to:
(a) induce tumor cell apoptosis;
(b) inhibit tumor cell growth, proliferation, differentiation, and/or angiogenesis;
(c) induce and/or increase complement-dependent cytotoxic activity;
(d) induce and/or increase antibody-dependent cytotoxic activity;
(e) inhibit the expression and activation of ROR1;
(f) inhibit ROR1-mediated cell signaling;
(g) prevent and/or treat a ROR1-mediated disease/disorder; or
(h) any combination of (a) to (g).

27. The use according to claim 25 or 26, wherein the tumor is a ROR1-positive tumor; preferably, the tumor is selected from the group consisting of lymphoma, breast cancer, colorectal cancer, ovarian cancer, lung cancer and/or pancreatic cancer.

28. A method for preventing and/or treating a tumor, and/or delaying tumor progression, and/or reducing or inhibiting tumor recurrence in a subject, the method comprising administering to a subject in need thereof an effective amount of the pharmaceutical composition according to any one of claims 22-23.

29. The method according to claim 28, which further comprises administering to the subject a second therapy, wherein the second therapy is selected from the group consisting of surgery, chemotherapy, radiotherapy, immunotherapy, gene therapy, DNA therapy, RNA therapy, nanotherapy, virotherapy, adjuvant therapy, and any combination thereof;
optionally, the second therapy may be administered separately, in combination, simultaneously or sequentially with the method according to claim 28.

30. The method according to claim 28 or 29, wherein the tumor is a ROR1-positive tumor; preferably, the tumor is selected from the group consisting of lymphoma, breast cancer, colorectal cancer, ovarian cancer, lung cancer and/or pancreatic cancer.

31. A method for detecting the presence or level of ROR1 in a sample, comprising contacting the sample with the antibody or antigen-binding fragment thereof according to any one of claims 1-11 under conditions that allow the formation of a complex between the antibody or antigen-binding fragment thereof and ROR1, and detecting the formation of the complex.

32. Use of the antibody or antigen-binding fragment according to any one of claims 1-11, the conjugate according to claim 18 or 19, or the multispecific antibody according to claim 20 in the manufacture of a diagnostic kit, wherein the kit is used for the diagnosis or differential diagnosis of a tumor or tumor metastasis;
preferably, the tumor is a ROR1-positive tumor;
preferably, the tumor is selected from the group consisting of lymphoma, breast cancer, colorectal cancer, ovarian cancer, lung cancer and/or pancreatic cancer.
